(19) Europäisches Patentamt / European Patent Office / Office européen des brevets

(11) **EP 4 756 026 A1**

(12) **EUROPEAN PATENT APPLICATION**
published in accordance with Art. 153(4) EPC

(43) Date of publication:
**10.06.2026 Bulletin 2026/24**

(21) Application number: **24848392.7**

(22) Date of filing: **02.08.2024**

(51) International Patent Classification (IPC):
**C12N 15/113** (2010.01)    **A61K 48/00** (2006.01)
**C12N 15/85** (2006.01)    **A61P 35/00** (2006.01)

(52) Cooperative Patent Classification (CPC):
**A61K 48/00; A61P 35/00; C12N 15/113; C12N 15/85**

(86) International application number:
**PCT/CN2024/109575**

(87) International publication number:
**WO 2025/026439 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.08.2023  CN 202310969498**
**11.01.2024  PCT/CN2024/071775**

(71) Applicant: **Guangzhou Reforgene Medicine Co., Ltd.**
**Guangzhou, Guangdong 510535 (CN)**

(72) Inventors:
- **LIANG, Junbin**
  **Guangzhou, Guangdong 510535 (CN)**
- **LIANG, Xingxiang**
  **Guangzhou, Guangdong 510535 (CN)**
- **XU, Hui**
  **Guangzhou, Guangdong 510535 (CN)**
- **ZHOU, Lin**
  **Guangzhou, Guangdong 510535 (CN)**
- **ZHANG, Haihui**
  **Guangzhou, Guangdong 510535 (CN)**
- **LI, Qiuting**
  **Guangzhou, Guangdong 510535 (CN)**

(74) Representative: **Dai, Simin**
**Reyda IP**
**A073**
**157, Quai du Président Roosevelt**
**92130 Issy-les-Moulineaux (FR)**

Remarks:
The complete document including Reference Table(s) and the Sequence Listing(s) can be downloaded from the EPO website

(54) **GENE EDITING SYSTEM AND USE THEREOF**

(57)    This disclosure discloses inhibitors of CTGF RNA, MITF RNA, and SRD5A2 RNA and their applications, wherein the inhibitors are gene editing systems. The gene editing systems disclosed herein can knock down CTGF RNA, MITF RNA, or SRD5A2 RNA, and can be used to repair scars, whiten skin, reduce or eliminate melasma, prevent or treat melanoma, and/or prevent or treat androgenetic alopecia. This disclosure also discloses a gene editing system that targets AR RNA encoded by the androgen receptor gene, which can be used to treat androgenetic alopecia and other diseases.

## Description

This application claims priority to Chinese Patent Application No. 202310969498.1, filed on Aug. 2, 2023, and International Patent Application No. PCT/CN2024/071775, filed on Jan. 11, 2024. The entire contents of each of the foregoing applications are incorporated herein by reference.

## TECHNICAL FIELD

The present disclosure relates to the field of gene editing technology, specifically involving gene editing systems and their applications.

## BACKGROUND

Wound healing is a complex cellular activity. Generally, wound healing proceeds in an organized manner, including four stages: hemostasis, inflammation, proliferation, and remodeling. Among these, proliferation and remodeling are particularly important in determining scar formation because they are related to the production and reorganization of the extracellular matrix(ECM). However, due to the high contractility of myofibroblasts, the healing process of scar formation largely produces a disorganized, dense, collagen-rich matrix, thereby disrupting the skin's natural structure. Fibrotic tissue leads to the uncontrolled over-accumulation of proteins under the skin, resulting in a thick, irregular, and uneven skin surface, forming scars. Connective tissue growth factor(CTGF, also known as CCN2) is a key mediator of scar formation.

Melanogenesis is a biosynthetic pathway in which melanin is produced in melanocytes, involving a series of complex enzymatic and chemical catalytic reactions. Five signaling pathways are involved in its regulation, among which microphthalmia-associated transcription factor(MITF) is the final target of multiple signal transduction pathways and is the main regulator of melanogenesis.

Androgenetic alopecia(AGA), also known as male pattern baldness or premature baldness, is a progressive hair loss that gradually occurs in young adults and is the most common type of hair loss. Under the influence of androgens, hair follicles on the scalp gradually shrink and become miniaturized, eventually degenerating into small vellus hairs, which appear as baldness to the naked eye. Androgens are a key factor in the pathogenesis of AGA, but almost all AGA patients have normal levels of androgens in their blood circulation. AGA may be related to increased expression of androgen receptor genes and/or 5a-reductase genes in hair follicles.

The androgen receptor(AR), also known as NR3C4(nuclear receptor subfamily 3, group C, member 4), is a type of nuclear receptor that is activated when the male hormones testosterone or dihydrotestosterone bind to it in the cytoplasm and are then transported into the nucleus.

Multiple genome-wide association studies(GWAS) have shown that the androgen receptor(AR) gene and the ectodysplasim-A2 receptor(EDA2R) gene on the X chromosome are the most important susceptibility genes for AGA.

In addition, the *SRD5A2* gene encodes 3-oxo-5a-steroid 4-dehydrogenase 2, also known as type 2 5a-reductase(5aR2), one of the three isoenzymes of 5a-reductase. SRD5A2 is a key enzyme in androgen metabolism, catalyzing the synthesis of the potent AR agonist dihydrotestosterone(DHT) from testosterone. Elevated SRD5A2 levels have been detected in scalp areas affected by AGA(androgenetic alopecia), and the SRD5A2 inhibitor finasteride has been used as a treatment for patients with AGA.

## SUMMARY

A first aspect of this disclosure provides an inhibitor of CTGF RNA, MITF RNA or SRD5A2 RNA, the inhibitor being a gene editing system.

In some embodiments of this disclosure, the gene editing system knocks down the levels of CTGF RNA, MITF RNA or SRD5A2 RNA, or inhibits the translation of CTGF RNA, MITF RNA or SRD5A2 RNA.

In some embodiments of this disclosure, the gene editing system knocks down the levels of CTGF RNA, MITF RNA or SRD5A2 RNA.

In some embodiments of this disclosure, the gene editing system comprises:

(a) a guide RNA comprising a guide sequence that hybridizes with a target RNA, or a polynucleotide sequence encoding the guide RNA; and

(b) an RNA-guided nuclease, or a polynucleotide sequence encoding the RNA-guided nuclease;

the guide RNA is capable of forming a complex with the nuclease and guiding the complex to bind specifically to the target RNA, which is CTGF RNA, MITF RNA or SRD5A2 RNA.

In some embodiments of this disclosure, the guide RNA is capable of forming a complex with the nuclease and directing the complex to bind to and cleave the target RNA.

In some embodiments of this disclosure, the target RNA is CTGF RNA, MITF RNA, or SRD5A2 RNA. In some embodiments of this disclosure, the target RNA is CTGF pre-mRNA, MITF pre-mRNA, or SRD5A2 pre-mRNA, or CTGF mRNA, MITF mRNA, or SRD5A2 mRNA. In some embodiments of this disclosure, the target RNA is CTGF mRNA, MITF mRNA, or SRD5A2 mRNA. In some embodiments of this disclosure, the target RNA is mammalian CTGF RNA, MITF RNA, or SRD5A2 RNA. In some embodiments of this disclosure, the target RNA is human CTGF RNA, MITF RNA, or SRD5A2 RNA. In some embodiments of this disclosure, the target RNA is human CTGF mRNA, MITF mRNA, or SRD5A2 mRNA.

In some embodiments of this disclosure, the target RNA sequence is as shown in SEQ ID NO: 14, 22 or 35.

In some embodiments of this disclosure, the target RNA sequence is nucleotides 494 to 785 of the sequence shown in SEQ ID NO: 14, nucleotides 404 to 606 of the sequence shown in SEQ ID NO: 22, or nucleotides 484 to 820 of the sequence shown in SEQ ID NO: 35.

In some embodiments of this disclosure, the target RNA sequence is the nucleotide sequence of nucleotides 494, 604, 678 or 761, to 518, 628, 702 or 785 of the sequence shown in SEQ ID NO: 14(human CCN2 mRNA, NCBI NM_001901.4).

In some embodiments of this disclosure, the target RNA sequence is the nucleotide sequence of nucleotides 404, 429, 453, 479, 509, 546 or 582, to 428, 453, 477, 503, 533, 570 or 606 of the sequence shown in SEQ ID NO: 22(human MITF mRNA, NCBI NM_001354607.2).

In some embodiments of this disclosure, the target RNA sequence is the nucleotide sequence of nucleotides 484, 524, 547, 585, 635, 721, 755 or 796, up to nucleotides 508, 548, 571, 609, 659, 745, 779 or 820 of the sequence shown in SEQ ID NO: 35(human SRD5A2 mRNA, NCBI XM_011533072.3).

In some embodiments of this disclosure, the guide sequence has at least 80% sequence identity with the target RNA. In some embodiments of this disclosure, the guide sequence has at least 85%, at least 90%, at least 95%, or 100% sequence identity with the target RNA. Further, in some embodiments of this disclosure, the guide sequence has 100% sequence identity with the target RNA.

In some embodiments of this disclosure, the guide sequence has at least 80% sequence identity with the sequence shown in any one of SEQ ID NO: 14, 22, or 35. In some embodiments of this disclosure, the guide sequence has at least 85%, at least 90%, at least 95%, or 100% sequence identity with the sequence shown in any one of SEQ ID NO: 14, 22, or 35. Further, in some embodiments of this disclosure, the guide sequence has 100% sequence identity with the sequence shown in any one of SEQ ID NO: 14, 22, or 35.

In some embodiments of this disclosure, the guide sequence has at least 80%, at least 85%, at least 90 %, at least 95%, or 100% sequence identity with any of the sequences shown in SEQ ID NOs: 5-13, 23-32, and 36-57. Further, in some embodiments of this disclosure, the guide sequence has 100% sequence identity with any of the sequences shown in SEQ ID NOs: 5-13, 23-32, and 36-57. In some embodiments of this disclosure, the guide sequence is any of the sequences shown in SEQ ID NOs: 5-13, 23-32, and 36-57.

In some embodiments of this disclosure, the guide sequence has at least 80%, at least 85%, at least 90%, at least 95%, or 100% sequence identity with any of the sequences shown in SEQ ID NOs: 8, 9, 10, 12, 23-29, 36, 38, 40, 42, 43, 44, 46, or 54. In some embodiments of this disclosure, the guiding sequence is any of the sequences shown in SEQ ID NOs: 8, 9, 10, 12, 23-29, 36, 38, 40, 42, 43, 44, 46, or 54.

In some embodiments of this disclosure, the guide RNA comprises a guide sequence and a scaffold sequence. The scaffold sequence interacts with an RNA-guided nuclease.

In some embodiments of this disclosure, the scaffold sequence is a repeating sequence in the same direction.

In some embodiments of this disclosure, the direct repeat sequence comprises a sequence having at least 50%, at least 55%, at least 60 %, at least 65 %, at least 70 %, at least 75 %, at least 80%, at least 85%, at least 90 %, at least 95 %, at least 96 %, at least 97 %, at least 98 %, at least 99 %, or 100% sequence identity with the sequence shown in SEQ ID NO: 2 or 3.

In some embodiments of this disclosure, the RNA-guided nuclease is selected from Cas9, Cas12, Cas13, TnpB, IscB, IsrB, Fancor nuclease, or fragments thereof(including but not limited to nucleic acid binding domain fragments).

In some embodiments of this disclosure, the RNA-guided nuclease is a Cas protein.

In some embodiments of this disclosure, the guide RNA is capable of forming a CRISPR complex with the Cas protein and guiding the CRISPR complex to bind to the target RNA in a sequence-specific manner.

In some embodiments of this disclosure, the guide RNA is capable of forming a CRISPR complex with the Cas protein and directing the CRISPR complex to bind to and cleave the target RNA.

In some embodiments of this disclosure, the RNA-guided nuclease is a Cas9 protein, a Cas12 protein, or a Cas13 protein.

In some embodiments of this disclosure, the RNA-guided nuclease is a Cas13 protein. In some embodiments, the Cas13 protein is preferably a Cas13a protein, a Cas13b protein, a Cas13c protein, or a Cas13d protein.

In some embodiments of this disclosure, the amino acid sequence of the Cas13 protein has at least 50%, at least 55 %, at least 60 %, at least 65%, at least 70%, at least 75%, at least 80%, at least 85%, at least 90%, at least 95%, at least 96 %, at least 97%, at least 98 %, at least 99 %, or 100% sequence identity with the sequence shown in SEQ ID NO: 1.

In some embodiments of this disclosure, the Cas13 protein comprises the sequence shown in SEQ ID NO: 1.

In some embodiments of this disclosure, the RNA-guided nuclease comprises any one or more of the following: subcellular localization signal, deaminase domain, translation activation domain, translation repression domain, RNA methylation domain, RNA demethylation domain, nuclease domain, splicing factor domain, reporter tag, and affinity tag.

In some embodiments of this disclosure, the Cas protein comprises any one or more of the following: a subcellular localization signal, a deaminase domain, a translation activation domain, a translation repression domain, an RNA methylation domain, an RNA demethylation domain, a nuclease domain, a splicing factor domain, a reporter tag, and an affinity tag.

In some embodiments of this disclosure, the subcellular localization signal may be selected from the nuclear localization signal and the nuclear output signal sequence.

In some embodiments of this disclosure, the gene editing system comprises:

(a) a guide RNA, or a polynucleotide sequence encoding the guide RNA; and
(b) a Cas13 protein, or a polynucleotide sequence encoding the Cas13 protein;

wherein the guide RNA is able to form a complex with the Cas13 protein and guide the complex to bind to and cleave the target RNA.

In some embodiments of this disclosure, the polynucleotide sequence encoding the guide RNA is linked to a first regulatory sequence, the first regulatory sequence being used to regulate the expression of the guide RNA.

In some embodiments of this disclosure, the polynucleotide sequence encoding the RNA-guided nuclease is linked to a second regulatory sequence, the second regulatory sequence being used to regulate the expression of the RNA-guided nuclease.

In some embodiments of this disclosure, the polynucleotide sequence encoding the RNA-guided nuclease is linked to a regulatory sequence, the regulatory sequence being used to regulate the expression of the RNA-guided nuclease.

In some embodiments of this disclosure, the polynucleotide sequence encoding the guide RNA is linked to a regulatory sequence, the regulatory sequence being used to regulate the expression of the guide RNA.

In some embodiments of this disclosure, the regulatory sequence regulating the expression of the RNA-guided nuclease may be the same as or different from the regulatory sequence regulating the expression of the guiding RNA.

In some embodiments of this disclosure, the regulatory sequence is a promoter sequence. In some embodiments of this disclosure, the regulatory sequence is an enhancer sequence. In some embodiments of this disclosure, the regulatory sequence is both a promoter and an enhancer sequence.

The gene editing systems described herein can be introduced into cells(or cell-free systems) in a variety of non-restrictive ways: (i) as mRNA encoding an RNA-guided nuclease and guide RNA,(ii) as part of a single vector or plasmid, or as a combination of multiple vectors or plasmids,(iii) as a single RNA-guided nuclease and guide RNA, or(iv) as an RNP complex of an RNA-guided nuclease and guide RNA.

In some embodiments of this disclosure, the complex reduces the level of the target RNA in mammals, such as humans.

In some embodiments of this disclosure, the complex reduces the level of the target RNA in cells, for example, the level of the target RNA in cells expressing the target RNA.

In some embodiments of this disclosure, the complex reduces the level of the target RNA in cells by at least 5%, at least 10%, at least 15%, at least 20%, at least 25 %, at least 30 %, at least 35%, at least 40%, at least 50%, at least 60 %, at least 70%, at least 80%, at least 90%, at least 95 %, at least 96 %, at least 97%, at least 98%, or at least 99%. The reduction in the target RNA level can be tested using methods conventional in the art; including but not limited to the qPCR method described in the examples, using untreated cells or cells treated with a gene editing system targeting a non-mammalian genome as a negative control, and calculating the target RNA knockdown level in the experimental group compared to the negative control.

In some embodiments of the present invention, the number of off-target genes when the complex binds to and cleaves the target RNA is less than 40, less than 35, less than 30, less than 25, less than 20, less than 19, less than 18, less than 17, less than 16, less than 15, less than 14, less than 13, less than 12, less than 11, less than 10, less than 9, less than 8, less than 7, less than 6, less than 5, less than 4, less than 3, less than 2, or less than 1. The number of off-target genes can be determined by conventional methods in the art. In some embodiments, the number of off-target genes is determined by differentially expressed genes identified through RNA sequencing.

In some embodiments of this disclosure, the complex reduces the level of the target RNA-encoded protein in an animal(such as a human).

In some embodiments of this disclosure, the complex, upon contact with a cell containing the target RNA, reduces the level of the protein encoded by the target RNA in the cell. In some embodiments of this disclosure, the protein encoded by the target RNA is CTGF protein, MITF protein, or SRD5A2 protein. In some embodiments of this disclosure, the complex reduces the level of CTGF protein, MITF protein, or SRD5A2 protein in the cell.

In some embodiments of this disclosure, the complex reduces the levels of CTGF, MITF, or SRD5A2 proteins in cells by at least 5%, at least 10%, at least 15%, at least 20%, at least 25 %, at least 30%, at least 35 %, at least 40%, at least 50%, at least 60 %, at least 70%, at least 80%, at least 90%, at least 95%, at least 96 %, at least 97%, at least 98%, or at least 99%. The reduction in the level of the target RNA-encoded protein can be tested using methods conventional in the art, including but not limited to ELISA and Western blotting. Untreated cells or cells treated with a gene-editing system targeting a non-mammalian genome can be used as negative controls to calculate the knockdown levels of CTGF, MITF, or SRD5A2 proteins in the experimental group compared to the negative control group.

A second aspect of this disclosure provides a guide RNA(gRNA) for a gene editing system.

In some embodiments of this disclosure, the guide RNA comprises a guide sequence for hybridization with a target RNA, which is CTGF RNA, MITF RNA or SRD5A2 RNA.

In some embodiments of this disclosure, the guide RNA comprises a guide sequence and a scaffold sequence. The scaffold sequence interacts with an RNA-guided nuclease. The scaffold sequence is the sequence in the guide RNA molecule that is typically kept unchanged when the guide RNA molecule is designed. For example, the scaffold sequence may refer to a portion of the guide RNA molecule other than the guide sequence. In some embodiments of this disclosure, the scaffold sequence is a direct repeat(DR) sequence.

In some embodiments of this disclosure, the guide sequence has at least 80% sequence identity with the target RNA. In some embodiments of this disclosure, the guide sequence has at least 85%, at least 90%, at least 95%, or 100% sequence identity with the target RNA. Further, in some embodiments of this disclosure, the guide sequence has 100% sequence identity with the target RNA.

In some embodiments of this disclosure, the guide sequence has at least 80% sequence identity with the sequence shown in any one of SEQ ID NO: 14, 22, or 35. In some embodiments of this disclosure, the guide sequence has at least 85%, at least 90%, at least 95%, or 100% sequence identity with the sequence shown in any one of SEQ ID NO: 14, 22, or 35. Further, in some embodiments of this disclosure, the guide sequence has 100% sequence identity with the sequence shown in any one of SEQ ID NO: 14, 22, or 35.

In some embodiments of this disclosure, the guide sequence has at least 85%, at least 90%, at least 95% or 100% sequence identity with the nucleotide sequence of nucleotides 494 to 785 of the sequence shown in SEQ ID NO: 14, nucleotides 404 to 606 of the sequence shown in SEQ ID NO: 22, or nucleotides 484 to 820 of the sequence shown in SEQ ID NO: 35.

In some embodiments of this disclosure, the guide sequence has at least 85 %, at least 90 %, at least 95%, or 100% sequence identity with the nucleotide sequence of the sequence shown in SEQ ID NO: 14, from the 494th, 604th, 678th, or 761st nucleotides to the 518th, 628th, 702nd, or 785th nucleotides.

In some embodiments of this disclosure, the guide sequence has at least 85 %, at least 90 %, at least 95%, or 100% sequence identity with the nucleotide sequence of nucleotides 404, 429, 453, 479, 509, 546, or 582, up to 428, 453, 477, 503, 533, 570, or 606 of the sequence shown in SEQ ID NO: 22.

In some embodiments of this disclosure, the guide sequence has at least 85 %, at least 90 %, at least 95%, or 100% sequence identity with the nucleotide sequence of nucleotides 484, 524, 547, 585, 635, 721, 755, or 796, up to 508, 548, 571, 609, 659, 745, 779, or 820 of the sequence shown in SEQ ID NO: 35.

In some embodiments of this disclosure, the guide sequence has at least 80%, at least 85%, at least 90%, at least 95%, or 100% sequence identity with any of the sequences shown in SEQ ID NOs: 5-13, 23-32, and 36-57. Further, in some embodiments of this disclosure, the guide sequence has 100% sequence identity with any of the sequences shown in SEQ ID NOs: 5-13, 23-32, and 36-57. In some embodiments of this disclosure, the guide sequence comprises any of the sequences shown in SEQ ID NOs: 5-13, 23-32, and 36-57. In some embodiments of this disclosure, the guide sequence is any of the sequences shown in SEQ ID NOs: 5-13, 23-32, and 36-57.

In some embodiments of this disclosure, the guide sequence has at least 80%, at least 85%, at least 90%, at least 95%, or 100% sequence identity with any of the sequences shown in SEQ ID NOs: 8, 9, 10, 12, 23-29, 36, 38, 40, 42, 43, 44, 46, or 54. In some embodiments of this disclosure, the guiding sequence comprises any of the sequences shown in SEQ ID NOs: 8, 9, 10, 12, 23-29, 36, 38, 40, 42, 43, 44, 46, or 54. In some embodiments disclosed herein, the guide sequence is any one of the sequences shown in SEQ ID NOs: 8, 9, 10, 12, 23-29, 36, 38, 40, 42, 43, 44, 46, 54.

In some embodiments of this disclosure, the scaffold sequence is a direct repeat sequence, which comprises a sequence having at least 50%, at least 55%, at least 60 %, at least 65%, at least 70%, at least 75 %, at least 80%, at least 85%, at least 90 %, at least 95%, at least 96%, at least 97 %, at least 98 %, at least 99%, or 100% sequence identity with the sequence shown in SEQ ID NO: 2 or 3.

In some embodiments of this disclosure, the direct repeat sequence comprises the sequence shown in SEQ ID NO: 2 or 3. In some embodiments of this disclosure, the direct repeat sequence consists of the sequence shown in SEQ ID NO: 2 or 3.

In some embodiments of this disclosure, the guide sequence is located at the 3' end or 5' end of the same-direction repeat sequence. In some embodiments of this disclosure, the guide sequence is located at the 3' end of the same-direction repeat sequence. In some embodiments of this disclosure, the guide sequence is located at the 5' end of the same-direction repeat sequence.

In some embodiments of this disclosure, the guide RNA comprises an aptamer sequence.

In some embodiments of this disclosure, the aptamer sequence is inserted into a loop of the stem-loop structure of the secondary structure of the same-direction repeat sequence of the guide RNA.

In some embodiments of this disclosure, the guide RNA comprises modified nucleotides. The modifications include, but are not limited to, 2'-O-methyl, 2'-O-methyl-3'-thiophosphate, or 2'-O-methyl-3'-thioPACE modifications. In some embodiments of this disclosure, the guide RNA comprises modified nucleotides selected from deoxyribonucleotides and locked nucleic acids(LNAs). In some embodiments, the guide RNA comprises at least one chemically modified nucleotide. In some embodiments, the guide RNA is a hybrid RNA-DNA guide, i.e., a portion of the RNA nucleotides in the guide RNA are replaced by DNA nucleotides. In some embodiments, the guide RNA is a hybrid RNA-LNA(locked nucleic acid) guide, i.e., a portion of the RNA nucleotides in the guide RNA are replaced by LNA nucleotides.

In some embodiments of this disclosure, the target RNA is located in the nucleus and/or cytoplasm of a eukaryotic cell.

In some embodiments of this disclosure, the guide RNA is capable of forming a complex(also known as a gene editing complex) with an RNA-guided nuclease and directing the complex to bind specifically to the target RNA.

In some embodiments of this disclosure, the guide RNA is capable of forming a complex with an RNA-guided nuclease and directing the complex to bind to and cleave the target RNA.

In some embodiments of this disclosure, the complex reduces the level of the target RNA in mammals, such as humans.

A third aspect of this disclosure provides an isolated nucleic acid that encodes a guide RNA as described in this disclosure.

A fourth aspect of this disclosure provides a vector comprising a polynucleotide sequence encoding a guide RNA according to this disclosure, and a regulatory sequence for regulating the expression of the guide RNA.

In some embodiments disclosed herein, the vector is an adeno-associated virus vector.

In some embodiments of this disclosure, the regulatory sequence is a promoter sequence. In some embodiments of this disclosure, the regulatory sequence is an enhancer sequence. In some embodiments of this disclosure, the regulatory sequence is both a promoter and an enhancer sequence.

In some embodiments of this disclosure, the regulatory sequence is a U6 promoter or an eye-specific promoter.

In some embodiments of this disclosure, the eye-specific promoter is selected from: retinal-specific promoter, K12 promoter, rhodopsin promoter, rod cell-specific promoter, cone cell-specific promoter, rhodopsin kinase promoter, GRK1 promoter, interphotoreceptor retinoid-binding protein proximal(IRBP) promoter, and opsin promoters(e.g., red opsin promoter, blue opsin promoter, etc.).

In some embodiments of this disclosure, the promoter is a chicken β-actin(CB) promoter. The chicken β-actin promoter may be a short chicken β-actin promoter or a long chicken β-actin promoter. In some embodiments, the promoter(e.g., a chicken β-actin promoter) contains an enhancer sequence, such as a cytomegalovirus(CMV) enhancer sequence. The CMV enhancer sequence may be a short CMV enhancer sequence or a long CMV enhancer sequence. In some embodiments, the promoter contains a long CMV enhancer sequence and a long chicken β-actin promoter. In some embodiments, the promoter contains a short CMV enhancer sequence and a short chicken β-actin promoter. However, those skilled in the art will recognize that a short CMV enhancer can be used with a long CB promoter, and a long CMV enhancer can be used with a short CB promoter. In some embodiments of this disclosure, the promoter is a CBh promoter.

In some embodiments of this disclosure, the promoter is the CBh promoter.

In some embodiments of this disclosure, the regulatory sequence includes an HRE enhancer element.

In some embodiments of this disclosure, the regulatory sequence comprises a series of NRS elements and an HRE enhancer element.

A fifth aspect of this disclosure provides a vector system comprising a polynucleotide sequence encoding the guide RNA of this disclosure and a first regulatory sequence regulating the expression of the guide RNA; and a polynucleotide sequence encoding a nuclease guided by the RNA and a second regulatory sequence regulating the expression of the nuclease guided by the RNA.

In some embodiments of this disclosure, the vector system comprises one or more vectors.

In some embodiments of this disclosure, the vector system comprises multiple vectors, wherein a first vector is located on a polynucleotide sequence encoding the guide RNA and a first regulatory sequence regulating the expression of the guide RNA, and a second vector is located on a polynucleotide sequence encoding the RNA-guided nuclease and a second regulatory sequence regulating the expression of the RNA-guided nuclease.

In some embodiments of this disclosure, the regulatory sequence is a promoter sequence. In some embodiments of this disclosure, the regulatory sequence is an enhancer sequence. In some embodiments of this disclosure, the regulatory sequence is both a promoter and an enhancer sequence.

A sixth aspect of this disclosure provides an adeno-associated virus vector, wherein the adeno-associated virus vector comprises an RNA-guided nuclease encoding the RNA and DNA of the directing RNA described in this disclosure.

A seventh aspect of this disclosure provides a lipid nanoparticle, wherein the lipid nanoparticle comprises the guide RNA described in this disclosure and an mRNA encoding a nuclease guided by the RNA.

An eighth aspect of this disclosure provides a lentiviral vector, wherein the lentiviral vector comprises the guide RNA described in this disclosure and an mRNA encoding an RNA-guided nuclease; optionally, the lentiviral vector is pseudotyped with an envelope protein; optionally, the mRNA encoding the RNA-guided nuclease is linked to an aptamer sequence.

A ninth aspect of this disclosure provides a ribonucleoprotein complex, wherein the ribonucleoprotein complex is formed by the guide RNA and RNA-guided nuclease described in this disclosure.

A tenth aspect of this disclosure provides a virus-like particle, wherein the virus-like particle comprises a ribonucleoprotein complex formed by the guide RNA and the RNA-guided nuclease described in this disclosure; optionally, the RNA-guided nuclease is fused with a gag protein.

An eleventh aspect of this disclosure provides a cell comprising the inhibitors, guide RNA, nucleic acids, vectors and/or vector systems described in this disclosure; optionally, the cell is a eukaryotic cell.

A twelfth aspect of this disclosure provides a pharmaceutical composition comprising the inhibitor, guide RNA, nucleic acid, vector and/or vector system described in this disclosure.

In some embodiments of this disclosure, the pharmaceutical composition comprises pharmaceutically acceptable excipients.

The thirteenth aspect of this disclosure provides the use of the inhibitors, guide RNAs, nucleic acids, vectors, vector systems, adeno-associated virus vectors, lipid nanoparticles, lentiviral vectors, ribonucleoprotein complexes, virus-like particles, eukaryotic cells, and/or pharmaceutical compositions described herein in any of the following or in the preparation of reagents that achieve any of the following:

Cutting one or more target RNA molecules or nicking one or more target RNA molecules, activating or upregulating one or more target RNA molecules, activating or inhibiting the translation of one or more target RNA molecules, inactivating one or more target RNA molecules, visualizing, labeling or detecting one or more target RNA molecules, binding one or more target RNA molecules, transporting one or more target RNA molecules, and masking one or more target RNA molecules.

Some embodiments of this disclosure provide for use of the inhibitors, guide RNAs, nucleic acids, vectors, vector systems, adeno-associated virus vectors, lipid nanoparticles, lentiviral vectors, ribonucleoprotein complexes, virus-like particles, eukaryotic cells, and/or pharmaceutical compositions described herein in any of the following or in the preparation of reagents that achieve any of the following:

Cut one or more target RNA molecules, inhibit the translation of one or more target RNA molecules, and bind to one or more target RNA molecules.

Some embodiments of this disclosure provide for use of the inhibitors, guide RNAs, nucleic acids, vectors, vector systems, adeno-associated virus vectors, lipid nanoparticles, lentiviral vectors, ribonucleoprotein complexes, virus-like particles, eukaryotic cells, and/or pharmaceutical compositions described herein in any of the following or in the preparation of reagents that achieve any of the following:

Binds to one or more target RNA molecules.

Some embodiments of this disclosure provide for use of the inhibitors, guide RNAs, nucleic acids, vectors, vector systems, adeno-associated virus vectors, lipid nanoparticles, lentiviral vectors, ribonucleoprotein complexes, virus-like particles, eukaryotic cells, and/or pharmaceutical compositions described herein in any of the following or in the preparation of reagents that achieve any of the following:

Cut one or more target RNA molecules.

In some embodiments of this disclosure, the target RNA is CTGF pre-mRNA, MITF pre-mRNA, or SRD5A2 pre-mRNA, or CTGF mRNA, MITF mRNA, or SRD5A2 mRNA(i.e., mature mRNA). In some embodiments of this disclosure, the target RNA is CTGF mRNA, MITF mRNA, or SRD5A2 mRNA. In some embodiments of this disclosure, the target RNA is mammalian CTGF RNA, MITF RNA, or SRD5A2 RNA, for example, CTGF RNA, MITF RNA, or SRD5A2 RNA selected from humans, rats, mice, and non-human primates such as monkeys, dogs, pigs, rabbits, etc. In some embodiments of this disclosure, the target RNA is human CTGF RNA, MITF RNA, or SRD5A2 RNA. In some embodiments of this disclosure, the target RNA is human CTGF mRNA, MITF mRNA, or SRD5A2 mRNA.

The fourteenth aspect of this disclosure provides a method for diagnosing, treating or preventing diseases or conditions associated with target RNA by administering an effective amount of an inhibitor, guide RNA, nucleic acid, vector, vector system, adeno-associated virus vector, lipid nanoparticle, lentiviral vector, ribonucleoprotein complex, virus-like particle, eukaryotic cell and/or pharmaceutical composition according to this disclosure to a sample of a subject in need or to a subject in need.

In some embodiments of this disclosure, the disease or condition associated with the target RNA refers to a disease or condition caused by abnormally high expression of the target RNA.

In some embodiments of this disclosure, the target RNA is CTGF pre-mRNA, MITF pre-mRNA, or SRD5A2 pre-mRNA, or CTGF mRNA, MITF mRNA, or SRD5A2 mRNA(i.e., mature mRNA). In some embodiments of this disclosure, the target RNA is CTGF mRNA, MITF mRNA, or SRD5A2 mRNA. In some embodiments of this disclosure, the target RNA is human CTGF RNA, MITF RNA, or SRD5A2 RNA. In some embodiments of this disclosure, the target RNA is human CTGF mRNA, MITF mRNA, or SRD5A2 mRNA.

In some embodiments of this disclosure, the diseases associated with the target RNA include: melanoma, androgenetic alopecia, and scarring.

In some embodiments of this disclosure, the conditions associated with the target RNA include: melanoma, androgenetic alopecia, and scarring.

The fifteenth aspect of this disclosure provides the use of the inhibitors, guide RNAs, nucleic acids, vectors, vector systems, adeno-associated virus vectors, lipid nanoparticles, lentiviral vectors, ribonucleoprotein complexes, virus-like particles, eukaryotic cells and/or pharmaceutical compositions described herein in the preparation of medicaments for diagnosing, treating or preventing of diseases or conditions associated with target RNA.

In some embodiments of this disclosure, the disease or condition associated with the target RNA refers to a disease or condition caused by abnormally high expression of the target RNA.

In some embodiments of this disclosure, the target RNA is CTGF pre-mRNA, MITF pre-mRNA, or SRD5A2 pre-mRNA, or CTGF mRNA, MITF mRNA, or SRD5A2 mRNA. In some embodiments of this disclosure, the target RNA is CTGF mRNA, MITF mRNA, or SRD5A2 mRNA. In some embodiments of this disclosure, the target RNA is human CTGF RNA, MITF RNA, or SRD5A2 RNA. In some embodiments of this disclosure, the target RNA is human CTGF mRNA, MITF mRNA, or SRD5A2 mRNA.

In some embodiments of this disclosure, the diseases associated with the target RNA include: melanoma, androgenetic alopecia, and scarring.

In some embodiments of this disclosure, the conditions associated with the target RNA include: melanoma, androgenetic alopecia, and scarring.

The sixteenth aspect of this disclosure provides the use of inhibitors, guide RNAs, nucleic acids, vectors, vector systems, adeno-associated virus vectors, lipid nanoparticles, lentiviral vectors, ribonucleoprotein complexes, virus-like particles and/or eukaryotic cells as described in this disclosure in the preparation of cosmetics.

In some embodiments of this disclosure, the cosmetic can be used to repair scars, whiten skin, reduce or eliminate melasma, prevent or treat melanoma, and/or prevent or treat androgenetic alopecia.

The seventeenth aspect of this disclosure provides a cosmetic product comprising an inhibitor, guide RNA, nucleic acid, vector, delivery system, adeno-associated virus vector, lipid nanoparticle, lentiviral vector, ribonucleoprotein complex, virus-like particle and/or eukaryotic cell as described in this disclosure.

In some embodiments of this disclosure, the cosmetic can be used to repair scars, whiten skin, reduce or eliminate melasma, prevent or treat melanoma, and/or prevent or treat androgenetic alopecia.

In some embodiments of this disclosure, the inhibitors, guide RNAs, nucleic acids, vectors, vector systems, adeno-associated virus vectors, lipid nanoparticles, lentiviral vectors, ribonucleoprotein complexes, virus-like particles, cells, pharmaceutical compositions, or cosmetics described herein may be administered to subjects, such as humans and animals, in an effective amount. The effective amount refers to an amount that is functional or active in humans and/or animals and is acceptable to humans and/or animals.

In some embodiments of this disclosure, the inhibitors, guide RNAs, nucleic acids, vectors, vector systems, adeno-associated virus vectors, lipid nanoparticles, lentiviral vectors, ribonucleoprotein complexes, virus-like particles, cells, pharmaceutical compositions, or cosmetics described herein may further comprise pharmaceutically, cosmetically, chemically, or biologically acceptable ingredients. For example, pharmaceutically acceptable excipients, and various cosmetically acceptable excipients, thickeners, or diluents.

The eighteenth aspect of this disclosure provides the use of a gene-editing system in the preparation of medicaments for the diagnosis, treatment, or prevention of androgenetic alopecia, the gene-editing system: knock down AR RNA levels or inhibit AR RNA translation.

In some embodiments of this disclosure, the gene editing system knocks down AR RNA levels or inhibits the translation of AR RNA.

In some embodiments of this disclosure, the gene editing system knocks down AR RNA levels.

In some embodiments of this disclosure, the gene editing system inhibits the translation of AR RNA.

In some embodiments of this disclosure, the gene editing system comprises: a guide RNA comprising a guide sequence that hybridizes with AR RNA or a polynucleotide sequence of the guide RNA, and an RNA-guided nuclease or a polynucleotide sequence encoding the nuclease.

The guide RNA is capable of forming a complex with the nuclease and guiding the complex to bind to the AR RNA in a sequence-specific manner.

In some embodiments of this disclosure, the gene editing system comprises:

a guide RNA comprising a guide sequence that hybridizes with AR RNA, and an RNA-guided nuclease;
wherein the guide RNA is capable of forming a complex with the nuclease and guiding the complex to bind specifically to the AR RNA sequence.

In some embodiments of this disclosure, the gene editing system comprises:

A polynucleotide sequence encoding a guide RNA comprising a guide sequence that hybridizes with AR RNA, and a polynucleotide sequence encoding an RNA-guided nuclease;
wherein the guide RNA is capable of forming a complex with the nuclease and guiding the complex to bind specifically to the AR RNA sequence.

In some embodiments of this disclosure, the AR RNA is pre-mRNA and/or mature mRNA.
In some embodiments of this disclosure, the AR RNA is human pre-mRNA and/or mature mRNA.
In some embodiments of this disclosure, the AR RNA sequence is selected from any of the sequences shown in SEQ ID NOs: 394-396.
In some embodiments of this disclosure, the AR RNA is the sequence numbered NM_000044.6(SEQ ID NO: 394) in the NCBI database, namely "transcript variant 1".
In some embodiments of this disclosure, the AR RNA is the sequence numbered NM_001011645.3(SEQ ID NO: 395) in the NCBI database, namely "transcript variant 2".
In some embodiments of this disclosure, the AR RNA is the sequence numbered ENST00000374690.9(SEQ ID NO: 396) in the Ensembl database.
In some embodiments of this disclosure, the guide RNA directs the complex to bind to and cleave the AR RNA.
In some embodiments of this disclosure, the complex, upon contact with a cell containing AR RNA, reduces the level of the AR RNA or AR protein(androgen receptor encoded by AR RNA) in the cell by at least 5%, at least 10%, at least 15 %, at least 20%, at least 25%, at least 30%, at least 35%, at least 40 %, at least 50%, at least 60%, at least 70 %, at least 80%, at least 90%, at least 95%, at least 96%, at least 97%, at least 98%, or at least 99%.
In some embodiments of this disclosure, the complex, upon contact with a cell containing AR RNA, reduces the level of the AR RNA in the cell by at least 5%, at least 10%, at least 15%, at least 20%, at least 25 %, at least 30%, at least 35%, at least 40 %, at least 50 %, at least 60%, at least 70%, at least 80%, at least 90%, at least 95%, at least 96%, at least 97%, at least 98%, or at least 99%.
In some embodiments of this disclosure, the complex, upon contact with a cell containing AR RNA, reduces the level of the AR protein in the cell by at least 5%, at least 10%, at least 15%, at least 20%, at least 25 %, at least 30%, at least 35%, at least 40 %, at least 50 %, at least 60%, at least 70%, at least 80%, at least 90%, at least 95%, at least 96%, at least 97%, at least 98%, or at least 99%.
In some embodiments of this disclosure, the cell is a eukaryotic cell. In some embodiments of this disclosure, the cell is a mammalian cell. In some embodiments of this disclosure, the cell is a human cell.
In some embodiments of this disclosure, the number of off-target genes when the complex binds to and cleaves the target RNA(the AR RNA) is less than 40, less than 35, less than 30, less than 25, less than 20, less than 19, less than 18, less than 17, less than 16, less than 15, less than 14, less than 13, less than 12, less than 11, less than 10, less than 9, less than 8, less than 7, less than 6, less than 5, less than 4, less than 3, less than 2, or less than 1.
In some embodiments of this disclosure, the guide sequence of the guide RNA has at least 80%, at least 85%, at least 90%, at least 95% or 100% sequence identity with the sequence shown in any one of SEQ ID NOs: 394-396.
In some embodiments of this disclosure, the guide sequence of the guide RNA has 100% sequence identity with the sequence shown in any one of SEQ ID NOs: 394-396.
In some embodiments of this disclosure, the guide sequence of the guide RNA has at least 80%, at least 85%, at least 90%, at least 95% or 100% sequence identity with the sequence shown in SEQ ID NO: 394.
In some embodiments of this disclosure, the guide sequence of the guide RNA has 100% sequence identity with the sequence shown in SEQ ID NO: 394.
In some embodiments of this disclosure, the guide sequence of the guide RNA has at least 80%, at least 85%, at least 90%, at least 95 % or 100% sequence identity with the nucleotide sequence consisting of nucleotides 2826-2990 of the sequence shown in SEQ ID NO: 394.
The inventors designed gRNAs for NM_001011645.3 transcript variant 2, mRNA(SEQ ID NO: 395) from NCBI and transcript ENST00000374690.9(SEQ ID NO: 396) from the Ensembl database, and their guide sequences are shown in Table 9.

Table 9. Designed gRNAs

| gRNA | Guide sequence | SEQ ID NO |
|---|---|---|
| AR-h1 | TGCATGCAATGATACGATCGAGTTC | 71 |
| AR-h2 | AGTACTGAATGACAGCCATCTGGTC | 72 |
| AR-h3 | ATAACATTTCCGAAGACGACAAGAT | 73 |
| AR-h4 | AAGAAGACCTTGCAGCTTCCACATG | 74 |
| AR-h5 | AGGACATTCAGAAAGATGGGCTGAC | 75 |
| AR-h6 | GTTCATTGAGGCTAGAGAGCAAGGC | 76 |
| AR-h7 | AATGCTGAAGAGTAGCAGTGCTTTC | 77 |
| AR-h8 | AGAGCTCCATAGTGACACCCAGAAG | 78 |
| AR-h9 | CAATCATTTCTGCTGGCGCACAGGT | 79 |
| AR-h10 | CATAGCCTTCAATGTGTGACACTGT | 80 |
| AR-h11 | GCAAACACCATGAGCCCCATCCAGG | 81 |
| AR-h12 | AAACTCTTGAGAGAGGTGCCTCATT | 82 |
| AR-h13 | TTGACATTGGTGAAGGATCGCCAGC | 83 |
| AR-h14 | AGCACACTACACCTGGCTCAATG | 84 |
| AR-h15 | AAACATGGTCCCTGGCAGTCTCCAA | 85 |
| AR-h16 | AACAGATTCTGGAAAGCTCCTCGGTAGGTC | 86 |
| AR-h17 | GAACAGATTCTGGAAAGCTCCTCGGTAGGT | 87 |
| AR-h18 | AACAGATTCTGGAAAGCTCCTCGGTAG | 88 |
| AR-h19 | GAACAGATTCTGGAAAGCTCCTCGGTA | 89 |
| AR-h20 | AACAGATTCTGGAAAGCTCCTCGGT | 90 |
| AR-h21 | GAACAGATTCTGGAAAGCTCCTCGG | 91 |
| AR-h22 | TGGATCACTTCGCGCACGCTCTGGAAC | 92 |
| AR-h23 | TCTGGATCACTTCGCGCACGCTCTGGA | 93 |
| AR-h24 | GTTCTGGATCACTTCGCGCACGCTCTG | 94 |
| AR-h25 | TCTGGATCACTTCGCGCACGCTCTG | 95 |
| AR-h26 | GTTCTGGATCACTTCGCGCACGCTC | 96 |
| AR-h27 | GGATGTCTTTAAGGTCAGCGGAGCAGCTGC | 97 |
| AR-h28 | AGGATGTCTTTAAGGTCAGCGGAGCAGCTG | 98 |
| AR-h29 | CAGGATGTCTTTAAGGTCAGCGGAGCAGCT | 99 |
| AR-h30 | TGTCTTTAAGGTCAGCGGAGCAGCT | 100 |
| AR-h31 | GGATGTCTTTAAGGTCAGCGGAGCAGC | 101 |
| AR-h32 | TCAGGATGTCTTTAAGGTCAGCGGAGCAGC | 102 |
| AR-h33 | AGGATGTCTTTAAGGTCAGCGGAGCAG | 103 |
| AR-h34 | CTCAGGATGTCTTTAAGGTCAGCGGAGCAG | 104 |
| AR-h35 | CAGGATGTCTTTAAGGTCAGCGGAGCA | 105 |
| AR-h36 | GGATGTCTTTAAGGTCAGCGGAGCA | 106 |
| AR-h37 | TCAGGATGTCTTTAAGGTCAGCGGAGC | 107 |
| AR-h38 | AGGATGTCTTTAAGGTCAGCGGAGC | 108 |
| AR-h39 | CTCAGGATGTCTTTTAAGGTCAGCGGAG | 109 |

(continued)

| gRNA | Guide sequence | SEQ ID NO |
|---|---|---|
| AR-h40 | CAGGATGTCTTTAAGGTCAGCGGAG | 110 |
| AR-h41 | TCAGGATGTCTTTAAGGTCAGCGGA | 111 |
| AR-h42 | CTCAGGATGTCTTTTAAGGTCAGCGG | 112 |
| AR-h43 | TAAGTAATTGTCCTTGGAGGAAGTGGGAGC | 113 |
| AR-h44 | CGACACTGCCTTACACAACTCCTTG | 114 |
| AR-h45 | CCCATGGACACCGACACTGCCTTACAC | 115 |
| AR-h46 | CCCATGGACACCGACACTGCCTTAC | 116 |
| AR-h47 | ACTCAGATGCTCCAACGCCTCCACACCCAG | 117 |
| AR-h48 | TCAGATGCTCCAACGCCTCCACACCCA | 118 |
| AR-h49 | CTCAGATGCTCCAACGCCTCCACACCC | 119 |
| AR-h50 | ACTCAGATGCTCCAACGCCTCCACACC | 120 |
| AR-h51 | TCAGATGCTCCAACGCCTCCACACC | 121 |
| AR-h52 | GACTCAGATGCTCCAACGCCTCCACAC | 122 |
| AR-h53 | CTCAGATGCTCCAACGCCTCCACAC | 123 |
| AR-h54 | ACTCAGATGCTCCAACGCCTCCACA | 124 |
| AR-h55 | GACTCAGATGCTCCAACGCCTCCAC | 125 |
| AR-h56 | AGAGAACCTTTGCATTCGGCCAATGGG | 126 |
| AR-h57 | AGAACCTTTGCATTCGGCCAATGGG | 127 |
| AR-h58 | GAGAACCTTTGCATTCGGCCAATGG | 128 |
| AR-h59 | AGAGAACCTTTGCATTCGGCCAATG | 129 |
| AR-h60 | AGCAGAGAACCTTTGCATTCGGCCAAT | 130 |
| AR-h61 | CAGTTCAAGTGTCCCGGAGCTCCCT | 131 |
| AR-h62 | GCAGTTCAAGTGTCCCGGAGCTCCC | 132 |
| AR-h63 | TAGACGGCAGTTCAAGTGTCCCGGA | 133 |
| AR-h64 | AGAGAGACAGGGTAGACGGCAGTTCAA | 134 |
| AR-h65 | AAAGTTGTAGTAGTCGCGACTCTGGTACGC | 135 |
| AR-h66 | GAAAGTTGTAGTAGTCGCCGACTCTGGTACG | 136 |
| AR-h67 | AAAGTTGTAGTAGTCGCGACTCTGGTA | 137 |
| AR-h68 | GAAAGTTGTAGTAGTCGCGACTCTGGT | 138 |
| AR-h69 | AAAGTTGTAGTAGTCGCGACTCTGG | 139 |
| AR-h70 | TGGAAAGTTGTAGTAGTCGCGACTCTG | 140 |
| AR-h71 | GAAAGTTGTAGTAGTCGCGACTCTG | 141 |
| AR-h72 | TGGAAAGTTGTAGTAGTCGCGACTC | 142 |
| AR-h73 | CAGTGGAAAGTTGTAGTAGTCGCGA | 143 |
| AR-h74 | CCCACCACCACCACACGGTCCATACAACTG | 144 |
| AR-h75 | CCACCACCACCACACGGTCCATACAAC | 145 |
| AR-h76 | CCCACCACCACCACACGGTCCATACAA | 146 |
| AR-h77 | CCACCACCACCACACGGTCCATACA | 147 |
| AR-h78 | CCCACCACCACCACACGGTCCATAC | 148 |

(continued)

| gRNA | Guide sequence | SEQ ID NO |
|------|----------------|-----------|
| AR-h79 | GCACTCTGCTCACCATGCCGCCAGG | 149 |
| AR-h80 | TTCATCTCCACAGATCAGGCAGGTC | 150 |
| AR-h81 | CCCAGAAGCTTCATCTCCACAGATCAG | 151 |
| AR-h82 | ACACCCAGAAGCTTCATCTCCACAGATCAG | 152 |
| AR-h83 | ACCCAGAAGCTTCATCTCCACAGATCA | 153 |
| AR-h84 | CCAGAAGCTTCATCTCCACAGATCA | 154 |
| AR-h85 | ACACCCAGAAGCTTCATCTCCACAGAT | 155 |
| AR-h86 | ACCCAGAAGCTTCATCTCCACAGAT | 156 |
| AR-h87 | GACACCCAGAAGCTTCATCTCCACAGA | 157 |
| AR-h88 | ACACCCAGAAGCTTCATCTCCACAG | 158 |
| AR-h89 | AGAGCTCCATAGTGACACCCAGAAGCTTCA | 159 |
| AR-h90 | GAGAGCTCCATAGTGACACCCAGAAGCTTC | 160 |
| AR-h91 | GAGCTCCATAGTGACACCCAGAAGCTT | 161 |
| AR-h92 | TGAGAGCTCCATAGTGACACCCAGAAGCTT | 162 |
| AR-h93 | AGAGCTCCATAGTGACACCCAGAAGCT | 163 |
| AR-h94 | GAGAGCTCCATAGTGACACCCAGAAGC | 164 |
| AR-h95 | GAGCTCCATAGTGACACCCAGAAGC | 165 |
| AR-h96 | TGAGAGCTCCATAGTGACACCCCAGAAG | 166 |
| AR-h97 | AGAGCTCCATAGTGACACCCAGAAG | 167 |
| AR-h98 | GAGAGCTCCATAGTGACACCCAGAA | 168 |
| AR-h99 | TGAGAGCTCCATAGTGACACCCAGA | 169 |
| AR-h100 | TTCCACATGTGAGAGCTCCATAGTG | 170 |
| AR-h101 | AAGAAGACCTTGCAGCTTCCACATGTGAGA | 171 |
| AR-h102 | GAAGAAGACCTTGCAGCTTCCACATGTGAG | 172 |
| AR-h103 | AAGACCTTGCAGCTTCCACATGTGA | 173 |
| AR-h104 | AAGAAGACCTTGCAGCTTCCACATGTG | 174 |
| AR-h105 | TTGAAGAAGACCTTGCAGCTTCCACATGTG | 175 |
| AR-h106 | GAAGAAGACCTTGCAGCTTCCACATGT | 176 |
| AR-h107 | TTTGAAGAAGACCTTGCAGCTTCCACATGT | 177 |
| AR-h108 | TGAAGAAGACCTTGCAGCTTCCACATG | 178 |
| AR-h109 | AAGAAGACCTTGCAGCTTCCACATG | 179 |
| AR-h110 | TTGAAGAAGACCTTGCAGCTTCCACAT | 180 |
| AR-h111 | GAAGAAGACCTTGCAGCTTCCACAT | 181 |
| AR-h112 | TTTGAAGAAGACCTTGCAGCTTCCACA | 182 |
| AR-h113 | TGAAGAAGACCTTGCAGCTTCCACA | 183 |
| AR-h114 | TTGAAGAAGACCTTGCAGCTTCCAC | 184 |
| AR-h115 | TTTGAAGAAGACCTTGCAGCTTCCA | 185 |
| AR-h116 | CAATCATTTCTGCTGGCGCACAGGTACTTC | 186 |
| AR-h117 | GCAATCATTTCTGCTGGCGCACAGGTACTT | 187 |

(continued)

| gRNA | Guide sequence | SEQ ID NO |
|------|---------------|-----------|
| AR-h118 | CAATCATTTCTGCTGGCGCACAGGTAC | 188 |
| AR-h119 | GCAATCATTTCTGCTGGCGCACAGGTA | 189 |
| AR-h120 | AGTGCAATCATTTCTGCTGGCGCACAGGTA | 190 |
| AR-h121 | TGCAATCATTTCTGCTGGCGCACAGGT | 191 |
| AR-h122 | CAATCATTTCTGCTGGCGCACAGGT | 192 |
| AR-h123 | ATAGTGCAATCATTTCTGCTGGCGCACAGG | 193 |
| AR-h124 | GCAATCATTTCTGCTGGCGCACAGG | 194 |
| AR-h125 | AGTGCAATCATTTCTGCTGGCGCACAG | 195 |
| AR-h126 | AATAGTGCAATCATTTCTGCTGGCGCACAG | 196 |
| AR-h127 | TGCAATCATTTCTGCTGGCGCACAG | 197 |
| AR-h128 | GTGCAATCATTTCTGCTGGCGCACA | 198 |
| AR-h129 | ATAGTGCAATCATTTCTGCTGGCGCAC | 199 |
| AR-h130 | AGTGCAATCATTTCTGCTGGCGCAC | 200 |
| AR-h131 | AATAGTGCAATCATTTCTGCTGGCGCA | 201 |
| AR-h132 | ATAGTGCAATCATTTCTGCTGGCGC | 202 |
| AR-h133 | AATAGTGCAATCATTTCTGCTGGCG | 203 |
| AR-h134 | GGAATTTATCAATAGTGCAATCATTTC | 204 |
| AR-h135 | CGGAATTTATCAATAGTGCAATCATTT | 205 |
| AR-h136 | GGAATTTATCAATAGTGCAATCATT | 206 |
| AR-h137 | CGGAATTTATCAATAGTGCAATCAT | 207 |
| AR-h138 | TAACATTTCCGAAGACGACAAGATGGACAA | 208 |
| AR-h139 | ATAACATTTCCGAAGACGACAAGATGGACA | 209 |
| AR-h140 | CATAACATTTCCGAAGACGACAAGATGGAC | 210 |
| AR-h141 | TAACATTTCCGAAGACGACAAGATGGA | 211 |
| AR-h142 | TCATAACATTTCCGAAGACGACAAGATGGA | 212 |
| AR-h143 | ATAACATTTCCGAAGACGACAAGATGG | 213 |
| AR-h144 | TTCATAACATTTCCGAAGACGACAAGATGG | 214 |
| AR-h145 | CATAACATTTCCGAAGACGACAAGATG | 215 |
| AR-h146 | CTTCATAACATTTCCGAAGACGACAAGATG | 216 |
| AR-h147 | TAACATTTCCGAAGACGACAAGATG | 217 |
| AR-h148 | TCATAACATTTCCGAAGACGACAAGAT | 218 |
| AR-h149 | ATAACATTTCCGAAGACGACAAGAT | 219 |
| AR-h150 | TTCATAACATTTCCGAAGACGACAAGA | 220 |
| AR-h151 | CATAACATTTCCGAAGACGACAAGA | 221 |
| AR-h152 | CTTCATAACATTTCCGAAGACGACAAG | 222 |
| AR-h153 | TCATAACATTTCCGAAGACGACAAG | 223 |
| AR-h154 | TTCATAACATTTCCGAAGACGACAA | 224 |
| AR-h155 | CTTCATAACATTTCCGAAGACGACA | 225 |
| AR-h156 | GCTCCCAGAGTCATCCCTGCTTCATAACAT | 226 |

(continued)

| gRNA | Guide sequence | SEQ ID NO |
|------|----------------|-----------|
| AR-h157 | ATTACCAAGTTTCTTCAGCTTCCGG | 227 |
| AR-h158 | CAGATTACCAAGTTTCTTCAGCTTCCG | 228 |
| AR-h159 | TTCAATGTGTGACACTGTCAGCTTC | 229 |
| AR-h160 | ATAGCCTTCAATGTGTGACACTGTCAGCTT | 230 |
| AR-h161 | CATAGCCTTCAATGTGTGACACTGTCAGCT | 231 |
| AR-h162 | ATAGCCTTCAATGTGTGACACTGTCAG | 232 |
| AR-h163 | AGCCTTCAATGTGTGACACTGTCAG | 233 |
| AR-h164 | CATAGCCTTCAATGTGTGACACTGTCA | 234 |
| AR-h165 | TAGCCTTCAATGTGTGACACTGTCA | 235 |
| AR-h166 | ATAGCCTTCAATGTGTGACACTGTC | 236 |
| AR-h167 | CATAGCCTTCAATGTGTGACACTGT | 237 |
| AR-h168 | CATTCAGAAAGATGGGCTGACATTCATAGC | 238 |
| AR-h169 | GACATTCAGAAAGATGGGCTGACATTCATA | 239 |
| AR-h170 | CATTCAGAAAGATGGGCTGACATTCAT | 240 |
| AR-h171 | GGACATTCAGAAAGATGGGCTGACATTCAT | 241 |
| AR-h172 | AGGACATTCAGAAAGATGGGCTGACATTCA | 242 |
| AR-h173 | GACATTCAGAAAGATGGGCTGACATTC | 243 |
| AR-h174 | CATTCAGAAAGATGGGCTGACATTC | 244 |
| AR-h175 | GGACATTCAGAAAGATGGGCTGACATT | 245 |
| AR-h176 | AGGACATTCAGAAAGATGGGCTGACAT | 246 |
| AR-h177 | GACATTCAGAAAGATGGGCTGACAT | 247 |
| AR-h178 | GGACATTCAGAAAGATGGGCTGACA | 248 |
| AR-h179 | AGGACATTCAGAAAGATGGGCTGAC | 249 |
| AR-h180 | AGCACACTACACCTGGCTCAATGGC | 250 |
| AR-h181 | AGCACACTACACCTGGCTCAATG | 251 |
| AR-h182 | CTGGTTGTTGTCGTGTCCAGCACAC | 252 |
| AR-h183 | TTCATTGAGGCTAGAGAGCAAGGCTGCAAA | 253 |
| AR-h184 | GTTCATTGAGGCTAGAGAGCAAGGCTGCAA | 254 |
| AR-h185 | AGTTCATTGAGGCTAGAGAGCAAGGCTGCA | 255 |
| AR-h186 | ATTGAGGCTAGAGAGCAAGGCTGCA | 256 |
| AR-h187 | TTCATTGAGGCTAGAGAGCAAGGCTGC | 257 |
| AR-h188 | GTTCATTGAGGCTAGAGAGCAAGGCTG | 258 |
| AR-h189 | AGTTCATTGAGGCTAGAGAGCAAGGCT | 259 |
| AR-h190 | TTCATTGAGGCTAGAGAGCAAGGCT | 260 |
| AR-h191 | GTTCATTGAGGCTAGAGAGCAAGGC | 261 |
| AR-h192 | AGTTCATTGAGGCTAGAGAGCAAGG | 262 |
| AR-h193 | GTACAAGCTGTCTCTCTCCCAGTTCATTGA | 263 |
| AR-h194 | TGTACAAGCTGTCTCTCTCCCAGTTCATTG | 264 |
| AR-h195 | TACAAGCTGTCTCTCTCCCAGTTCATT | 265 |

(continued)

| gRNA | Guide sequence | SEQ ID NO |
|------|----------------|-----------|
| AR-h196 | GTGTACAAGCTGTCTCTTCTCCCAGTTCATT | 266 |
| AR-h197 | GTACAAGCTGTCTCTCTCCCAGTTCAT | 267 |
| AR-h198 | CGTGTACAAGCTGTCTCTCTCCCAGTTCAT | 268 |
| AR-h199 | TGTACAAGCTGTCTCTCTCCCAGTTCA | 269 |
| AR-h200 | ACGTGTACAAGCTGTCTCTCTCCCAGTTCA | 270 |
| AR-h201 | TACAAGCTGTCTCTCTCCCAGTTCA | 271 |
| AR-h202 | GTGTACAAGCTGTCTCTTCTCCCAGTTC | 272 |
| AR-h203 | CACGTGTACAAGCTGTCTCTCTCCCAGTTC | 273 |
| AR-h204 | GTACAAGCTGTCTCTCTCCCAGTTC | 274 |
| AR-h205 | CGTGTACAAGCTGTCTCTCTCCCAGTT | 275 |
| AR-h206 | TGTACAAGCTGTCTCTCTCCCAGTT | 276 |
| AR-h207 | ACGTGTACAAGCTGTCTCTCTCCCAGT | 277 |
| AR-h208 | ACCACGTGTACAAGCTGTCTCTCTCCCAGT | 278 |
| AR-h209 | GTGTACAAGCTGTCTCTTCTCCCAGT | 279 |
| AR-h210 | CACGTGTACAAGCTGTCTCTCTCCCAG | 280 |
| AR-h211 | GACCACGTGTACAAGCTGTCTCTCTCCCAG | 281 |
| AR-h212 | CGTGTACAAGCTGTCTCTCTCCCAG | 282 |
| AR-h213 | TGACCACGTGTACAAGCTGTCTCTCTCCCA | 283 |
| AR-h214 | ACGTGTACAAGCTGTCTCTCTCCCA | 284 |
| AR-h215 | ACCACGTGTACAAGCTGTCTCTCTCCC | 285 |
| AR-h216 | CACGTGTACAAGCTGTCTCTCTCCC | 286 |
| AR-h217 | GACCACGTGTACAAGCTGTCTCTCTCC | 287 |
| AR-h218 | TGACCACGTGTACAAGCTGTCTCTCTC | 288 |
| AR-h219 | ACCACGTGTACAAGCTGTCTCTCTC | 289 |
| AR-h220 | GACCACGTGTACAAGCTGTCTCTCT | 290 |
| AR-h221 | TGACCACGTGTACAAGCTGTCTCTC | 291 |
| AR-h222 | CACGTGTAAGTTGCGGAAGCCAGGCAA | 292 |
| AR-h223 | TCGTCCACGTGTAAGTTGCGGAAGCCA | 293 |
| AR-h224 | ACAGCCATCTGGTCGTCCACGTGTAAG | 294 |
| AR-h225 | ACAGCCATCTGGTCGTCCACGTGTA | 295 |
| AR-h226 | AATGACAGCCATCTGGTCGTCCACGTG | 296 |
| AR-h227 | AATGACAGCCATCTGGTCGTCCACG | 297 |
| AR-h228 | AGTACTGAATGACAGCCATCTGGTCGTCCA | 298 |
| AR-h229 | AGTACTGAATGACAGCCATCTGGTCGT | 299 |
| AR-h230 | GTACTGAATGACAGCCATCTGGTCG | 300 |
| AR-h231 | AGTACTGAATGACAGCCATCTGGTC | 301 |
| AR-h232 | CCAGGAGTACTGAATGACAGCCATCTG | 302 |
| AR-h233 | AGGAGTACTGAATGACAGCCATCTG | 303 |
| AR-h234 | CAGGAGTACTGAATGACAGCCATCT | 304 |

(continued)

| gRNA | Guide sequence | SEQ ID NO |
|------|----------------|-----------|
| AR-h235 | CCAGGAGTACTGAATGACAGCCATC | 305 |
| AR-h236 | AGTTGACATTGGTGAAGGATCGCCA | 306 |
| AR-h237 | AACTCTTGAGAGAGGTGCCTCATTCGGACA | 307 |
| AR-h238 | AAACTCTTGAGAGAGGTGCCTCATTCGGAC | 308 |
| AR-h239 | CAAACTCTTGAGAGAGGTGCCTCATTCGGA | 309 |
| AR-h240 | CCAAACTCTTGAGAGAGGTGCCTCATTCGG | 310 |
| AR-h241 | AAACTCTTGAGAGAGGTGCCTCATTCG | 311 |
| AR-h242 | CAAACTCTTGAGAGAGGTGCCTCATTC | 312 |
| AR-h243 | CCAAACTCTTGAGAGAGGTGCCTCATT | 313 |
| AR-h244 | AAACTCTTGAGAGAGGTGCCTCATT | 314 |
| AR-h245 | CAAACTCTTGAGAGAGGTGCCTCAT | 315 |
| AR-h246 | CCAAACTCTTGAGAGAGGTGCCTCA | 316 |
| AR-h247 | AGTAGCAGTGCTTTCATGCACAGGAAT | 317 |
| AR-h248 | AATGCTGAAGAGTAGCAGTGCTTTCATGCA | 318 |
| AR-h249 | AATGCTGAAGAGTAGCAGTGCTTTCAT | 319 |
| AR-h250 | AATAATGCTGAAGAGTAGCAGTGCTTTCAT | 320 |
| AR-h251 | GAATAATGCTGAAAGAGTAGCAGTGCTTTCA | 321 |
| AR-h252 | GGAATAATGCTGAAGAGTAGCAGTGCTTTC | 322 |
| AR-h253 | AATGCTGAAGAGTAGCAGTGCTTTC | 323 |
| AR-h254 | AATAATGCTGAAGAGTAGCAGTGCTTT | 324 |
| AR-h255 | GAATAATGCTGAAGAGTAGCAGTGCTT | 325 |
| AR-h256 | GGAATAATGCTGAAGAGTAGCAGTGCT | 326 |
| AR-h257 | AATAATGCTGAAGAGTAGCAGTGCT | 327 |
| AR-h258 | GAATAATGCTGAAAGAGTAGCAGTGC | 328 |
| AR-h259 | GGAATAATGCTGAAGAGTAGCAGTG | 329 |
| AR-h260 | TGCAATGATACGATCGAGTTCCTTGATGTA | 330 |
| AR-h261 | TGCAATGATACGATCGAGTTCCTTGAT | 331 |
| AR-h262 | TGCATGCAATGATACGATCGAGTTCCTTGA | 332 |
| AR-h263 | TTGCATGCAATGATACGATCGAGTTCCTTG | 333 |
| AR-h264 | TGCAATGATACGATCGAGTTCCTTG | 334 |
| AR-h265 | TTTGCATGCAATGATACGATCGAGTTCCTT | 335 |
| AR-h266 | TGCATGCAATGATACGATCGAGTTCCT | 336 |
| AR-h267 | TTGCATGCAATGATACGATCGAGTTCC | 337 |
| AR-h268 | TTTGCATGCAATGATACGATCGAGTTC | 338 |
| AR-h269 | TGCATGCAATGATACGATCGAGTTC | 339 |
| AR-h270 | TTGCATGCAATGATACGATCGAGTT | 340 |
| AR-h271 | TTTGCATGCAATGATACGATCGAGT | 341 |
| AR-h272 | AAGTGAACTGATGCAGCTCTCTCGCAATAG | 342 |
| AR-h273 | AAAGTGAACTGATGCAGCTCTCTCGCAATA | 343 |

(continued)

| gRNA | Guide sequence | SEQ ID NO |
|------|----------------|-----------|
| AR-h274 | AAGTGAACTGATGCAGCTCTCTCGCAA | 344 |
| AR-h275 | AAAGTGAACTGATGCAGCTCTCTCGCA | 345 |
| AR-h276 | AAAGTGAACTGATGCAGCTCTCTCG | 346 |
| AR-h277 | ATTTCCGGAAAGTCCACGCTCACCATGTGT | 347 |
| AR-h278 | TTCCGGAAAGTCCACGCTCACCATGTG | 348 |
| AR-h279 | CATTTCCGGAAAGTCCACGCTCACCATGTG | 349 |
| AR-h280 | TTTCCGGAAAGTCCACGCTCACCATGT | 350 |
| AR-h281 | TCATTTCCGGAAAGTCCACGCTCACCATGT | 351 |
| AR-h282 | ATTTCCGGAAAGTCCACGCTCACCATG | 352 |
| AR-h283 | ATCATTTCCGGAAAGTCCACGCTCACCATG | 353 |
| AR-h284 | TTCCGGAAAGTCCACGCTCACCATG | 354 |
| AR-h285 | CATTTCCGGAAAGTCCACGCTCACCAT | 355 |
| AR-h286 | CATCATTTCCGGAAAGTCCACGCTCACCAT | 356 |
| AR-h287 | TTTCCGGAAAGTCCACGCTCACCAT | 357 |
| AR-h288 | TCATTTCCGGAAAGTCCACGCTCACCA | 358 |
| AR-h289 | CCATCATTTCCGGAAAGTCCACGCTCACCA | 359 |
| AR-h290 | ATTTCCGGAAAGTCCACGCTCACCA | 360 |
| AR-h291 | ATCATTTCCGGAAAGTCCACGCTCACC | 361 |
| AR-h292 | CATTTCCGGAAAGTCCACGCTCACC | 362 |
| AR-h293 | CATCATTTCCGGAAAGTCCACGCTCAC | 363 |
| AR-h294 | TCATTTCCGGAAAGTCCACGCTCAC | 364 |
| AR-h295 | CCATCATTTCCGGAAAGTCCACGCTCA | 365 |
| AR-h296 | ATCATTTCCGGAAAGTCCACGCTCA | 366 |
| AR-h297 | CATCATTTCCGGAAAGTCCACGCTC | 367 |
| AR-h298 | CCATCATTTCCGGAAAGTCCACGCT | 368 |
| AR-h299 | CAGAAAGGATCTTGGGCACTTGCACAGAGA | 369 |
| AR-h300 | CCAGAAAGGATCTTGGGCACTTGCACAGAG | 370 |
| AR-h301 | CCCAGAAAGGATCTTGGGCACTTGCACAGA | 371 |
| AR-h302 | CAGAAAGGATCTTGGGCACTTGCACAG | 372 |
| AR-h303 | CCAGAAAGGATCTTGGGCACTTGCACA | 373 |
| AR-h304 | CCCAGAAAGGATCTTGGGCACTTGCAC | 374 |
| AR-h305 | CAGAAAGGATCTTGGGCACTTGCAC | 375 |
| AR-h306 | CCCAGAAAGGATCTTGGGCACTTGC | 376 |
| AR-h307 | AAATAGATGGGCTTGACTTTCCCAGAAAGG | 377 |
| AR-h308 | AAATAGATGGGCTTGACTTTCCCAGAA | 378 |

In some embodiments of this disclosure, the guide sequence of the guide RNA has at least 80%, at least 85%, at least 90%, at least 95% or 100% sequence identity with the sequence shown in any one of SEQ ID NOs: 71-378.

In some embodiments of this disclosure, the guide sequence of the guide RNA has at least 80%, at least 85%, at least 90%, at least 95% or 100% sequence identity with the sequence shown in any one of SEQ ID NOs: 71-80.

In some embodiments of this disclosure, the guide sequence of the guide RNA has 100% sequence identity with the sequence shown in any one of SEQ ID NOs: 71-80.

In some embodiments of this disclosure, the guide RNA comprises a guide sequence and a direct repeat sequence, the direct repeat sequence interacting with an RNA-guided nuclease.

In some embodiments of this disclosure, the RNA-guided nuclease is a Cas protein; alternatively, the RNA-guided nuclease is a Cas9 protein, a Cas12 protein, or a Cas13 protein.

In some embodiments of this disclosure, the RNA-guided nuclease is a Cas13 protein. Further optionally, the RNA-guided nuclease is a Cas13a protein, a Cas13b protein, a Cas13c protein, or a Cas13d protein.

In some embodiments of this disclosure, the Cas13 protein comprises the sequence shown in SEQ ID NO: 1.

In some embodiments of this disclosure, the RNA-guided nuclease comprises any one or more of the following: subcellular localization signal, deaminase domain, translation activation domain, translation repression domain, RNA methylation domain, RNA demethylation domain, nuclease domain, splicing factor domain, reporter tag, and affinity tag.

In some embodiments of this disclosure, the subcellular localization signal is a nuclear localization signal and/or a nuclear output signal sequence.

In some embodiments disclosed herein, the subcellular localization signal is a mitochondrial localization signal or a chloroplast localization signal sequence.

In some embodiments of this disclosure, the RNA-guided nuclease comprises a nuclear localization signal and/or nuclear output signal sequence, and optionally a deaminase domain, a translation activation domain, or a translation repression domain.

In some embodiments of this disclosure, the polynucleotide sequence encoding the RNA-guided nuclease is linked to a regulatory sequence 1 that regulates its expression, and the polynucleotide sequence encoding the guide RNA is linked to a regulatory sequence 2 that regulates its expression.

In some embodiments of this disclosure, the regulatory sequence is a promoter sequence. In some embodiments of this disclosure, the regulatory sequence is an enhancer sequence. In some embodiments of this disclosure, the regulatory sequence is both a promoter and an enhancer sequence.

In some embodiments of this disclosure, the regulatory sequence is selected from CMV promoter, CMV enhancer, CBh promoter, U6 promoter and specific promoter.

In some embodiments of this disclosure, the gene editing system comprises:

a guide RNA comprising a guide sequence that hybridizes with AR RNA or a polynucleotide sequence encoding the guide RNA, and a Cas13 protein or a polynucleotide sequence encoding the Cas13 protein; wherein the guide RNA is capable of forming a CRISPR complex with the Cas13 protein and directing the complex to bind specifically to and cleave AR RNA.

In some embodiments, the coding sequence is linked to a regulatory sequence that regulates its expression.

In some embodiments, the polynucleotide sequence encoding the Cas13 protein is linked to a regulatory sequence 1 that regulates its expression, and the polynucleotide sequence encoding the guide RNA is linked to a regulatory sequence 2 that regulates its expression.

In some embodiments, at least two(e.g., 2, 3, 4, 5, 6, 7, 8, 9, 10, 11, 12, 13, 14, 15 or more) guide RNAs are expressed in tandem under the regulation of the same regulatory sequence. Further, tandem expression yields a pre-cRNA, which is then processed by the Cas13 protein to obtain a mature guide RNA molecule.

In some embodiments, the polynucleotide sequence encoding the guide RNA and the polynucleotide sequence encoding the RNA-guided nuclease are located on the same vector.

In some embodiments of this disclosure, the gene editing system is introduced into a cell or cell-free system in any of the following ways: (i) as mRNA encoding an RNA-guided nuclease and guide RNA,(ii) as part of a single vector or plasmid, or divided into multiple vectors or plasmids,(iii) as a separate RNA-guided nuclease and guide RNA, or(iv) as an RNP complex of an RNA-guided nuclease and guide RNA.

The nineteenth aspect of this disclosure provides a gene editing system, the gene editing system:

Knock down AR RNA levels or inhibit AR RNA translation.

In some embodiments of this disclosure, the gene editing system knocks down AR RNA levels or inhibits AR RNA translation.

In some embodiments of this disclosure, the gene editing system knocks down AR RNA levels.

In some embodiments of this disclosure, the gene editing system inhibits the translation of AR RNA.

In some embodiments of this disclosure, the gene editing system comprises:

a guide RNA comprising a guide sequence that hybridizes with AR RNA, or a polynucleotide sequence encoding the guide RNA; and an RNA-guided nuclease, or a polynucleotide sequence encoding the nuclease; wherein the guide RNA forms a complex with the nuclease and guids the complex to bind specifically to the AR RNA sequence.

In some embodiments of this disclosure, the gene editing system comprises:

a guide RNA comprising a guide sequence that hybridizes with AR RNA, and an RNA-guided nuclease;
wherein the guide RNA is capable of forming a complex with the nuclease and guiding the complex to bind specifically to the AR RNA sequence.

In some embodiments of this disclosure, the gene editing system comprises:

A polynucleotide sequence encoding a guide RNA comprising a guide sequence that hybridizes with AR RNA, and a polynucleotide sequence encoding an RNA-guided nuclease;
wherein the guide RNA is capable of forming a complex with the nuclease and guiding the complex to bind specifically to the AR RNA sequence.

In some embodiments of this disclosure, the AR RNA is pre-mRNA and/or mature mRNA.

In some embodiments of this disclosure, the AR RNA is human pre-mRNA and/or mature mRNA.

In some embodiments of this disclosure, the AR RNA sequence is selected from any of the sequences shown in SEQ ID NOs: 394-396.

In some embodiments of this disclosure, the AR RNA is the sequence numbered NM_000044.6(SEQ ID NO: 394) in the NCBI database, namely "transcript variant 1".

In some embodiments of this disclosure, the AR RNA is the sequence numbered NM_001011645.3(SEQ ID NO: 395) in the NCBI database, namely "transcript variant 2".

In some embodiments of this disclosure, the AR RNA is the sequence numbered ENST00000374690.9(SEQ ID NO: 396) in the Ensembl database.

In some embodiments of this disclosure, the guide RNA directs the complex to bind to and cleave the AR RNA.

In some embodiments of this disclosure, the complex, upon contact with a cell containing AR RNA, reduces the level of the AR RNA or AR protein in the cell by at least 5%, at least 10%, at least 15 %, at least 20 %, at least 25 %, at least 30%, at least 35%, at least 40 %, at least 50 %, at least 60%, at least 70%, at least 80%, at least 90 %, at least 95%, at least 96%, at least 97%, at least 98%, or at least 99%.

In some embodiments of this disclosure, the complex, upon contact with a cell containing AR RNA, reduces the level of the AR RNA in the cell by at least 5%, at least 10%, at least 15%, at least 20%, at least 25 %, at least 30%, at least 35%, at least 40 %, at least 50 %, at least 60%, at least 70%, at least 80%, at least 90%, at least 95%, at least 96%, at least 97%, at least 98%, or at least 99%.

In some embodiments of this disclosure, the complex, upon contact with a cell containing AR RNA, reduces the level of the AR protein in the cell by at least 5%, at least 10%, at least 15%, at least 20%, at least 25 %, at least 30%, at least 35%, at least 40 %, at least 50 %, at least 60%, at least 70%, at least 80%, at least 90%, at least 95%, at least 96%, at least 97%, at least 98%, or at least 99%.

In some embodiments of this disclosure, the cell is a eukaryotic cell. In some embodiments of this disclosure, the cell is a mammalian cell. In some embodiments of this disclosure, the cell is a human cell.

The reduction in the RNA levels can be tested using methods conventional in the art, including but not limited to qPCR methods such as RT-qPCR. The levels of target RNA(the AR RNA) in untreated cells or cells treated with a gene-editing system targeting a non-mammalian genome can be tested as a negative control, and the target RNA knockdown level in the experimental group compared to the negative control can be calculated. Differences in target RNA levels can be compared between the experimental group and the negative control group after editing with the same Cas protein(e.g., by expressing the same Cas protein) and gRNAs containing different guide sequences; for example, the experimental group can be edited using C13-2 and a gRNA as claimed in this disclosure, and the negative control group can be edited using C13-2 and a gRNA targeting, for example, a bacterial genome. The experimental group can also be compared with known editing tools, such as CasRx+gRNA editing tools.

In some embodiments of this disclosure, the number of off-target genes when the complex binds to and cleaves the target RNA(the AR RNA) is less than 40, less than 35, less than 30, less than 25, less than 20, less than 19, less than 18, less than 17, less than 16, less than 15, less than 14, less than 13, less than 12, less than 11, less than 10, less than 9, less than 8, less than 7, less than 6, less than 5, less than 4, less than 3, less than 2, or less than 1. The number of off-target genes can be determined by conventional methods in the art. In some embodiments, the number of off-target genes is determined by taking the intersection of the differentially expressed gene set determined by RNA sequencing and the off-target gene set predicted by the program. In some embodiments, the number of off-target genes is determined by taking the intersection of the differentially expressed gene set with downregulated expression determined by RNA sequencing and the off-target gene set predicted by the program. Off-target genes can be predicted using off-target gene prediction programs known in the art under conventional parameter settings. For example, the method of prediction by the program is as follows: using the EMBOSS-water program to predict the whole genome and whole cDNA sequence of the target species(e.g., Homo sapiens or Mus musculus, etc.), setting the parameters to gap_extend=0.5 & gap_extend=10, comparing the forward and reverse strands of the gRNA-guided sequence, filtering the prediction results, and obtaining the predicted potential target genes(including target genes and/or off-target genes).

The reduction in the level of the target RNA(the AR RNA) encoded protein can be tested using conventional methods in the art; including but not limited to ELISA, Western blotting, and untreated cells or cells treated with gene editing systems targeting non-mammal genomes as negative controls, and the level of AR protein in the experimental group compared to the negative control group can be calculated.

In some embodiments of this disclosure, the guide sequence of the guide RNA has at least 80%, at least 85%, at least 90%, at least 95% or 100% sequence identity with the sequence shown in any one of SEQ ID NOs: 394-396.

In some embodiments of this disclosure, the guide sequence of the guide RNA has 100% sequence identity with the sequence shown in any one of SEQ ID NOs: 394-396.

In some embodiments of this disclosure, the guide sequence of the guide RNA has at least 80%, at least 85%, at least 90%, at least 95% or 100% sequence identity with the sequence shown in SEQ ID NO: 394.

In some embodiments of this disclosure, the guide sequence of the guide RNA has 100% sequence identity with the sequence shown in SEQ ID NO: 394.

In some embodiments of this disclosure, the guide sequence of the guide RNA has at least 80%, at least 85%, at least 90%, at least 95 % or 100% sequence identity with the nucleotide sequence consisting of nucleotides 2826-2990 of the sequence shown in SEQ ID NO: 394.

In some embodiments of this disclosure, the guide sequence of the guide RNA has at least 80%, at least 85%, at least 90%, at least 95% or 100% sequence identity with the sequence shown in any one of SEQ ID NOs: 71-378.

In some embodiments of this disclosure, the guide sequence of the guide RNA has at least 80%, at least 85%, at least 90%, at least 95% or 100% sequence identity with the sequence shown in any one of SEQ ID NOs: 71-80.

In some embodiments of this disclosure, the guide sequence of the guide RNA has 100% sequence identity with the sequence shown in any one of SEQ ID NOs: 71-80.

In some embodiments of this disclosure, the guide RNA comprises a guide sequence and a direct repeat sequence, the direct repeat sequence interacting with an RNA-guided nuclease.

In some embodiments of this disclosure, the RNA-guided nuclease is a Cas protein; alternatively, the RNA-guided nuclease is a Cas9 protein, a Cas12 protein, or a Cas13 protein.

In some embodiments of this disclosure, the RNA-guided nuclease is a Cas13 protein. Further optionally, the RNA-guided nuclease is a Cas13a protein, a Cas13b protein, a Cas13c protein, or a Cas13d protein.

In some embodiments of this disclosure, the Cas13 protein comprises the sequence shown in SEQ ID NO: 1.

In some embodiments of this disclosure, the RNA-guided nuclease comprises any one or more of the following: subcellular localization signal, deaminase domain, translation activation domain, translation repression domain, RNA methylation domain, RNA demethylation domain, nuclease domain, splicing factor domain, reporter tag, and affinity tag.

In some embodiments of this disclosure, the subcellular localization signal is a nuclear localization signal and/or a nuclear output signal sequence.

In some embodiments disclosed herein, the subcellular localization signal is a mitochondrial localization signal or a chloroplast localization signal sequence.

In some embodiments of this disclosure, the RNA-guided nuclease comprises a nuclear localization signal and/or nuclear output signal sequence, and optionally a deaminase domain, a translation activation domain, or a translation repression domain.

In some embodiments of this disclosure, the polynucleotide sequence encoding the RNA-guided nuclease is linked to a regulatory sequence 1 that regulates its expression, and the polynucleotide sequence encoding the guide RNA is linked to a regulatory sequence 2 that regulates its expression.

In some embodiments of this disclosure, the regulatory sequence is a promoter sequence. In some embodiments of this disclosure, the regulatory sequence is an enhancer sequence. In some embodiments of this disclosure, the regulatory sequence is both a promoter and an enhancer sequence.

In some embodiments of this disclosure, the regulatory sequence is selected from CMV promoter, CMV enhancer, CBh promoter, U6 promoter and specific promoter.

In some embodiments of this disclosure, the gene editing system comprises:

a guide RNA comprising a guide sequence that hybridizes with AR RNA or a polynucleotide sequence encoding the guide RNA, and a Cas13 protein or a polynucleotide sequence encoding the Cas13 protein;
wherein the guide RNA is able to form a CRISPR complex with the Cas13 protein and guide the complex to bind specifically to and cleave AR RNA.

In some embodiments, the coding sequence is linked to a regulatory sequence that regulates its expression.

In some embodiments, the polynucleotide sequence encoding the Cas13 protein is linked to a regulatory sequence 1 that regulates its expression, and the polynucleotide sequence encoding the guide RNA is linked to a regulatory sequence 2 that regulates its expression.

In some embodiments, at least two(e.g., 2, 3, 4, 5, 6, 7, 8, 9, 10, 11, 12, 13, 14, 15 or more) guide RNAs are expressed tandemly under the regulation of the same regulatory sequence. Further, tandem expression yields a pre-cRNA, which is then processed by the Cas13 protein to obtain a mature guide RNA molecule.

In some embodiments, the polynucleotide sequence encoding the guide RNA and the polynucleotide sequence encoding the RNA-guided nuclease are located on the same vector.

In some embodiments of this disclosure, the gene editing system is introduced into a cellular or cell-free system in any of the following ways: (i) as mRNA encoding an RNA-guided nuclease and guide RNA,(ii) as part of a single vector or plasmid, or divided into multiple vectors or plasmids,(iii) as a separate RNA-guided nuclease and guide RNA, or(iv) as an RNP complex of an RNA-guided nuclease and guide RNA.

The twentieth aspect of this disclosure provides a guide RNA(gRNA) for a gene editing system, which contains a guide sequence that hybridizes with AR RNA.

In some embodiments of this disclosure, the AR RNA is pre-mRNA or mature mRNA. In some embodiments of this disclosure, the AR RNA is mature mRNA.

In some embodiments of this disclosure, the AR RNA is human AR RNA. In some embodiments of this disclosure, the AR RNA is human mRNA.

In some embodiments of this disclosure, the guide sequence of the guide RNA has at least 80%, at least 85%, at least 90%, at least 95% or 100% sequence identity with the sequence shown in any one of SEQ ID NOs: 394-396.

In some embodiments of this disclosure, the guide sequence of the guide RNA has 100% sequence identity with the sequence shown in any one of SEQ ID NOs: 394-396.

In some embodiments of this disclosure, the guide sequence of the guide RNA has at least 80%, at least 85%, at least 90%, at least 95% or 100% sequence identity with the sequence shown in SEQ ID NO: 394.

In some embodiments of this disclosure, the guide sequence of the guide RNA has 100% sequence identity with the sequence shown in SEQ ID NO: 394.

In some embodiments of this disclosure, the guide sequence of the guide RNA has at least 80%, at least 85%, at least 90%, at least 95 % or 100% sequence identity with the nucleotide sequence consisting of nucleotides 2826-2990 of the sequence shown in SEQ ID NO: 394.

In some embodiments of this disclosure, the guide RNA has a guide sequence that has at least 80%, at least 85%, at least 90%, at least 95% or 100% sequence identity with the sequence shown in any one of SEQ ID NOs : 71-378.

In some embodiments of this disclosure, the guide sequence of the guide RNA has at least 80%, at least 85%, at least 90%, at least 95% or 100% sequence identity with the sequence shown in any one of SEQ ID NOs: 71-80.

In some embodiments of this disclosure, the guide sequence of the guide RNA has 100% sequence identity with the sequence shown in any one of SEQ ID NOs: 71-80.

In some embodiments of this disclosure, the guide RNA is capable of forming a complex with an RNA-guided nuclease and directing the complex to bind specifically to the AR RNA sequence.

In some embodiments of this disclosure, the guide RNA is capable of forming a complex with the nuclease and directing the complex to bind to and cleave the AR RNA.

In some embodiments of this disclosure, the complex reduces the level of the AR RNA in mammals, such as humans.

In some embodiments of this disclosure, the complex, upon contact with a cell containing AR RNA, reduces the level of the AR RNA in the cell. For example, the level of target RNA in cells expressing target RNA.

In some embodiments of this disclosure, the complex reduces the level of the AR RNA in cells by at least 5%, at least 10%, at least 15%, at least 20%, at least 25%, at least 30 %, at least 35%, at least 40%, at least 50%, at least 60 %, at least 70%, at least 80%, at least 90%, at least 95 %, at least 96 %, at least 97%, at least 98%, or at least 99%. The reduction in the AR RNA level can be tested using methods conventional in the art; including but not limited to the qPCR method described in the examples, using untreated cells or cells treated with a gene-editing system targeting a non-mammalian genome as a negative control, and calculating the AR RNA knockdown level of the experimental group compared to the negative control.

In some embodiments of the present invention, the number of off-target genes when the complex binds to and cleaves the AR RNA is less than 40, less than 35, less than 30, less than 25, less than 20, less than 19, less than 18, less than 17, less than 16, less than 15, less than 14, less than 13, less than 12, less than 11, less than 10, less than 9, less than 8, less than 7, less than 6, less than 5, less than 4, less than 3, less than 2, or less than 1. The number of off-target genes can be determined by conventional methods in the art. In some embodiments, the number of off-target genes is determined by taking the intersection of the differentially expressed gene set determined by RNA sequencing and the off-target gene set predicted by the program. For example, the method of prediction by the program is as follows: the EMBOSS-water program is used to predict the whole genome and whole cDNA sequence of the target species(Homo sapiens/Mus musculus), with the parameters set as gap_extend=0.5 & gap_extend=10. The forward and reverse strands of the gRNA-guided sequence are compared, and the prediction results are filtered to obtain the predicted potential target genes(including target genes and off-target genes).

In some embodiments of this disclosure, the complex reduces the level of the AR RNA-encoded protein in an animal(such as a human).

In some embodiments of this disclosure, the complex, upon contact with a cell containing the target RNA, reduces the level of the AR RNA-encoded protein in the cell. In some embodiments of this disclosure, the AR RNA-encoded protein is an AR protein. In some embodiments of this disclosure, the complex reduces the level of the AR protein in the cell.

In some embodiments of this disclosure, the complex reduces AR protein levels in cells by at least 5%, at least 10 %, at least 15%, at least 20%, at least 25%, at least 30 %, at least 35%, at least 40%, at least 50%, at least 60%, at least 70%, at least 80%, at least 90%, at least 95 %, at least 96%, at least 97%, at least 98%, or at least 99%. The reduction in AR RNA-encoded protein levels can be tested using methods conventional in the art, including but not limited to ELISA and Western blotting. Untreated cells or cells treated with gene-editing systems targeting non-mammalian genomes can be used as negative controls to calculate the AR protein knockdown level in the experimental group compared to the negative control group.

In some embodiments of this disclosure, the guide RNA comprises a guide sequence and a scaffold sequence, the scaffold sequence interacting with an RNA-guided nuclease. In some embodiments of this disclosure, the scaffold sequence is a direct repeat(DR) sequence.

The twenty-first aspect of this disclosure provides an isolated nucleic acid that encodes a guide RNA according to any one of the claims of this disclosure.

A twenty-second aspect of this disclosure provides a vector comprising a polynucleotide sequence encoding a guide RNA according to any one of the present disclosures, and a regulatory sequence for regulating the expression of the guide RNA.

In some embodiments of this disclosure, the vector is an adeno-associated virus vector.

In some embodiments of this disclosure, the regulatory sequence is a promoter sequence. In some embodiments of this disclosure, the regulatory sequence is an enhancer sequence. In some embodiments of this disclosure, the regulatory sequence is both a promoter and an enhancer sequence.

In some embodiments of this disclosure, the regulatory sequence is selected from CMV promoter, CMV enhancer, CBh promoter, U6 promoter and specific promoter.

In some embodiments of this disclosure, the promoter is a chicken β-actin(CB) promoter. The chicken β-actin promoter may be a short chicken β-actin promoter or a long chicken β-actin promoter. In some embodiments, the promoter(e.g., a chicken β-actin promoter) includes an enhancer sequence, such as a cytomegalovirus(CMV) enhancer sequence. The CMV enhancer sequence may be a short CMV enhancer sequence or a long CMV enhancer sequence. In some embodiments, the promoter includes a long CMV enhancer sequence and a long chicken β-actin promoter. In some embodiments, the promoter includes a short CMV enhancer sequence and a short chicken β-actin promoter. However, those skilled in the art will recognize that a short CMV enhancer can be used with a long CB promoter, and a long CMV enhancer can be used with a short CB promoter. In some embodiments of this disclosure, the promoter is a CBh promoter.

In some embodiments of this disclosure, the promoter is the CBh promoter.

In some embodiments of this disclosure, the regulatory sequence includes an HRE enhancer element.

In some embodiments of this disclosure, the regulatory sequence comprises a series of NRS elements and an HRE enhancer element.

The twenty-third aspect of this disclosure provides a vector system comprising a polynucleotide sequence encoding the guide RNA disclosed herein and a second regulatory sequence regulating the expression of the guide RNA; and a polynucleotide sequence encoding the RNA-guided nuclease and a first regulatory sequence regulating the expression of the RNA-guided nuclease.

In some embodiments of this disclosure, the delivery system includes one or more vectors.

In some embodiments of this disclosure, the vector system comprises multiple vectors, wherein a polynucleotide sequence encoding the guide RNA and a second regulatory sequence regulating the expression of the guide RNA are located on a second vector, and a polynucleotide sequence encoding the RNA-guided nuclease and a first regulatory sequence regulating the expression of the RNA-guided nuclease are located on a first vector.

In some embodiments of this disclosure, the regulatory sequence is a promoter sequence. In some embodiments of this disclosure, the regulatory sequence is an enhancer sequence. In some embodiments of this disclosure, the regulatory sequence is both a promoter and an enhancer sequence.

The twenty-fourth aspect of this disclosure provides an adeno-associated virus vector, wherein the adeno-associated virus vector comprises an RNA-guided nuclease encoding the RNA and DNA of the directing RNA described in this disclosure.

The twenty-fifth aspect of this disclosure provides a lipid nanoparticle, wherein the lipid nanoparticle comprises the guide RNA described in this disclosure and an mRNA encoding a nuclease guided by the RNA.

The twenty-sixth aspect of this disclosure provides a lentiviral vector, wherein the lentiviral vector comprises the guide RNA described in this disclosure and mRNA encoding an RNA-guided nuclease; optionally, the lentiviral vector is pseudotyped with an envelope protein; optionally, the mRNA encoding the RNA-guided nuclease is linked to an aptamer sequence.

The twenty-seventh aspect of this disclosure provides a ribonucleoprotein complex, wherein the ribonucleoprotein complex is formed by the guide RNA and RNA-guided nuclease described in this disclosure.

The twenty-eighth aspect of this disclosure provides a virus-like particle, wherein the virus-like particle comprises a ribonucleoprotein complex formed by the guide RNA and the RNA-guided nuclease described in this disclosure; optionally, the RNA-guided nuclease is fused with a gag protein.

The twenty-ninth aspect of this disclosure provides a eukaryotic cell comprising the gene editing system, guide RNA, nucleic acid, vector and/or vector system described in this disclosure; optionally, the eukaryotic cell is a mammalian cell; further optionally, the eukaryotic cell is a human cell.

In some embodiments of this disclosure, the eukaryotic cells comprise the gene editing system described in this disclosure.

A thirtieth aspect of this disclosure provides a pharmaceutical composition comprising the gene editing system, guide RNA, nucleic acid, vector and/or vector system described in any one of the present disclosures.

In some embodiments of this disclosure, the pharmaceutical composition comprises the gene editing system described in any one of the present disclosures.

In some embodiments of this disclosure, the pharmaceutical composition comprises the guide RNA, nucleic acid, vector and/or vector system described in any one of the present disclosures.

In some embodiments of this disclosure, the pharmaceutical composition comprises pharmaceutically acceptable excipients.

Thirty-one aspect of this disclosure provides the use of gene editing systems, guide RNAs, nucleic acids, vectors, vector systems, adeno-associated virus vectors, lipid nanoparticles, lentiviral vectors, ribonucleoprotein complexes, virus-like particles, eukaryotic cells, or pharmaceutical compositions according to any one of the following or in the preparation of reagents for realizing any one of the following schemes:

cutting one or more AR RNA molecules or nicking one or more AR RNA molecules, activating or upregulating one or more AR RNA molecules, activating or inhibiting the translation of one or more AR RNA molecules, inactivating one or more AR RNA molecules, visualizing, labeling or detecting one or more AR RNA molecules, binding one or more AR RNA molecules, transporting one or more AR RNA molecules, and masking one or more AR RNA molecules.

Some embodiments of this disclosure provide for use of the gene editing system, guide RNA, nucleic acid, vector, vector system, adeno-associated virus vector, lipid nanoparticle, lentiviral vector, ribonucleoprotein complex, virus-like particle, eukaryotic cell, or pharmaceutical composition according to any one of the following or in the preparation of reagents for realizing any one of the following schemes:

Cutting AR RNA molecules, inhibiting the translation of AR RNA molecules, and binding AR RNA molecules.

Some embodiments of this disclosure provide the use of gene editing systems, guide RNA, nucleic acids, vectors, vector systems, adeno-associated virus vectors, lipid nanoparticles, lentiviral vectors, ribonucleoprotein complexes, virus-like particles, eukaryotic cells, or pharmaceutical compositions as described in any one of these disclosures in cleaving AR RNA molecules or in the preparation of reagents for cleaving AR RNA molecules.

Some embodiments of this disclosure provide the use of gene editing systems, guide RNAs, nucleic acids, vectors, vector systems, adeno-associated virus vectors, lipid nanoparticles, lentiviral vectors, ribonucleoprotein complexes, virus-like particles, eukaryotic cells, or pharmaceutical compositions as described in any one of these disclosures in binding AR RNA molecules or in the preparation of reagents for binding AR RNA molecules.

In some embodiments of this disclosure, the AR RNA is pre-mRNA or mature mRNA. In some embodiments of this disclosure, the AR RNA is mature mRNA. In some embodiments of this disclosure, the AR RNA is human AR RNA. In some embodiments of this disclosure, the AR RNA is human AR mRNA.

The thirty-second aspect of this disclosure provides a method for diagnosing, treating or preventing a disease or condition by administering an effective amount of a gene editing system, guide RNA, nucleic acid, vector, vector system, adeno-associated virus vector, lipid nanoparticle, lentiviral vector, ribonucleoprotein complex, virus-like particle, eukaryotic cell and/or pharmaceutical composition according to this disclosure to a sample of or to a subject in need.

Some embodiments of this disclosure provide a method for diagnosing, treating or preventing a disease or condition by administering an effective amount of the gene editing system, eukaryotic cells and/or pharmaceutical composition according to this disclosure to a sample of a subject in need or to a subject in need.

Some embodiments of this disclosure provide a method for diagnosing, treating or preventing a disease or condition by administering an effective amount of the gene editing system according to this disclosure to a sample of a subject in need or to a subject in need.

In some embodiments of this disclosure, the disease or condition is an AR RNA-related disease or condition; alternatively, the disease or condition refers to a disease or condition caused by abnormally high expression of AR RNA.

In some embodiments of this disclosure, the AR RNA is pre-mRNA or mature mRNA. In some embodiments of this disclosure, the AR RNA is mature mRNA. In some embodiments of this disclosure, the AR RNA is human AR RNA. In some embodiments of this disclosure, the AR RNA is human AR mRNA.

In some embodiments of this disclosure, the disease or condition is androgenetic alopecia.

**BRIEF DESCRIPTION OF THE DRAWINGS**

FIG. 1 shows the designed gRNA targeting CTGF(CCN2) RNA.
FIG. 2 shows the CTGF RNA level after C13-2 and gRNA were edited together(qPCR detection using CTGF-my primers).
FIG. 3 shows the CTGF RNA level after C13-2 and gRNA combination editing(using CTGF-11 primers).
FIG. 4 shows the designed gRNA targeting MITF RNA.
FIG. 5 shows the MITF RNA level after C13-2 and gRNA were edited together.
FIG. 6 shows the designed gRNA targeting SRD5A2 RNA.
FIG. 7 shows the SRD5A2 RNA level after C13-2 and gRNA combination editing(qPCR detection using primer 2).
FIG. 8 shows the SRD5A2 RNA level after C13-2 and gRNA combination editing(qPCR detection using primer 3).
FIG. 9 shows the CTGF RNA level after CasRx and gRNA combined editing(CTGF-my primers)
FIG. 10 shows the CTGF RNA level after CasRx and gRNA combined editing(CTGF-II primers)
FIG. 11 shows the MITF RNA level after CasRx and gRNA were edited together.
FIG. 12 shows the SRD5A2 RNA level after CasRx and gRNA combined editing(primer 2).
FIG. 13 shows the gRNA screening results.
FIG. 14 shows the results of the comparison test between the Cas13 system and shRNA.
FIG. 15 shows the test results of C13-2 protein and CasRx in the same batch of experiments.
FIG. 16 shows the results of AR protein expression level test in the edited cells.

**DETAILED DESCRIPTION**

The present invention will be further illustrated below by way of examples, but the invention is not limited to the scope of the examples described herein. Experimental methods not specified in the following examples were performed according to conventional methods and conditions, or according to the product instructions.

**Definition section**

As used herein, the term "gene editing system" refers to a system that, when introduced into a cell, can modify.

Proteins, nucleic acids, or combinations thereof that are endogenous target nucleic acid sequences(e.g., target RNA). The gene editing system includes, but is not limited to, CRISPR-Cas systems, TALEN systems, and ZFN systems. Specifically, the gene editing system may comprise an RNA-guided nuclease and a guide RNA. Many gene editing systems suitable for use in this disclosure are known in the art, including but not limited to: Cas1, Cas1B, Cas2, Cas3, Cas4, Cas5, Cas5d, Cas5t, Cas5h, Cas5a, Cas6, Cas7, Cas8, Cas8a, Cas8b, Cas8c, Cas9, Cas10, Cas10d, Cas12a/Cpfl, Cas12b/C2cl, Cas12c/C2c3, Cas12d/CasY, Cas12e/CasX, Cas12f/CasZ, Cas12g, Cas12h, Cas12i, Csy1, Csy2, Csy3, Csy4, Cse1, Cse2, Cse3, and Cse4., Cse5e, Csc1, Csc2, Csa5, Csn1, Csn2, Csm1, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx1S, Csx11, Csf1, Csf2, CsO, Csf4, Csd1, Csd2, Cst1, Cst2, Csh1, Csh2, Csa1, Csa2, Csa3, Csa4, Csa5, Cas13a, Cas13b, Cas13c, Cas13d,Systems containing Cas13e, Cas13f, TnpB, IscB, IsrB, Fancor, or fragments thereof(non-restrictive examples such as nucleic acid-binding domain fragments); and systems containing Cas9, Cas12, Cas13, TnpB, IscB, IsrB, Fancor nucleases, or fragments thereof.

In some embodiments, the gene editing system used in the methods described herein is a clustered regularly spaced short palindromic repeat(CRISPR)/CRISPR-associated(Cas) nuclease system, which is an engineered nuclease system based on bacterial systems that can be used for mammalian genome engineering. Typically, the system includes a CRISPR-associated endonuclease(e.g., a Cas endonuclease) and a guide RNA(gRNA).

As used herein, the term "knockdown" refers to a measurable reduction in the level of target RNA in genetically modified cells compared to the level of target RNA in control cells. For example, the level of target RNA in the genetically modified cells is reduced by >0%, ≥5%, ≥10%, ≥15%, ≥20%, ≥25%, ≥30 %, ≥40%, ≥50%, ≥60 %, ≥70 %, ≥80%, ≥90 %, or ≥95% compared to the level of target RNA in control cells. Those skilled in the art will readily understand how gene-editing-mediated repression techniques can be used to knock down target RNA or portions thereof based on the details described herein.

As used herein, the term "inhibitor" refers to the selective inhibition of a target RNA, such as reducing the expression of the target RNA or inhibiting the translation of the target RNA. The inhibition may be artificially induced, for example by contacting the gene editing system with the target RNA to cleave the target RNA, thereby reducing the level of the target RNA.

As used herein, the terms "guide RNA," "guide RNA," and "gRNA" are used interchangeably. The term "guide RNA" is used to refer to a molecule in a gene editing system that forms a complex with an RNA-guided nuclease and guides the specific binding of the complex sequence to a target sequence. The guide RNA contains a guide sequence that can hybridize with the target sequence. When the RNA-guided nuclease is a Cas protein, especially Cas13, the guide RNA typically contains a direct repeat sequence linked to the guide sequence.

As used herein, the terms "guide sequence" and "target domain" are used interchangeably and refer to a continuous nucleotide sequence in gRNA that is partially or completely complementary to a target sequence in target RNA and can hybridize with the target sequence in target RNA by base pairing promoted by RNA-guided nucleases. Complete complementarity between the guide sequence and the target sequence is not required, as long as sufficient complementarity exists to induce hybridization and promote the formation of a gene-editing complex.

Suitable direct repeat(DR) sequences can be obtained through experimental screening in the CRISPR locus structure of prokaryotes(such as bacteria and archaea); they can also be obtained through sequence modification or optimization. Non-limiting examples include the deletion, substitution, or addition of one, two, three, four, or more complementary base pairs in the complementary double-stranded region of the secondary structure of the DR sequence, and the deletion, substitution, or addition of nucleotides on the loop of the stem-loop structure of the secondary structure of the DR sequence(e.g., inserting aptamer sequences into the loop). The size of a direct repeat sequence is usually tens of nucleotides, and some of its segments are anticomplementary to each other, which means that a secondary structure is formed inside the RNA molecule, such as a stem-loop structure(often called a hairpin structure), while other segments are unstructured. Direct repeat sequences are the constant part that guides RNA molecules and contain strong secondary structures, which is beneficial to the interaction between RNA-guided nucleases and guide RNA molecules.

The term "hybridization" or "hybridizing" refers to the process by which fully or partially complementary polynucleotide chains come together under suitable hybridization conditions to form a double-stranded structure or region, including association between nucleic acids via hydrogen bonds. As used herein, the term "hybridization" includes cases where the double-stranded structure or region contains one or more protrusions or mismatches. The strength of hybridization and hybridization(i.e., the strength of association between nucleic acids) is influenced by factors such as the degree of complementarity between nucleic acids, the strictness of the conditions involved, and the Tm of the hybrid formed. While hydrogen bonds are generally formed between adenine and thymine, adenine and uracil, or cytosine and guanine, other non-classical base pairs can also form hydrogen bonds. It is expected that modified nucleotides can form hydrogen bonds that allow or promote hybridization via non-classical pathways.

As used herein, the term "target RNA" refers to a polynucleotide containing a target sequence, indicating a specific sequence or its inverse complementary sequence that one would like to bind to, target or modify using a gene editing system. For example, it could be a complete mature mRNA molecule or a pre-mRNA molecule, or a fragment thereof.

As used herein, the term "target sequence" refers to a short sequence in a target RNA molecule that is complementary(fully or partially complementary) to the guide sequence of a gRNA molecule. The gene editing complex specifically locates the target sequence via the guide sequence and performs its function at or near this location. Target sequences are often tens of nucleotides(nt) in length, for example, they can be about 10 nt, about 20 nt, about 30 nt, about 40 nt, about 50 nt, or about 60 nt.

As used herein, the term "cleavage" refers to the breaking of covalent bonds(e.g., covalent phosphodiester bonds) in the ribosyl phosphodiester backbone of a polynucleotide.

The ability of the guide RNA guide complex to bind sequence-specifically to the target RNA can be assessed by any suitable assay. For example, components of a gene editing system sufficient to form a gene editing complex, including the guide RNA to be tested, can be provided to a host cell containing the corresponding target RNA molecule, for example by transfection with a vector encoding a component of the gene editing complex, and then the preferential cleavage within the target sequence can be assessed. Similarly, the cleavage of the target RNA sequence can be assessed in vitro by providing the target RNA, components of the gene editing complex, including the guide RNA to be tested and a control guide RNA different from the test guide RNA, and comparing the ability of the test and control guide RNAs to bind to the target RNA or the rate of target RNA cleavage. The ability of the guide RNA guide complex to cleave the target RNA can also be assessed by the assays described above.

The term "RNA-guided nuclease" refers to a polypeptide that binds to a specific target RNA sequence in a sequence-specific manner, and the polypeptide is guided to the target RNA by a guide RNA, which complexes with the polypeptide and hybridizes to the target sequence on the target RNA. Cleavage of the target sequence by an RNA-guided nuclease can result in strand breaks. Although RNA-guided nucleases can cleave the target sequence upon binding, the term "RNA-guided nuclease" also includes inactivated RNA-guided nucleases that can bind but not cleave the target sequence. The RNA-guided nucleases described in this disclosure include, but are not limited to, wild-type RNA-guided nucleases(e.g., C13-2, CasRx, etc.), their variants(e.g., mutants with complete loss of cleavage activity, mutants with partial loss of cleavage activity, mutants with increased cleavage activity, mutants with reduced off-target effects, mutants with reduced paracleavage effects), or their functional fragments or fusion proteins.

As used herein, the term "Cas protein" is a CRISPR-associated(Cas) polypeptide or protein that, when combined or functionally combined with one or more guide RNAs, is guided to a target sequence in a target RNA and may subsequently bind to or cleave the target RNA.

As used herein, the term "sequence identity"(or "percent identity") refers to the matching of sequences between two polypeptides or two nucleic acids. When a position in two compared sequences is occupied by the same base or amino acid monomer subunit(e.g., a position in each of two DNA molecules is occupied by adenine, or a position in each of two polypeptides is occupied by lysine), then the molecules are identical at that position. The "percent identity" between two sequences is a function of the number of matching positions shared by the two sequences divided by the number of positions compared × 100%. For example, if six out of ten positions in two sequences match, then the two sequences have 60 % sequence identity. Typically, two sequences are compared to produce the maximum sequence identity. Such alignments can be made using publicly available and commercially available alignment algorithms and procedures, such as, but not limited to, Clustal Ω, MAFFT, Probcons, T-Coffee, Probalign, and BLAST, which can be reasonably chosen by those skilled in the art. Those skilled in the art can determine suitable parameters for the alignment of sequences, including any algorithm required to achieve a better or better alignment of the entire length of the sequences being compared, and any algorithm required to achieve a better or better alignment of a local portion of the sequences being compared.

As used herein, the term "regulatory sequence" is intended to include promoters, enhancers, internal ribosome entry sites(IRES), and other expression control elements(e.g., transcription termination signals, such as polyadenylation signals and poly-U sequences). Regulatory sequences include those elements that guide the expression of nucleotide sequences sequentially in many types of host cells and those that guide the expression of nucleotide sequences only in certain host cells(e.g., tissue-specific regulatory sequences). Tissue-specific promoters may guide expression primarily in desired tissues of interest such as muscle, neurons, bone, skin, blood, specific organs(e.g., liver, pancreas), or specific cell types(e.g., neurons, lymphocytes). Regulatory sequences may also guide expression in a time-dependent manner, such as cell cycle-dependent or developmental stage-dependent, which may or may not be tissue-specific or cell type-specific. The term regulatory sequence also encompasses enhancer elements such as WPRE, CMV enhancers, SV40 enhancers, and intron sequences between exons 2 and 3 of rabbit β- globulin. Those skilled in the art will understand that the design of the expression vector can depend on factors such as the selection of the host cell to be transformed and the desired expression level. The vector can be introduced into the host cell to generate the RNA-guided nuclease and/or guide RNA described in this invention.

As used herein, the term "promoter" has the meaning commonly understood in the art.

As used herein, the term "enhancer" has the meaning commonly understood in the art.

As used herein, when referring to a nucleotide sequence/DNA/RNA encoding a protein, RNA, or gene editing complex, the coding sequence may be codon-optimized. For example, the coding sequence may be codon-optimized for expression in a eukaryotic cell environment, for expression in a mammalian cell environment, or for expression in a human cell environment.

As used herein, the term "codon optimization" refers to the process of altering the codons of a given gene in a manner that keeps the sequence of the polypeptide encoded by the gene unchanged, while the altered codons improve the expression of the polypeptide sequence. For example, if the polypeptide is a human protein sequence and is expressed in E. coli, codon optimization of the DNA sequence to change the human codons to codons that are more effective for expression in E. coli would generally improve expression.

As used herein, the term pharmaceutically acceptable excipient means a diluent, adjuvant, drug delivery vehicle, or other excipient administered with the active ingredient. Its selection depends on the intended use and the intended route of administration. Excipients should not be incompatible with the active ingredient, for example, producing any undesirable biological effects or interacting harmfully with any other component of the pharmaceutical composition. Pharmaceutical compositions can be prepared by methods known in the field of pharmaceutical preparation.

When referring to an RNA sequence, the "T" in the sequence can be used interchangeably with the "U". When referring to a "guide sequence", the "T" in the sequence can be used interchangeably with the "U". When referring to a "directed repeat sequence", the "T" in the sequence can be used interchangeably with the "U". "

As used herein, the term " cosmetics " means a product applied to any part of the human body surface(skin, hair, nails, lips, etc.) by smearing, sprinkling, spraying or other similar means to achieve the purpose of cleansing, fragrance, altering appearance, correcting body odor, maintaining health, or preserving good condition. Cosmetics may include all products described in the National Standard of the People's Republic of China - Nomenclature and Terminology of Cosmetics(GB/T 27578-2011).

As used herein, the term "pharmaceutically, cosmetically, chemically, or biologically acceptable" refers to a substance that is suitable for use in humans and/or animals without excessive adverse side effects(such as toxicity, irritation, and allergic reactions), i.e., a substance with a reasonable benefit/risk ratio. Examples include pharmaceutically acceptable excipients, cosmetically acceptable excipients, thickeners, or diluents, etc. For example, cosmetics disclosed herein may contain liquids such as water, saline, glycerin, and ethanol; in addition, auxiliary substances such as fillers, lubricants, flow aids, wetting agents or emulsifiers, fragrances, pH buffers, etc., may also be present.

**Guide RNA**

In some embodiments of this disclosure, the guide RNA is capable of forming a complex(also known as a gene editing complex) with an RNA-guided nuclease and directing the complex to specifically bind to and cleave the target RNA(CTGF RNA, MITF RNA, SRD5A2 RNA, or AR RNA) at a specific sequence. The guide RNA comprises a guide sequence and a scaffold sequence. The scaffold sequence interacts with the RNA-guided nuclease. The scaffold sequence is the sequence in the guide RNA molecule that is typically kept unchanged during the design of the guide RNA molecule. For example, the scaffold sequence may refer to a portion of the guide RNA molecule other than the guide sequence. In some embodiments of this disclosure, the scaffold sequence is a direct repeat(DR) sequence.

In some embodiments of this disclosure, the guide sequence has at least 80% sequence identity with the target RNA. In some embodiments of this disclosure, the guide sequence has at least 85%, at least 90%, at least 95%, or 100% sequence identity with the target RNA. Further, in some embodiments of this disclosure, the guide sequence has 100% sequence identity with the target RNA.

In some embodiments of this disclosure, the guide sequence has at least 80% sequence identity with the sequence shown in any one of SEQ ID NO: 14, 22, or 35. In some embodiments of this disclosure, the guide sequence has at least 85%, at least 90%, at least 95%, or 100% sequence identity with the sequence shown in any one of SEQ ID NO: 14, 22, or 35. Further, in some embodiments of this disclosure, the guide sequence has 100% sequence identity with the sequence shown in any one of SEQ ID NO: 14, 22, or 35.

In some embodiments of this disclosure, the guide sequence has at least 85%, at least 90 %, at least 95% or 100% sequence identity with the nucleotide sequence of nucleotides 494 to 785 of the sequence shown in SEQ ID NO: 14, nucleotides 404 to 606 of the sequence shown in SEQ ID NO: 22, or nucleotides 484 to 820 of the sequence shown in SEQ ID NO: 35.

In some embodiments of this disclosure, the guide sequence has at least 85 %, at least 90 %, at least 95%, or 100% sequence identity with the nucleotide sequence of the sequence shown in SEQ ID NO: 14, from the 494th, 604th, 678th, or 761st nucleotides to the 518th, 628th, 702nd, or 785th nucleotides.

In some embodiments of this disclosure, the guide sequence has at least 85 %, at least 90%, at least 95%, or 100% sequence identity with the nucleotide sequence of nucleotides 404, 429, 453, 479, 509, 546, or 582, up to 428, 453, 477, 503, 533, 570, or 606 of the sequence shown in SEQ ID NO: 22.

In some embodiments of this disclosure, the guide sequence has at least 85 %, at least 90%, at least 95%, or 100% sequence identity with the nucleotide sequence of nucleotides 484, 524, 547, 585, 635, 721, 755, or 796, up to 508, 548, 571, 609, 659, 745, 779, or 820 of the sequence shown in SEQ ID NO: 35.

In some embodiments of this disclosure, the guide sequence has at least 80%, at least 85%, at least 90%, at least 95%, or 100% sequence identity with any of the sequences shown in SEQ ID NOs: 5-13, 23-32, and 36-57. Further, in some embodiments of this disclosure, the guide sequence has 100% sequence identity with any of the sequences shown in SEQ ID NOs: 5-13, 23-32, and 36-57. In some embodiments of this disclosure, the guide sequence comprises any of the sequences shown in SEQ ID NOs: 5-13, 23-32, and 36-57. In some embodiments of this disclosure, the guide sequence is any of the sequences shown in SEQ ID NOs: 5-13, 23-32, and 36-57.

In some embodiments of this disclosure, the guide sequence has at least 80%, at least 85%, at least 90%, at least 95%, or 100% sequence identity with the sequence shown in any one of SEQ ID NO: 8, 9, 10, 12, 23-29, 36, 38, 40, 42, 43, 44, 46, or 54. In some embodiments of this disclosure, the guide sequence comprises the sequence shown in any one of SEQ ID NO: 8, 9, 10, 12, 23-29, 36, 38, 40, 42, 43, 44, 46, or 54. In some embodiments disclosed herein, the guide sequence is any one of the sequences shown in SEQ ID NO: 8, 9, 10, 12, 23-29, 36, 38, 40, 42, 43, 44, 46, 54.

In some embodiments of this disclosure, the guide sequence comprises 20-40, 20-35, 20-30 or 25-30 nucleotides.

In some embodiments of this disclosure, the guide sequence hybridizes with the target RNA in a number of mismatches not exceeding 6, 5, 4, 3, 2, or 1 nucleotide.

In some embodiments of this disclosure, the guide sequence is 100% identical to the target RNA sequence, i.e., completely complementary.

In some embodiments of this disclosure, the scaffold sequence is a direct repeat sequence, which includes a sequence having at least 50%, at least 55%, at least 60%, at least 65%, at least 70%, at least 75 %, at least 80%, at least 85%, at least 90 %, at least 95%, at least 96%, at least 97%, at least 98 %, at least 99%, or 100% sequence identity with the sequence shown in SEQ ID NO: 2 or 3.

In some embodiments of this disclosure, the direct repeat sequence comprises the sequence shown in SEQ ID NO: 2 or 3. In some embodiments of this disclosure, the direct repeat sequence consists of the sequence shown in SEQ ID NO: 2 or 3.

In some embodiments of this disclosure, the guide sequence is located at the 3' end or 5' end of the direct repeat sequence. In some embodiments of this disclosure, the guide sequence is located at the 3' end of the direct repeat sequence. In some embodiments of this disclosure, the guide sequence is located at the 5' end of the direct repeat sequence.

In some embodiments of this disclosure, the guide RNA comprises an aptamer sequence.

In some embodiments of this disclosure, the aptamer sequence is inserted into the loop of the stem-loop structure of the secondary structure of the same-direction repeat sequence of the guide RNA.

In some embodiments of this disclosure, the guide RNA comprises a modified nucleotide. The modification includes, but is not limited to, 2'-O-methyl, 2'-O-methyl-3'-thiophosphate, or 2'-O-methyl-3'-thioPACE modification. In some embodiments of this disclosure, the guide RNA comprises a modified nucleotide selected from deoxyribonucleotides and locked nucleic acids(LNAs). In some embodiments, the guide RNA comprises at least one chemically modified nucleotide. Chemically modified guide RNAs are described in Hendel et al., Nat. Biotechnol. 33(9): 985-989(2015), the full text of which is incorporated herein by reference.

In some embodiments, the guide RNA is a hybrid RNA-DNA guide, meaning that some RNA nucleotides in the guide RNA are replaced by DNA nucleotides. In some embodiments, the guide RNA is a hybrid RNA-LNA(locked nucleotide) guide, meaning that some RNA nucleotides in the guide RNA are replaced by LNA nucleotides. Hybrid RNA-DNA guide polynucleotides are described in WO2016/123230, which is incorporated herein by reference in its entirety.

In some embodiments of this disclosure, the target RNA is located in the nucleus and/or cytoplasm of a eukaryotic cell.

In some embodiments of this disclosure, the guide RNA is capable of forming a complex(also known as a gene editing complex) with an RNA-guided nuclease and directing the complex to bind specifically to the target RNA.

In some embodiments of this disclosure, the guide RNA is capable of forming a complex with an RNA-guided nuclease and directing the complex to bind to and cleave the target RNA.

In some embodiments of this disclosure, the complex reduces the level of the target RNA in the cell.

In some embodiments of this disclosure, the complex, upon contact with a cell containing the target RNA, reduces the level of the target RNA in the cell, for example, the level of the target RNA in a cell expressing the target RNA.

In some embodiments of this disclosure, the complex reduces the level of the target RNA in cells by at least 5%, at least 10%, at least 15%, at least 20%, at least 25 %, at least 30 %, at least 35%, at least 40%, at least 50%, at least 60 %, at least 70%, at least 80%, at least 90%, at least 95 %, at least 96 %, at least 97%, at least 98%, or at least 99%. The reduction in the target RNA level can be tested using methods conventional in the art, including but not limited to qPCR methods as described in the examples, such as RT-qPCR. Untreated cells or cells treated with a gene-editing system targeting a non-mammalian genome can be used as negative controls to calculate the target RNA knockdown level in the experimental group compared to the negative control. The experimental and negative control groups can be compared by editing the same Cas protein(e.g., by expressing the same Cas protein) with gRNAs containing different guide sequences, followed by comparison of differences in target RNA levels; for example, the experimental group can be edited using C13-2 and a gRNA as claimed in this disclosure, while the negative control group can be edited using C13-2 and a gRNA targeting, for example, the bacterial genome. The experimental group can also be compared with known editing tools, such as the CasRx+gRNA editing tool.

In some embodiments of this disclosure, the complex reduces the level of the target RNA in cells by at least 5%, at least 10%, at least 15%, at least 20%, at least 25 %, at least 30%, at least 35%, at least 40 %, at least 45%, at least 50%, at least 55%, at least 60%, at least 65 %, at least 70%, at least 75%, at least 80%, at least 85%, or at least 90%. In some embodiments of this disclosure, the complex reduces the level of the target RNA in cells by at least 40%. In some embodiments of this disclosure, the complex reduces the level of the target RNA in cells by at least 80%. In some embodiments of this disclosure, the complex reduces the level of the target RNA in cells by at least 85%. In some embodiments of this disclosure, the complex reduces the level of the target RNA in cells by at least 90%.

In some embodiments of the present invention, the number of off-target genes when the complex binds to and cleaves the target RNA is less than 40, less than 35, less than 30, less than 25, less than 20, less than 19, less than 18, less than 17, less than 16, less than 15, less than 14, less than 13, less than 12, less than 11, less than 10, less than 9, less than 8, less than 7, less than 6, less than 5, less than 4, less than 3, less than 2, or less than 1. The number of off-target genes can be determined by conventional methods in the art. In some embodiments, the number of off-target genes is determined by differentially expressed genes identified by RNA sequencing. In some embodiments, the number of off-target genes is determined by taking the intersection of the set of differentially expressed genes identified by RNA sequencing and the set of off-target genes predicted by the program. For example, the method of prediction by the program is as follows: the EMBOSS-water program is used to predict the whole genome and whole cDNA sequence of the target species(Homo sapiens/Mus musculus), with the parameters set as gap_extend=0.5 & gap_extend=10. The forward and reverse strands of the gRNA-guided sequence are compared, and the prediction results are filtered to obtain the predicted potential target genes(including target genes and off-target genes).

In some embodiments of this disclosure, the complex, upon contact with a cell containing the target RNA, reduces the level of the protein encoded by the target RNA in the cell. In some embodiments of this disclosure, the protein encoded by the target RNA is CTGF protein, MITF protein, SRD5A2 protein, or AR protein. In some embodiments of this disclosure, the complex reduces the level of CTGF protein, MITF protein, SRD5A2 protein, or AR protein in the cell.

In some embodiments of this disclosure, the complex reduces the levels of CTGF, MITF, SRD5A2, or AR proteins in cells by at least 5%, at least 10%, at least 15%, at least 20%, at least 25%, at least 30 %, at least 35%, at least 40%, at least 50%, at least 60%, at least 70 %, at least 80%, at least 90%, at least 95%, at least 96%, at least 97 %, at least 98%, or at least 99%. The reduction in the level of the target RNA-encoded protein can be tested using methods conventional in the art, including but not limited to ELISA and Western blotting. Untreated cells or cells treated with a gene-editing system targeting a non-mammalian genome can be used as negative controls to calculate the knockdown levels of CTGF, MITF, SRD5A2, or AR proteins in the experimental group compared to the negative control group.

In some embodiments of this disclosure, the RNA-guided nuclease is a Cas protein. In some embodiments, the RNA-guided nuclease has completely or partially lost its cleavage activity.

In some embodiments of this disclosure, the RNA-guided nuclease is a Cas protein.

In some embodiments of this disclosure, the RNA-guided nuclease is a Cas protein, and the guiding RNA comprises a guide sequence and a direct repeat sequence.

In some embodiments of this disclosure, the Cas protein is a Cas9 protein, a Cas12 protein, or a Cas13 protein.

In some embodiments, the Cas9 protein is selected from SpCas9, SaCas9, Nme2Cas9, Nme3Cas9, CjCas9, NmCas9 and FnCas9.

In some embodiments, the Cas12 protein is selected from Cas12a, Cas12b, Cas12c, Cas12d, Cas12e, Cas12f, Cas12g, Cas12h, Cas12i, Cas12j, Cas12k, Cas12I, and Cas12m proteins.

In some embodiments of this disclosure, the Cas protein is the Cas13 protein.

In some embodiments of this disclosure, the Cas protein is a Cas13a protein, a Cas13b protein, a Cas13c protein, or a Cas13d protein. In some embodiments of this disclosure, the Cas protein is a Cas13a protein, a Cas13b protein, or a Cas13c protein in which both HEPN domains carry mutations in their catalytic RxxxxH motifs, and these Cas proteins have completely or partially lost their cleavage activity. In some embodiments, the Cas protein is a CasRx protein. In some embodiments, the Cas protein is a dCasRx in which both HEPN domains carry mutations(R239A and H244A of HEPN-1 and R858A and H863A of HEPN-2).

In some embodiments of this disclosure, the Cas protein comprises the sequence shown in SEQ ID NO: 1.

In some embodiments of this disclosure, the guide sequence is located at the 3' end or 5' end of the direct repeat sequence. In some embodiments of this disclosure, the guide sequence is located at the 3' end of the direct repeat sequence. In some embodiments of this disclosure, the guide sequence is located at the 5' end of the direct repeat sequence.

In some embodiments of this disclosure, the direct repeat sequence comprises the sequence shown in SEQ ID NO: 2. In some embodiments of this disclosure, the direct repeat sequence consists of the sequence shown in SEQ ID NO: 2.

**Target RNA**

The gene editing systems and compositions described herein can be used to target one or more target RNA molecules, such as target RNA molecules present in biological samples. In some embodiments, the target RNA is pre-mRNA or mRNA(mature mRNA).

In some embodiments of this disclosure, the target RNA is CTGF RNA, MITF RNA, or SRD5A2 RNA, or a fragment thereof. In some embodiments of this disclosure, the target RNA is CTGF pre-mRNA, MITF pre-mRNA, or SRD5A2 pre-mRNA, CTGF mRNA, MITF mRNA, or SRD5A2 mRNA, or a fragment thereof. In some embodiments of this disclosure, the target RNA is CTGF mRNA, MITF mRNA, or SRD5A2 mRNA, or a fragment thereof. In some embodiments of this disclosure, the target RNA is CTGF mRNA, MITF mRNA, or SRD5A2 mRNA. In some embodiments of this disclosure, the target RNA is mammalian CTGF RNA, MITF RNA, or SRD5A2 RNA. In some embodiments of this disclosure, the target RNA is human CTGF RNA, MITF RNA, or SRD5A2 RNA. In some embodiments of this disclosure, the target RNA is human CTGF mRNA, MITF mRNA, or SRD5A2 mRNA.

In some embodiments of this disclosure, the target RNA sequence is as shown in SEQ ID NO: 14, 22 or 35.

In some embodiments of this disclosure, the target RNA sequence is nucleotides 494 to 785 of the sequence shown in SEQ ID NO: 14, nucleotides 404 to 606 of the sequence shown in SEQ ID NO: 22, or nucleotides 484 to 820 of the sequence shown in SEQ ID NO: 35.

In some embodiments of this disclosure, the target RNA sequence is the nucleotide sequence of nucleotides 494, 604, 678 or 761, to 518, 628, 702 or 785 of the sequence shown in SEQ ID NO: 14(human CCN2 mRNA, NCBI NM_001901.4).

In some embodiments of this disclosure, the target RNA sequence is the nucleotide sequence of nucleotides 404, 429, 453, 479, 509, 546 or 582, to 428, 453, 477, 503, 533, 570 or 606 of the sequence shown in SEQ ID NO: 22(human MITF mRNA, NCBI NM_001354607.2).

In some embodiments of this disclosure, the target RNA sequence is the nucleotide sequence of nucleotides 484, 524, 547, 585, 635, 721, 755 or 796, up to nucleotides 508, 548, 571, 609, 659, 745, 779 or 820 of the sequence shown in SEQ ID NO: 35(human SRD5A2 mRNA, NCBI XM_011533072.3).

When this document refers to target nucleic acids, CTGF mRNA, MITF mRNA or SRD5A2 mRNA transcript variants(NCBI accession numbers NM_001901.4, NM_001354607.2, XM_011533072.3), it is not intended to limit the gRNA disclosed herein to targeting only this nucleic acid molecule; those skilled in the art should know that the gRNA disclosed herein can be used to target other nucleic acid molecules, such as human CTGF pre-mRNA, MITF pre-mRNA or SRD5A2 pre-mRNA, other transcript variants of CTGF mRNA, MITF mRNA or SRD5A2 mRNA, etc.

In some embodiments of this disclosure, the gene editing system described herein can be used to reduce the expression levels of CTGF RNA, MITF RNA, or SRD5A2 RNA; for example, by using an RNA-guided nuclease, under the guidance of gRNA, to contact and cleave CTGF RNA, MITF RNA, or SRD5A2 RNA. In some embodiments, the gene editing system described herein can be used to reduce the intracellular expression levels of CTGF RNA, MITF RNA, or SRD5A2 RNA.

In some embodiments of this disclosure, the target RNA is an AR RNA. In some embodiments of this disclosure, the target RNA is a human AR RNA. In some embodiments of this disclosure, the target RNA is a human mRNA.

In some embodiments of this disclosure, the target RNA is an AR RNA or a fragment thereof.

In some embodiments of this disclosure, the target RNA is AR mRNA or a fragment thereof.

In some embodiments of this disclosure, the target RNA is AR pre-mRNA or a fragment thereof.

In some embodiments of this disclosure, the target RNA is selected from any one of the sequences shown in SEQ ID NO s: 394-396.

In some embodiments of this disclosure, the gene editing system described herein can be used to reduce the expression level of AR RNA; for example, by using an RNA-guided nuclease to contact and cleave AR RNA under the guidance of gRNA. In some embodiments, the gene editing system described herein can be used to reduce the expression level of AR RNA in cells.

### Aptamer/aptamer sequence

In some embodiments, the guiding polynucleotide further comprises an aptamer sequence. In some embodiments, the aptamer sequence is inserted into a loop of the guiding polynucleotide. In some embodiments, the aptamer sequence is attached to the end of the guiding polynucleotide.

In some embodiments, the aptamer sequence includes the MS2 aptamer sequence, the PP7 aptamer sequence, or the Qβ aptamer sequence.

### Adaptor protein

In some embodiments, the gene editing system further comprises a fusion protein containing an adaptor protein and a fusion domain, or a nucleic acid encoding the fusion protein, wherein the adaptor protein is capable of binding an aptamer sequence.

In some embodiments, the adaptor protein includes MS2 phage capsid protein(MCP), PP7 phage capsid protein(PCP), or Qβ phage capsid protein(QCP). In some embodiments, the fusion domain includes a cytosine deaminase domain, an adenosine deaminase domain, a translation activation domain, a translation repression domain, an RNA methylation domain, an RNA demethylation domain, a nuclease domain, a splicing factor domain, or an affinity or reporter tag or domain.

### RNA-guided nucleases

When referring to RNA-guided nucleases in this disclosure, it may refer to the RNA-guided nuclease itself in a narrow sense, or to fusion proteins obtained by covalently linking or fusing RNA-guided nucleases with other domains.

In some embodiments of this disclosure, the RNA-guided nuclease is optionally selected from Cas1, Cas1B, Cas2, Cas3, Cas4, Cas5, Cas5d, Cas5t, Cas5h, Cas5a, Cas6, Cas7, Cas8, Cas8a, Cas8b, Cas8c, Cas9, Cas10, Cas10d, Cas12a/Cpfl, Cas12b/C2cl, Cas12c/C2c3, Cas12d/CasY, Cas12e/CasX, Cas12f/CasZ, Cas12g, Cas12h, Cas12i, Csy1, Csy2, Csy3, Csy4, Cse1, Cse2, Cse3, Cse4, Cse5e, Csc1, Csc2, Csa5, Csn1, Csn2, Csm1, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx1S, Csx11, Csf1, Csf2, CsO, Csf4, Csd1, Csd2, Cst1, Cst2, Csh1, Csh2, Csa1, Csa2, Systems of Csa3, Csa4, Csa5, Cas13a, Cas13b, Cas13c, Cas13d, Cas13e, Cas13f, TnpB, IscB, IsrB, Fancor; or fragments thereof(non-restrictive examples such as nucleic acid binding domain fragments).

In some embodiments of this disclosure, the RNA-guided nuclease is selected from Cas9, Cas12, Cas13, TnpB, IscB, IsrB, Fancor nucleases; or fragments thereof, including but not limited to nucleic acid binding domain fragments.

In some embodiments of this disclosure, the RNA-guided nuclease is a Cas protein.

In some embodiments of this disclosure, the RNA-guided nuclease is the Cas13 protein.

In some embodiments of this disclosure, the RNA-guided nuclease is selected from SpCas9, SaCas9, Nme2Cas9, Nme3Cas9, CjCas9, NmCas9, FnCas9, PpnCas9, FrCas9, SauCas9, SauriCas9, ScaCas9, St1Cas9, BlatCas9, CdiCas9, GeoCas9, fragments thereof, and mutants thereof or fragments thereof.

In some embodiments of this disclosure, the RNA-guided nuclease is selected from AsCpf1, enAsCas12a(addgene plasmid #196724), dFnCas12a(addgene plasmid #136379), ErCas12a, LbCas12a D832A, LbCas12a H759A, LbCas12a E795L, FnCas12a3, FnCas12a D917A, AsCas12a R1226A, AsCas12a D908A, AsCas12a E174R/S542R, AsCas12a(S542R/K548V/N552R), PrCas12a, PxCas12a, PcCas12a, PdCas12a, Mb2Cas12a, Mb3Cas12a, MlCas12a, CMaCas12a, CMtCas12a, HkCas12a, Lb5Cas12a, ErCas12a, TsCas12a, FnCpf1, LbCas12a, ttHsCas12a, AaCas12b, AaCas12b D570A, AaCas12b Q119F/E475R/E758R, BhCas12b, BvCas12b, BrCas12b, AkCas12b, AmCas12b, BsCas12b, OspCas12c, Cas12c2(addgene plasmid #183072), Cas12c_4(addgene plasmid #183071 Cas12c1(addgene plasmid #120872), CasY.1(from Katanobacteria), CasY.2(from Vogelbacteria), CasY.3(from Vogelbacteria), CasY.4(from Parcubacteria), CasY.5(from Komeilibacteria), CasY.6(from Kerfeldbacteria), PlmCasX, DpbCasX, Un1Cas12f, CnCas12f1, enRhCas12f1, AsCas12f1, SpaCas12f1, Cas12g1(addgene plasmid #120879), Cas12h of WO2021113522A1(SEQ ID NO: 1), Cas12i1(addgene plasmid #171670)), Cas12i2(addgene plasmid #188275),Cas12i1(adgene plasmid #120882), Cas12i2(adgene plasmid #120883), CN111757889B Manufacturer Cas12f.4/Cas12f.5/Cas12f.6 Cas12i Process dSiCas12i(D1049A), SiCas12i, Si2Cas12i, WiCas12i. Wi2Cas12i, Wi3-Cas12i, SaCas12i, Sa2Cas12i, Sa3Cas12i, WaCas12i, Wa2Cas12i, xCas12i, hfCas12Max, Cas12i-Max(addgene plasmid). #188276) Cas12i1 D647A(adgene plasmid #171671) Cas12i-HiFi(adgene plasmid #188269) Cas12i1 D647A Cas12j3(adgene plasmid #188497) Cas12j2(adgene plasmid #188498) AsCas12j-2( adgene plasmid # 191655) Cas12j-8(adgene plasmid #194966) ShCas12k N7Cas12k, AcCas12k, Cas12k-TniQ(adgene plasmid #181787), Cas12k-TnsC(adgene plasmid #181789), Cas12l, MmCas12m, MmCas12m ΔZF(H549A, C552A), dCas12m-ΔZF(D485A, H549A, C552A), AcCas12n, dAcCas12n(D240), TnpB Actinomature_cellulosilytic_strain_DSM_45823; TnpB Actinomadura_namibiensis_strain_DSM_44197, TnpB Actinomadura_umbrina_strain_DSM_43927_$, TnpB Actinoplanes_lobatus_strain_DSM_43150(TnpB-1 and TnpB-2), TnpB Alicyclobacillus_macrosporagiidus_strain_DSM_17980, TnpB Haloactinospora_alba_strain_DSM_45015, TnpB Lipingzhangella_halophila_strain_DSM_102030, TnpB Meiothermus_Silvanus_DSM_9946 TnpB QNFX01000004, ISDra2 TnpB(PDB: 8H1J), KraIscB-1, AwaIscB, OgeuIscB, GtFz1(Guillardia theta) SpuFz1(speak Spizellomyces punctatus), NlovFz2(speakPercolozoa Naegleria lovaniensis), MmeFz2(from Mercenaria mercenaria), fragments of them, and their mutants or fragments of mutants.

In some embodiments of this disclosure, the RNA-guided nuclease is selected from wild-type RNA-guided nucleases(including but not limited to CasRx, C13-2, etc.), their variants(including but not limited to mutants with complete loss of cleavage activity, mutants with partial loss of cleavage activity, mutants with increased cleavage activity, mutants with reduced off-target/sidecut effects), or their functional fragments or fusion proteins.

In some embodiments of this disclosure, the RNA-guided nuclease comprises any one or more of the following fused domains: subcellular localization signal, deaminase domain, translation activation domain, translation repression domain, RNA methylation domain, RNA demethylation domain, nuclease domain, splicing factor domain, reporter tag, and affinity tag.

In some embodiments of this disclosure, the RNA-guided nuclease contains a subcellular localization signal.

In some embodiments of this disclosure, the RNA-guided nuclease includes a subcellular localization signal and a deaminase domain.

In some embodiments of this disclosure, the subcellular localization signal may be selected from the nuclear localization signal and the nuclear output signal.

In some embodiments, the RNA-guided nuclease is fused with at least one subcellular localization signal. Exemplary subcellular localization signals include organelle localization signals, such as nuclear localization signals(NLS), nuclear export signals(NES), or mitochondrial localization signals.

In some embodiments, the RNA-guided nuclease is fused with at least one heterologous NLS. In some embodiments, the RNA-guided nuclease is fused with at least two NLSs. In some embodiments, the RNA-guided nuclease is fused with at least three NLSs. In some embodiments, the RNA-guided nuclease is fused with at least one N-terminal NLS and at least one C-terminal NLS. In some embodiments, the RNA-guided nuclease is fused with at least two C-terminal NLSs. In some embodiments, the RNA-guided nuclease is fused with at least two N-terminal NLSs.

In some embodiments, the NLS is independently selected from SPKKKRKVEAS(SEQ ID NO: 397), GPKKKRKVAAA(SEQ ID NO: 398), PKKKRKV(SEQ ID NO: 399), KRPAATKKAGQAKKKK(SEQ ID NO: 400), PAAKRVKLD(SEQ ID NO: 401), RQRRNELKRSP(SEQ ID NO: 402), NQSSNFGPMKGGNFGGRSSGPYGGGGQYFAKPRNQGGY(SEQ ID NO: 403), RMRIZFKNKGKDTAELRRRRVEVSVELRKAKKDEQILKRRNV( SEQ ID NO: 404), VSRKRPRP(SEQ ID NO: 404). NO: 405), PPKKARED(SEQ ID NO: 406), POPKKKPL(SEQ ID NO: 407), SALIKKKKKMAP(SEQ ID NO: 408), DRLRR(SEQ ID NO: 409), PKQKKRK(SEQ ID NO: 410), RKLKKKIKKL(SEQ ID NO: 389), REKKKFLKRR(SEQ ID NO: 392), KRKGDEVDGVDEVAKKKSKK(SEQ ID NO: 379), and RKCLQAGMNLEARKTKK(SEQ ID NO: 386).

In some embodiments, the RNA-guided nuclease is fused with a heterologous NES. In some embodiments, the RNA-guided nuclease is fused with at least two NES. In some embodiments, the RNA-guided nuclease is fused with at least three NES. In some embodiments, the RNA-guided nuclease is fused with at least one N-terminal NES and at least one C-terminal NES. In some embodiments, the RNA-guided nuclease is fused with at least two C-terminal NES. In some embodiments, the RNA-guided nuclease is fused with at least two N-terminal NES.

In some embodiments, the NES is independently selected from adenovirus type 5 E1B NES, HIV Rev NES, MAPK NES, or PTK2 NES.

In some embodiments, the RNA-guided nuclease is fused with an NLS and an NES, with a cleavable linker between the NLS and the NES. In some embodiments, the NES in a production cell line facilitates the production of delivery particles(e.g., virus-like particles) containing the RNA-guided nuclease. In some embodiments, cleavage of the linker in a target cell can expose the NLS and facilitate nuclear localization of the RNA-guided nuclease in the target cell. Exemplary fusion domains(such as fused heterologous protein domains) include domains capable of cleaving RNA(e.g., PIN endonuclease domains, NYN domains, SMR domains from SOT1, or RNase domains from staphylococcal nucleases), domains capable of affecting RNA stability(e.g., tristetraprolin(TTP) or domains from UPF1, EXOSC5, and STAU1), domains capable of editing nucleotides or ribonucleotides(e.g., cytidine deaminases, PPR proteins, adenosine deaminases, ADAR family proteins, or APOBEC family proteins), domains capable of activating translation(e.g., eIF4E and other translation initiation factors, yeast poly(A)-binding proteins, or GLD2 domains), domains capable of inhibiting translation(e.g., Pumilio or FBF PUF proteins, deadenosylases, CAF1, Argonaute proteins), and domains capable of methylating RNA(e.g., m6A methyltransferase factors such as METTL14, METTL3, or WTAP). Domains that can demethylate RNA(e.g., human alkylation repair homolog 5), domains that can affect splicing(e.g., RS-rich domain of SRSF1, Gly-rich domain of hnRNP A1, alanine-rich motif of RBM4, or proline-rich motif of DAZAP1), domains that can enable affinity purification or immunoprecipitation(e.g., FLAG, HA, biotin, or HALO tags), and domains that can enable proximity-based protein labeling and recognition(e.g., biotin ligases(such as BirA) or peroxidases(such as APEX2) to biotinylate target DNA interacting proteins.

In some embodiments of this disclosure, the deaminase domain comprises an adenosine deaminase domain. In some embodiments, an RNA-guided nuclease(non-limiting example, such as the Cas13 protein having a mutant HEPN domain or the Cas13 protein catalytically inactivated) that retains, partially inactivates, or completely inactivates nuclease activity is covalently linked or fused to the adenosine deaminase domain to guide A-to-I deaminase activity of RNA transcripts in mammalian cells. An ADAR2-engineered adenosine deaminase domain for targeting A-to-I RNA editing is described in Cox et al., Science 358(6366): 1019-1027(2017), which is incorporated herein by reference in its entirety. In other embodiments, the adenosine deaminase domain is covalently linked or fused to an adaptor protein capable of binding an aptamer sequence inserted into or attached to the guiding polynucleotide, thereby allowing the adenosine deaminase domain to be non-covalently linked to the RNA-guided nuclease complexed with the guiding polynucleotide.

In some embodiments of this disclosure, the deaminase domain comprises a cytosine deaminase domain. In some embodiments, an RNA-guided nuclease(non-limiting examples such as the Cas13 protein having a mutant HEPN domain or the Cas13 protein catalytically inactivated) that retains, partially inactivates, or completely inactivates nuclease activity is covalently linked or fused to the cytosine deaminase domain to guide C-to-U deaminase activity of RNA transcripts in mammalian cells. A cytosine deaminase domain for targeting C-to-U RNA editing, derived from ADAR2, is described in Abudayyeh et al., Science 365(6451): 382-386(2019), which is incorporated herein by reference in its entirety. In other embodiments, the cytosine deaminase domain is covalently linked or fused to an adaptor protein capable of binding to an insert or attachment aptamer sequence of the guiding polynucleotide, thereby allowing the cytosine deaminase domain to be non-covalently linked to the RNA-guided nuclease complexed with the guiding polynucleotide.

In some embodiments, the fusion domain comprises a splicing factor domain. In some embodiments, a Cas13 protein having a mutated HEPN domain or a catalytically inactivated Cas13 protein is covalently linked or fused to the splicing factor domain to guide alternative splicing of target RNA in mammalian cells. Non-limiting examples of splicing factor domains include an RS-rich domain of SRSF1, a Gly-rich domain of hnRNPA1, an alanine-rich motif of RBM4, or a proline-rich motif of DAZAP1. In other embodiments, the splicing factor domain is covalently linked or fused to an adaptor protein capable of binding to an aptamer sequence inserted into or attached to the guide RNA, thereby allowing the splicing factor domain to be non-covalently linked to the Cas13 protein complexed with the guide RNA.

In some embodiments of this disclosure, the RNA-guided nuclease is covalently linked or fused to a translation activation domain. In some embodiments, an RNA-guided nuclease that is active, partially inactivated, or completely inactivated(non-limiting examples include, for example, the Cas13 protein with a mutated HEPN domain or the Cas13 protein that is catalytically inactivated) is covalently linked or fused to a translation activation domain to activate or increase the expression of the target RNA. Non-limiting examples of translation activation domains include domains of eIF4E and other translation initiation factors, yeast poly(A)-binding proteins, or GLD2. In other embodiments, the translation activation domain is covalently linked or fused to an adaptor protein capable of binding to an aptamer sequence inserted into or attached to the guiding polynucleotide, thereby allowing the translation activation domain to be non-covalently linked to the RNA-guided nuclease complexed with the guiding polynucleotide.

In some embodiments of this disclosure, the RNA-guided nuclease is covalently linked or fused to a translation repression domain. In some embodiments, an RNA-guided nuclease that is active, partially inactivated, or completely inactivated(non-limiting examples include, for example, the Cas13 protein with a mutated HEPN domain or the Cas13 protein that is catalytically inactivated) is covalently linked or fused to a translation repression domain to inhibit or reduce the expression of the target RNA. Non-limiting examples of translation repression domains include Pumilio or FBF PUF proteins, deadenosylase, CAF1, and Argonaute proteins. In other embodiments, the translation repression domain is covalently linked or fused to an adaptor protein capable of binding an aptamer sequence inserted into or attached to the guiding polynucleotide, thereby allowing the translation repression domain to be non-covalently linked to the RNA-guided nuclease complexed with the guiding polynucleotide.

In some embodiments of this disclosure, the RNA-guided nuclease is covalently linked or fused to an RNA methylation domain. In some embodiments, an RNA-guided nuclease that retains its activity, is partially inactivated, or is completely inactivated(non-limiting examples include, for instance, the Cas13 protein having a mutated HEPN domain or the Cas13 protein that is catalytically inactivated) is covalently linked or fused to an RNA methylation domain for methylation of a target RNA. Non-limiting examples of RNA methylation domains include an m6A domain, such as METTL14, METTL3, or WTAP. In other embodiments, the RNA methylation domain is covalently linked or fused to an adaptor protein capable of binding to an aptamer sequence inserted into or attached to the guiding polynucleotide, thereby allowing the RNA methylation domain to be non-covalently linked to the RNA-guided nuclease complexed with the guiding polynucleotide.

In some embodiments of this disclosure, the RNA-guided nuclease is covalently linked or fused to an RNA demethylation domain. In some embodiments, an RNA-guided nuclease that is activity-retained, partially inactivated, or completely inactivated(non-limiting examples include, for instance, the Cas13 protein having a mutated HEPN domain or the Cas13 protein that is catalytically inactivated) is covalently linked or fused to an RNA demethylation domain for the demethylation of a target RNA. Non-limiting examples of RNA demethylation domains include human alkylation repair homolog 5 or ALKBH5. In other embodiments, the RNA demethylation domain is covalently linked or fused to an adaptor protein capable of binding an aptamer sequence inserted into or attached to the guiding polynucleotide, thereby allowing the RNA demethylation domain to be non-covalently linked to the RNA-guided nuclease complexed with the guiding polynucleotide.

In some embodiments of this disclosure, the RNA-guided nuclease is covalently linked or fused to a ribonuclease domain. In some embodiments, an RNA-guided nuclease that retains, partially inactivates, or completely inactivates nuclease activity(non-limiting examples include, for instance, the Cas13 protein with a mutated HEPN domain or the Cas13 protein that is catalytically inactivated) is covalently linked or fused to a ribonuclease domain to cleave target RNA. Non-limiting examples of ribonuclease domains include a PIN endonuclease domain, an NYN domain, an SMR domain from SOT1, or an RNase domain from staphylococcal nucleases.

In some embodiments of this disclosure, the RNA-guided nuclease is covalently linked or fused to an affinity tag, affinity domain, reporter tag, or reporter domain. In some embodiments, the RNA-guided nuclease is covalently linked or fused to a reporter domain, such as a fluorescent protein. Non-limiting examples of reporter domains include GST, HRP, CAT, GFP, HcRed, DsRed, CFP, YFP, and BFP. In some embodiments, the RNA-guided nuclease is covalently linked or fused to an affinity tag, such as a purification tag. Non-limiting examples of affinity tags include HA-tags, His-tags(e.g., 6-His), Myc-tags, E-tags, S-tags, calmodulin tags, FLAG-tags, GST-tags, MBP-tags, Halo tags, or biotin.

In some embodiments disclosed herein, the affinity label and the affinity field are used interchangeably, and the report label and the report field are used interchangeably.

In some embodiments of this disclosure, the RNA-guided nuclease is covalently linked to the fusion domain with or without a linker sequence; that is, the RNA-guided nuclease is directly covalently linked to the fusion domain(without a linker sequence), or it can be covalently linked with a linker sequence. Typically, the linker sequence consists of 1-100, 1-50, 1-30, 1-20, 1-10, or 1-5 amino acids.

In some embodiments of this disclosure, the guide RNA is able to form a complex with the RNA-guided nuclease and guide the complex to bind specifically to the target RNA.

In some embodiments of this disclosure, the guide RNA is able to form a complex with the RNA-guided nuclease and guide the complex sequence to specifically bind to and cleave the target RNA.

**Cas protein**

In some embodiments of this disclosure, the Cas protein is a Cas9 protein, a Cas12 protein, or a Cas13 protein. In some embodiments, the Cas protein is a Cas12a protein, a Cas12b protein, a Cas12c protein, a Cas12d protein, a Cas12e protein, a Cas12f protein, a Cas12g protein, a Cas12h protein, a Cas12i protein, a Cas12j protein, or a Cas12k protein.

In some embodiments of this disclosure, the Cas protein is selected from SpCas9, SaCas9, Nme2Cas9, Nme3Cas9, CjCas9, NmCas9, FnCas9, PpnCas9, FrCas9, SauCas9, SauriCas9, ScaCas9, St1Cas9, BlatCas9, CdiCas9, GeoCas9, fragments thereof, and mutants or fragments thereof. The Cas12 protein is selected from AsCpf1, LbCas12a, AkCas12b, AacCas12b, PlmCasX, DpbCasX, Cas12i1, Cas12i2, Cas12i3, Casφ-2.

In some embodiments disclosed herein, the Cas protein is optionally selected from AsCpf1, enAsCas12a(addgene plasmid #196724), dFnCas12a(addgene plasmid #136379), ErCas12a, LbCas12a D832A, LbCas12a H759A, LbCas12a E795L, FnCas12a3, FnCas12a D917A, AsCas12a R1226A, AsCas12a D908A, AsCas12a E174R/S542R, AsCas12a(S542R/K548V/N552R), PrCas12a, PxCas12a, PcCas12a, PdCas12a, Mb2Cas12a, Mb3Cas12a, MlCas12a, CMaCas12a, CMtCas12a, HkCas12a, Lb5Cas12a, ErCas12a, TsCas12a, FnCpf1, LbCas12a, ttHsCas12a, AaCas12b, AaCas12b D570A, AaCas12b Q119F/E475R/E758R, BhCas12b, BvCas12b, BrCas12b, AkCas12b, AmCas12b, BsCas12b, OspCas12c, Cas12c2(addgene plasmid #183072), Cas12c_4(addgene plasmid #183071), Cas12c1(addgene plasmid #120872), CasY.1(from Katanobacteria), CasY.2(from Vogelbacteria), CasY.3(from Vogelbacteria), CasY.4(from Parcubacteria), CasY.5(from Komeilibacteria), CasY.6(from Kerfeldbacteria), PlmCasX, DpbCasX, Un1-Cas12f, CnCas12f1, enRhCas12f1, AsCas12f1, SpaCas12f1, Cas12g1(addgene plasmid #120879), Cas12h of WO2021113522A1, Cas12i1( addgene plasmid #171670), Cas12i2(addgene plasmid #171670). #188275), Cas12i1(adgene plasmid #120882) Cas12i2(adgene plasmid #120883) CN111757889B a strain of the Cas12f.4/Cas12f.5/Cas12f.6 gene. dSiCas12i(D1049A), SiCas12i, Si2Cas12i, WiCas12i, Wi2Cas12i, Wi3Cas12i, SaCas12i, Sa2Cas12i, Sa3Cas12i, WaCas12i Wa2Cas12i, xCas12i, hfCas12Max, Cas12i-Max(adgene plasmid #188276), Cas12i1 D647A(adgene plasmid #171671), Cas12i-HiFi(adgene plasmid). #188269) Cas12i1 D647A) Cas12j3(adgene plasmid #188497) Cas12j2(adgene plasmid #188498) AsCas12j-2(adgene plasmid #191655).) Cas12j-8(adgene plasmid #194966) ShCas12k(N7-Cas12k) AcCas12k) Cas12k-TniQ(adgene plasmid #181787) Cas12k-TnsC(adgene plasmid #181789) Cas12l, MmCas12m, MmCas12mΔZF(H549A, C552A), dCas12m-ΔZF(D485A, H549A, C552A), AcCas12n; dAcCas12n(D240), TnpB Actinomature_cellulosilytica_strain_DSM_45823, TnpB Actinomature_namibiensis_strain_DSM_44197, TnpB Actinomature_umbrina_strain_DSM_43927_$, TnpB Actinoplanes_lobatus_strain_DSM_43150(TnpB-1 and TnpB-2) TnpB Alicyclobacillus_macrosporagiidus_strain_DSM_17980 | Lipingzhangella_halophila_strain_DSM_102030, TnpB Meiothermus_Silvanus_DSM_9946, TnpB QNFX01000004, ISDra2 TnpB(PDB: 8H1J), KraIscB-1, AwaIscB; OgeuIscB, GtFz1(from Guillardia theta), SpuFz1(from Spizellomyces punctatus), NlovFz2(from Percolozoa Naegleria lovaniensis).MmeFz2(from Mercenaria mercenaria), fragments of them, and their mutants or fragments of mutants.

In some embodiments of this disclosure, the Cas13 protein is a Cas13a protein, a Cas13b protein, a Cas13c protein, or a Cas13d protein. In some embodiments of this disclosure, the Cas protein is a Cas13a protein, a Cas13b protein, a Cas13c protein, or a Cas13d protein with mutations in both HEPN domains, and these Cas proteins have completely or partially lost their cleavage activity. For example: LwaCas13a, LsCas13a, LbuCas13a, dLbuCas13a(R472-A/H477A/R1048A/H1053A), TccCas13a, LneCas13a(LneC2c2), LbmCas13a, LbnCas13a, PpCas13a, LbfCas13a, CgCas13a, Cg2Cas13a, PspCas13b, PspCas13b H133A/H1058A, PbuCas13b, PgiCas13b, BzCas13b, RanCas13b, PguCas13b, dPguCas13b(H151A/H1121A), Cas13bt1, Cas13bt3, CcaCas13b, MisCas13b, Hgm4Cas13b, Pba4-Cas13b, Bba2Cas13b, CasRx, dCasRx(R239A/H244A/R858A/H863A), CasRx_N2V8(A134V,A140V,A141V,A143V), RspCas13d, C13-2.

In some embodiments of this disclosure, the Cas13 protein is the Cas13d protein.

In some embodiments of this disclosure, the Cas13 protein has at least 50%, at least 55%, at least 60%, at least 65 %, at least 70%, at least 75%, at least 80%, at least 85%, at least 90 %, at least 95%, at least 96%, at least 97%, at least 98%, at least 99 %, or 100% sequence identity with the CasRx or C13-2 protein.

In some embodiments of this disclosure, the Cas13 protein is a CasRx protein. In some embodiments, the Cas13 protein is dCasRx in which both HEPN domains carry mutations(R239A and H244A of HEPN-1 and R858A and H863A of HEPN-2).

In some embodiments of this disclosure, the Cas13 protein comprises the sequence shown in SEQ ID NO: 1.

In some embodiments of this disclosure, the amino acid sequence of the Cas13 protein has at least 50%, at least 55 %, at least 60 %, at least 65%, at least 70%, at least 75%, at least 80%, at least 85%, at least 90%, at least 95%, at least 96 %, at least 97%, at least 98 %, at least 99 %, or 100% sequence identity with the sequence shown in SEQ ID NO: 1.

In some embodiments of this disclosure, the Cas13 protein is the C13-2 protein. In some embodiments, the Cas13 protein is the dead C13-2 protein.

In some embodiments, the Cas13 protein contains one, two, three, four, five, or six mutations at the corresponding positions of amino acid residues R210, H215, R750, H755, R785, and/or H790 of the reference protein shown in SEQ ID NO: 1(C13-2). In some embodiments, the Cas13 protein is mutated to A(alanine) at the corresponding positions of amino acid residues R210, H215, R750, H755, R785, and/or H790 of the reference protein shown in SEQ ID NO: 1.

In some embodiments, the Cas13 protein contains mutations at the corresponding positions of amino acid residues R210 and H215 of the reference protein shown in SEQ ID NO: 1. In some embodiments, the Cas13 protein contains mutations at the corresponding positions of amino acid residues R750 and H755 of the reference protein shown in SEQ ID NO: 1. In some embodiments, the Cas13 protein contains mutations at the corresponding positions of amino acid residues R785 and H790 of the reference protein shown in SEQ ID NO: 1.

In some embodiments, the Cas13 protein contains mutations at the corresponding positions of amino acid residues R210, H215, R750 and H755 of the reference protein shown in SEQ ID NO: 1.

In some embodiments, the Cas13 protein contains mutations at the corresponding positions of amino acid residues R750, H755, R785 and H790 of the reference protein shown in SEQ ID NO: 1.

In some embodiments, the Cas13 protein contains mutations at the corresponding positions of amino acid residues R210, H215, R785 and/or H790 of the reference protein shown in SEQ ID NO: 1.

In some embodiments, the Cas13 protein contains mutations at the corresponding positions of amino acid residues R210, H215, R750, H755, R785 and H790 of the reference protein shown in SEQ ID NO: 1.

In some embodiments, the corresponding positions of R210, R750, or R785 are abruptly changed to A. In some embodiments, the corresponding positions of H215, H755, or H790 are abruptly changed to A. In some embodiments, the corresponding positions of R210, H215, R750, H755, R785, and H790 are all abruptly changed to A.

In some embodiments, the Cas13 protein is obtained by introducing mutations into the RxxxxH motifs at positions 210-215, 750-755, and/or 785-790 of the sequence shown in SEQ ID NO: 1.

In some embodiments, the Cas13 protein is obtained by introducing one, two, three, four, five, or six mutations at positions R210, H215, R750, H755, R785, and/or H790 of the sequence shown in SEQ ID NO: 1. In some embodiments, the Cas13 protein is obtained by mutating to A(alanine) at positions R210, H215, R750, H755, R785, and/or H790 of the sequence shown in SEQ ID NO: 1.

In some embodiments, the Cas13 protein is obtained by mutating the R210, H215, R785, and H790 positions of the sequence shown in SEQ ID NO: 1 to A. In some embodiments, the Cas13 protein is obtained by mutating the R210, H215, R750, and H755 positions of the sequence shown in SEQ ID NO: 1 to A. In some embodiments, the Cas13 protein is obtained by mutating the R750, H755, R785, and H790 positions of the sequence shown in SEQ ID NO: 1 to A. In some embodiments, the Cas13 protein is obtained by mutating the R210, H215, R750, H755, R785, and H790 positions of the sequence shown in SEQ ID NO: 1 to A.

In some embodiments, compared with the reference protein shown in SEQ ID NO: 1, the Cas13 protein contains any one or more mutations at the corresponding positions of the following amino acid residues of the reference protein shown in SEQ ID NO: 1 : R11, N34, R35, R47, R58, R63, R64, N68, N87, N265, N274, R276, R290, R294, N299, N303, R308, R314, R320, R328, N332, R341, N346, R358, N372, N383, N390, N394, R47+R290, R47+R314, R290+R314, R47+R290+R314, R308+N68, N394+N68, N87+N68, R308+N265, N394 +N265, N87+N265, R308+N68+N265, N87+N68+N265, T7, A16, S260, A263, M266, N274, F288, M302, N303, L304, V305, I311, D313, H324, P326, H327, N332, N346, T353, T360, E365, A373, M380, S382, K395, Y396, D402, D411, S418.

In some embodiments, the Cas13 protein is obtained by introducing any one or more mutations at the following positions into the sequence shown in SEQ ID NO: 1 : R11, N34, R35, R47, R58, R63, R64, N68, N87, N265, N274, R276, R290, R294, N299, N303, R308, R314, R320, R328, N332, R341, N346, R358, N372, N383, N390, N394, R47+R290, R47+R314, R290+R314, R47 +R290+R314. R308+N68, N394+N68, N87+N68, R308+N265, N394+N265, N87+N265, R308+N68+N265, N87+N68+N265, T7, A16, S260, A263, M266, N274, F288, M302, N303, L304, V305, I311, D313, H324, P326, H327, N332, N346, T353, T360, E365, A373, M380, S382, K395, Y396, D402, D411, S418.

In some embodiments, the C13-2 protein, similar proteins of the C13-2 protein, mutants of the C13-2 protein, and their functional fragments or fusion proteins may form a complex with the homologous repeat sequence containing the sequence shown in SEQ ID NO: 2 or 3 as disclosed herein, thereby specifically targeting the target RNA of the present disclosure under the guidance of the guide sequence.

In some embodiments of this disclosure, the Cas protein comprises any one or more of the following fusion domains: subcellular localization signal, deaminase domain, translation activation domain, translation repression domain, RNA methylation domain, RNA demethylation domain, nuclease domain, splicing factor domain, reporter tag, and affinity tag.

In some embodiments of this disclosure, the Cas protein contains a subcellular localization signal.

In some embodiments of this disclosure, the Cas protein includes a subcellular localization signal and a deaminase domain.

In some embodiments of this disclosure, the subcellular localization signal may be selected from the nuclear localization signal and the nuclear output signal.

In some embodiments of this disclosure, the Cas protein is covalently linked to the fusion domain with or without a linker sequence; that is, the Cas protein is directly covalently linked to the fusion domain(without a linker sequence), or it can be covalently linked with a linker sequence. Typically, the linker sequence consists of 1-100, 1-50, 1-30, 1-20, 1-10, or 1-5 amino acids.

In some embodiments of this disclosure, the guide RNA is able to form a CRISPR complex with the Cas protein and guide the CRISPR complex to bind to the target RNA in a sequence-specific manner.

In some embodiments of this disclosure, the guide RNA is able to form a CRISPR complex with the Cas protein and guide the CRISPR complex sequence to specifically bind to and cleave the target RNA.

## Nucleotide sequence encoding guide RNA or RNA-guided nuclease

In some embodiments, the nucleotide sequence encoding the RNA-guided nuclease is a plasmid. In some embodiments, the nucleotide sequence encoding the RNA-guided nuclease is part of a viral vector genome, such as the DNA genome of an AAV vector flanked by an ITR. In some embodiments, the nucleotide sequence encoding the RNA-guided nuclease is mRNA.

In some embodiments of this disclosure, the nucleotide sequence encoding the guide RNA or RNA-guided nuclease is codon-optimized.

In some embodiments of this disclosure, the nucleotide sequence encoding the guide RNA is DNA. In some embodiments, the DNA sequence is codon-optimized.

In some embodiments of this disclosure, the nucleotide sequence encoding the RNA-guided nuclease is DNA or mRNA. In some embodiments, the DNA sequence is codon-optimized. In some embodiments, the mRNA sequence is codon-optimized.

In some embodiments of this disclosure, codon optimization is performed for expression in a desired cell type. In some embodiments, codon optimization is performed for expression in a eukaryotic cell environment. In some embodiments, codon optimization is performed for expression in a mammalian cell environment. In some embodiments, codon optimization is performed for expression in a human cell environment.

Generally, codon optimization refers to modifying a nucleic acid sequence to enhance its expression in the host cell of interest by replacing at least one codon of the original sequence(e.g., about 1, 2, 3, 4, 5, 10, 15, 20, 25, 50 or more codons) with codons that are more frequently used in the gene of the host cell while maintaining the original amino acid sequence. Computational programs or algorithms for codon optimization are also available, such as those using tools or algorithms like ExpOptimizer, Codon OptimWiz, NGTM Codon, Codon optimization, Synthetic Gene Designer, and DNAWorks, etc.

## Vector(s)

Vectors can contain any type of nucleotides, including but not limited to DNA and RNA, which can be single-stranded or double-stranded, can be partially derived from natural sources, and can contain natural, non-natural, or altered nucleotides. Suitable vectors include those designed for expression, such as plasmids and viruses.

In some embodiments, the recombinant vector contains regulatory sequences, such as transcription and translation start codons and stop codons, which are specific to the host cell type to which the vector is to be introduced(e.g., bacteria, fungi, plants or animals), depending on the case.

In some embodiments, the recombinant vector optionally includes gene vector elements(nucleic acids), such as optional marker regions, lactose operons, CMV promoters, CAG promoters, tac promoters, T7 RNA polymerase promoters, SP6 RNA polymerase promoters, SV40 promoters, IRES sequences, WPRE elements, ITR sequences, FLAG tag coding regions, c-myc tag coding regions, polyHis tag coding regions, HA tag coding regions, MBP tag coding regions, GST tag coding regions, ployA coding regions, SV40 polyadenylation signals, SV40 origin of replication, Col E1 origin of replication, loxP sites, or Cre recombinase coding regions.

In some embodiments, the coding sequence in the vector is codon-optimized for expression in prokaryotic cells(e.g., bacteria) or eukaryotic cells(e.g., mammalian cells, human cells).

## Regulatory sequence

In some embodiments of this disclosure, the regulatory sequence comprises one or more pol III promoters(e.g., 1, 2, 3, 4, 5, or more pol III promoters), one or more pol II promoters(e.g., 1, 2, 3, 4, 5, or more pol II promoters), one or more pol I promoters(e.g., 1, 2, 3, 4, 5, or more pol I promoters), or combinations thereof.

In some embodiments of this disclosure, the regulatory sequence is a U6 promoter or an eye-specific promoter.

In some embodiments of this disclosure, the regulatory sequence is a CBh promoter. In some embodiments, the CBh promoter contains a CMV enhancer sequence. In some embodiments, the CBh promoter contains a chicken β-actin promoter. In some embodiments, the CBh promoter contains a hybrid intron.

In some embodiments of this disclosure, the regulatory sequence comprises an HRE enhancer element( hypoxia response element). In some embodiments, the regulatory sequence comprises at least two, at least three, at least four, at least five, at least six, at least seven, at least eight, or at least nine HRE enhancer elements in series. In some embodiments, the regulatory sequence comprises at least two, three, four, five, six, seven, eight, or nine HRE enhancer elements in series.

In some embodiments of this disclosure, the regulatory sequence comprises an NRS element(neuronal restrictive silencer) from the human synapsin gene. In some embodiments, the regulatory sequence comprises an NRS element and an HRE enhancer element. In some embodiments, the regulatory sequence comprises tandem NRS elements and HRE enhancer elements. To enhance the regulatory effect, the tandem NRS elements and HRE enhancer elements may be repeated multiple times. In some embodiments, the regulatory sequence comprises tandem NRS elements and HRE enhancer elements repeated at least 2, 3, 4, 5, or 6 times consecutively. In some embodiments, the regulatory sequence comprises tandem NRS elements and HRE enhancer elements repeated 2, 3, 4, 5, or 6 times consecutively.

**Promoter**

In some embodiments of this disclosure, the vector comprises a pol III promoter(e.g., U6 and H1 promoters), a pol II promoter(e.g., a retroviral Rous sarcoma virus(RSV) LTR promoter(optionally with an RSV enhancer), a cytomegalovirus(CMV) promoter(optionally with a CMV enhancer), an SV40 promoter, a dihydrofolate reductase promoter, a β-actin promoter, a glycerol phosphokinase(PGK) promoter, or an EF1α promoter), or a pol III promoter and a pol II promoter.

In some embodiments of this disclosure, the promoter is a constitutive promoter that is continuously active and not regulated by external signals or molecules. Suitable constitutive promoters include, but are not limited to, CMV, RSV, SV40, EF1α, CAG, and β-actin promoters. In some embodiments, the promoter is an inducible promoter regulated by external signals or molecules(e.g., transcription factors).

In some embodiments of this disclosure, the promoter is a tissue-specific promoter that can be used to drive tissue-specific expression of the Cas13 protein. Suitable muscle-specific promoters include, but are not limited to, CK8, MHCK7, myoglobin promoter(Mb), desmin promoter, muscle creatine kinase promoter(MCK) and its variants, and SPc5-12 synthesis promoter. Suitable immune cell-specific promoters include, but are not limited to, B29 promoter(B cells), CD14 promoter(monocytes), CD43 promoter(leukocytes and platelets), CD68(macrophages), and SV40/CD43 promoter(leukocytes and platelets). Suitable blood cell-specific promoters include, but are not limited to, CD43 promoter(leukocytes and platelets), CD45 promoter(hematopoietic cells), INF-β(hematopoietic cells), WASP promoter(hematopoietic cells), SV40/CD43 promoter(leukocytes and platelets), and SV40/CD45 promoter(hematopoietic cells). Suitable pancreas-specific promoters include, but are not limited to, the elastase-1 promoter. Suitable endothelial cell-specific promoters include, but are not limited to, the Fit-1 promoter and the ICAM-2 promoter. Suitable neuronal tissue/cell-specific promoters include, but are not limited to, the GFAP promoter(astrocytes), the SYN1 promoter(neurons), and the NSE/RU5' promoter(mature neurons). Neuronal tissue/cell-specific promoters can be the GFAP promoter or the SYN1 promoter. Suitable kidney-specific promoters include, but are not limited to, the NphsI promoter(podocytes). Suitable bone-specific promoters include, but are not limited to, the OG-2 promoter(osteoblasts, odontoblasts). Suitable lung-specific promoters include, but are not limited to, the SP-B promoter(lung). Suitable liver-specific promoters include, but are not limited to, the SV40/Alb promoter. Suitable heart-specific promoters include, but are not limited to, α-MHC.

In some embodiments of this disclosure, the promoter is an eye-specific promoter.

In some embodiments of this disclosure, the eye-specific promoter is selected from: retinal-specific promoter, K12 promoter, rhodopsin promoter, rod cell-specific promoter, cone cell-specific promoter, rhodopsin kinase promoter, GRK1 promoter, interphotoreceptor retinoid-binding protein proximal(IRBP) promoter, and opsin promoters(e.g., red opsin promoter, blue opsin promoter, etc.).

In some embodiments of this disclosure, the promoter is a chicken β-actin(CB) promoter. The chicken β-actin promoter may be a short chicken β-actin promoter or a long chicken β-actin promoter. In some embodiments, the promoter(e.g., a chicken β-actin promoter) includes an enhancer sequence, such as a cytomegalovirus(CMV) enhancer sequence. The CMV enhancer sequence may be a short CMV enhancer sequence or a long CMV enhancer sequence. In some embodiments, the promoter includes a long CMV enhancer sequence and a long chicken β-actin promoter. In some embodiments, the promoter includes a short CMV enhancer sequence and a short chicken β-actin promoter. However, those skilled in the art will recognize that a short CMV enhancer can be used with a long CB promoter, and a long CMV enhancer can be used with a short CB promoter. In some embodiments of this disclosure, the promoter is a CBh promoter.

**Enhancer**

In some embodiments of this disclosure, the enhancer is selected from WPRE, CMV enhancer, SV40 enhancer, and intron sequence between exons 2 and 3 of rabbit β- globulin.

In some embodiments of this disclosure, the enhancer is located upstream of the promoter element; however, it may also be located downstream or internal to the coding sequence regulated by the promoter and retain its function. Thus, the enhancer or a portion thereof may be present in the RNA sequence transcribed from the coding sequence.

In some embodiments of this disclosure, the enhancer may be located in 100, 200, 300, 400, 500 or more base pairs upstream or downstream of the coding sequence regulated by the promoter.

In some embodiments of this disclosure, the enhancer increases the expression of the coding sequence to a higher level than that provided by the promoter.

## Delivery system

In some embodiments of this disclosure, the RNA-encoding nuclease or the polynucleotide sequence of the guiding RNA is codon-optimized for expression in eukaryotic cells.

In some embodiments of this disclosure, the nuclease encoding the RNA or the polynucleotide sequence of the guiding RNA is codon-optimized for expression in mammalian cells.

In some embodiments of this disclosure, the RNA-encoding nuclease or the polynucleotide sequence of the guide RNA is codon-optimized for expression in human cells.

In some embodiments of this disclosure, the RNA-encoding nuclease or the polynucleotide sequence of the guide RNA is codon-optimized for expression in prokaryotic cells.

In some embodiments of this disclosure, the RNA-encoding nuclease or the polynucleotide sequence of the guiding RNA is codon-optimized for expression in bacterial cells.

In some embodiments of this disclosure, the nucleic acid molecule encoding the RNA-guided nuclease or the guide RNA is a plasmid. In some embodiments, the nucleic acid molecule encoding the RNA-guided nuclease or the guide RNA is part of a viral vector genome, a non-limiting example being the DNA genome of an AAV vector flanked by an ITR. In some embodiments, the nucleic acid molecule encoding the RNA-guided nuclease or the guide RNA is mRNA.

## Adeno-associated virus vector(AAV vector)

The delivery of CRISPR-Cas systems via AAV vectors is described in Maeder et al., Nature Medicine 25: 229-233(2019), which is incorporated herein by reference in its entirety. In some embodiments disclosed herein, the AAV vector contains an ssDNA genome containing an RNA-guided nuclease side-linked to an ITR and a coding sequence for the guide RNA.

In some embodiments of this disclosure, the guide RNA or gene editing system described herein is packaged in an AAV vector, for example, packaged in a capsid of AAV1, AAV2, AAV3, AAV4, AAV5, AAV6, AAV7, AAV8, AAV9, AAV10, AAV11, AAV12, AAV13, AAV PHP.B, AAV PHP.B2, AAV PHP.B3, AAV PHP.A, AAV PHP.eB, AAV PHP.eS, AAV2.7m8, AAV8.7m8, AAV ShH10, AAVrh10, or AAVrh74.

In some embodiments of this disclosure, the guide RNA or gene editing system described herein is packaged in an AAV2, AAV5, AAV6, AAV8, AAV9 or AAVPHP.eB capsid.

In some embodiments disclosed herein, the AAV vector described herein may be selected from: AAV2/2, AAV2/3, AAV2/4, AAV2/5, AAV2/6, AAV2/7, AAV2/8, AAV2/9, AAV2/10, AAV2/11, AAV2/12, AAV2/13, AAV2/PHP.B, AAV2/PHP.B2, AAV2/PHP.B3, AAV2/PHP.A, AAV2/PHP.eB, AAV2/PHP.eS, AAV2/2.7m8, AAV2/8.7m8, AAV2/ShH10, AAV2/rh10 and AAV2/rh74.

In some embodiments disclosed herein, the AAV vector described herein may be selected from: AAV2/2, AAV2/5, AAV2/6, AAV2/8, AAV2/9, AAV2/PHP.eB.

In some embodiments, the gene editing system described herein is packaged in an AAV vector containing an engineered capsid with tissue tropism, such as an engineered eye tissue tropism capsid.

## Lipid nanoparticles

Gillmore et al., N. Engl. J. Med., 385: 493-502(2021) describes LNP delivery using the CRISPR-Cas system, the full text of which is incorporated herein by reference. In some embodiments, in addition to the RNA payload(Cas13 mRNA and guide RNA), the lipid nanoparticles(LNPs) comprise four components: cationic or ionizable lipids, cholesterol, cofactor lipids, and PEG-lipids. In some embodiments, the cationic or ionizable lipids include cKK-E12, C12-200, ALC-0315, DLin-MC3-DMA, DLin-KC2-DMA, FTT5, Moderna SM-102, and Intellia LP01. In some embodiments, the PEG-lipids comprise PEG-2000-C-DMG, PEG-2000-DMG, or ALC-0159. In some embodiments, the cofactor lipids include DSPC. The components of LNP are described in Paunovska et al., Nature Reviews Genetics 23: 265-280(2022), which is incorporated herein by reference in its entirety.

## Lentiviral vector

In some embodiments of this disclosure, the lentiviral vector is pseudotyped with a homologous or heterologous envelope protein such as VSV-G. In some embodiments, the mRNA encoding an RNA-guided nuclease is linked to an aptamer sequence.

**RNP complex(ribonucleoprotein complex)**

In some embodiments of this disclosure, the RNP complex(ribonucleoprotein complex) can be delivered to eukaryotic cells, mammalian cells, or human cells by microinjection or electroporation. In some embodiments, the ribonucleoprotein complex can be packaged in virus-like particles and delivered in vivo to mammalian or human subjects.

**Virus-like particles**

In some embodiments of this disclosure, the engineered virus-like particles(VLPs) are pseudotyped with homologous or heterologous envelope proteins such as VSV-G. In some embodiments, the RNA-guided nuclease is fused to a gag protein(e.g., MLVgag) via a cleavable linker, wherein cleavage of the linker in the target cell exposes an NLS located between the linker and the RNA-guided nuclease. In some embodiments, the fusion protein comprises(e.g., from 5' to 3') a gag protein(e.g., MLVgag), one or more NES, a cleavable linker, one or more NLS, and an RNA-guided nuclease. In some embodiments, the RNA-guided nuclease is fused to a first dimerizing domain capable of dimerizing or heterodimerizing with a second dimerizing domain fused to the membrane protein, wherein the presence of a ligand promotes the dimerization and enriches the RNA-guided nuclease or fusion protein into the VLP.

**cells**

The cells disclosed herein may be isolated cells. The cells disclosed herein(e.g., which can be used to generate cell-free systems) may be eukaryotic or prokaryotic. Examples of such cells include, but are not limited to, bacterial, archaea, plant, fungal, yeast, insect, and mammalian cells, such as lactobacillus, lactococcus, Bacillus(e.g., Bacillus subtilis), Escherichia coli(e.g., Escherichia coli), Clostridium, yeast or Pichia pastoris(e.g., Saccharomyces cerevisiae or Pichia pastoris), Kluyveromyces lactis, Salmonella typhimurium, Drosophila cells, Caenorhabditis elegans cells, Xenopus laevis cells, SF9 cells, C129 cells, 293 cells, Neurospora, and immortalized mammalian cell lines(e.g., HeLa cells, bone marrow cell lines, and lymphoid cell lines).

In some embodiments, the cell is a prokaryotic cell, such as a bacterial cell, such as *Escherichia coli*. In some embodiments, the cell is a eukaryotic cell, such as a mammalian cell or a human cell. In some embodiments, the cell is a primary eukaryotic cell, stem cell, tumor/cancer cell, circulating tumor cell(CTC), blood cells(e.g., T cells, B cells, NK cells, Tregs, etc.), hematopoietic stem cells, specialized immune cells(e.g., tumor-infiltrating lymphocytes or tumor suppressor lymphocytes), or stromal cells in the tumor microenvironment(e.g., cancer-associated fibroblasts, etc.). In some embodiments, the cell is a brain or neuronal cell of the central or peripheral nervous system(e.g., neurons, astrocytes, microglia, retinal ganglion cells, rod/cone cells, etc.).

**Diseases or conditions related to target RNA**

In some embodiments of this disclosure, the disease or condition associated with the target RNA refers to a disease or condition caused by abnormally high expression of the target RNA, which is CTGF RNA, MITF RNA, SRD5A2 RNA or AR RNA.

The gene editing system disclosed herein that targets CTGF RNA, MITF RNA, SRD5A2 RNA or AR RNA can effectively knock down CTGF RNA, MITF RNA, SRD5A2 RNA or AR RNA, and therefore can be used to prevent, treat or diagnose these diseases.

In some embodiments, the pharmaceutical composition is delivered in vivo to a human subject. The pharmaceutical composition can be delivered by any effective route to deliver a therapeutically effective amount of the pharmaceutical composition to the subject in need. Exemplary routes of administration include, but are not limited to, intravenous infusion, intravenous injection, intraperitoneal injection, intramuscular injection, intratumoral injection, subcutaneous injection, intradermal injection, intraventricular injection, intravascular injection, cerebellar injection, intraocular injection, subretinal injection, intravitreal injection, intra-anterior chamber injection, intratympanic injection, intranasal administration, and inhalation.

In some embodiments of this disclosure, a therapeutically effective amount of the gene editing system or pharmaceutical composition described herein may be delivered to a subject in need using a suitable delivery method to achieve scar repair, skin whitening, reduction or elimination of melasma, prevention or treatment of melanoma, and/or downregulation of androgen receptor expression, ultimately for the prevention or treatment of androgenetic alopecia.

In some embodiments of this disclosure, the use of inhibitors, guide RNAs, nucleic acids, vectors, vector systems, adeno-associated virus vectors, lipid nanoparticles, lentiviral vectors, ribonucleoprotein complexes, virus-like particles, eukaryotic cells, and/or pharmaceutical compositions as described herein can downregulate MITF expression, thereby reducing melanin expression, reducing or eliminating melasma, achieving skin whitening effects, and/or treating melanoma. The literature Yi X, et al. MITF-siRNA formulation is a safe and effective therapy for human melasma[J].

Molecular Therapy, 2011, 19(2): 362-371. describes siRNA targeting MITF for the treatment of melasma, the full text of which is incorporated herein by reference.

In some embodiments of this disclosure, the use of inhibitors, guide RNAs, nucleic acids, vectors, vector systems, adeno-associated virus vectors, lipid nanoparticles, lentiviral vectors, ribonucleoprotein complexes, virus-like particles, eukaryotic cells, and/or pharmaceutical compositions as described herein can downregulate SRD5A2 expression and prevent or treat hair loss caused by androgenetic alopecia. The literature Khantham C, et al. Antioxidation, Anti-Inflammation, and Regulation of SRD5A Gene Expression of Oryza sativa cv. Bue Bang 3 CMU Husk and Bran Extracts as Androgenetic Alopecia Molecular Treatment Substances. Plants. 2022; 11(3): 330. https: //doi.org/10.3390/plants11030330 describes plant extracts that can downregulate SRD5A2 expression for AGA, the full text of which is incorporated herein by reference.

**Cosmetics**

In some embodiments of this disclosure, the cosmetic can be used to repair scars, whiten skin, reduce or eliminate melasma, prevent or treat melanoma, and/or prevent or treat androgenetic alopecia.

In some embodiments of this disclosure, the use of inhibitors, guide RNAs, nucleic acids, vectors, vector systems, adeno-associated virus vectors, lipid nanoparticles, lentiviral vectors, ribonucleoprotein complexes, virus-like particles, eukaryotic cells and/or pharmaceutical compositions as described herein can downregulate CTGF expression, prevent excessive collagen expression during wound repair, prevent excessive fibrosis of wounds leading to scar formation, and ultimately achieve the purpose of scar repair and/or skin rejuvenation. The literature Cho KH, et al. Local delivery of CTGF siRNA with poly(sorbitol-co-PEI) reduces scar contraction in cutaneous wound healing[J]. Tissue Engineering and Regenerative Medicine, 2017, 14(3): 211-220. describes the reduction of scar contraction in the skin wound healing process by siRNA targeting CTGF, the full text of which is incorporated herein by reference.

In some embodiments of this disclosure, the use of inhibitors, guide RNAs, nucleic acids, vectors, vector systems, adeno-associated virus vectors, lipid nanoparticles, lentiviral vectors, ribonucleoprotein complexes, virus-like particles, eukaryotic cells, and/or pharmaceutical compositions as described herein can downregulate MITF expression, thereby reducing melanin expression, reducing or eliminating melasma, achieving skin whitening effects, and/or treating melanoma. The literature Yi X, et al. MITF-siRNA formulation is a safe and effective therapy for human melasma[J]. Molecular Therapy, 2011, 19(2): 362-371. describes siRNA targeting MITF for the treatment of melasma, the full text of which is incorporated herein by reference.

In some embodiments of this disclosure, the use of inhibitors, guide RNAs, nucleic acids, vectors, vector systems, adeno-associated virus vectors, lipid nanoparticles, lentiviral vectors, ribonucleoprotein complexes, virus-like particles, eukaryotic cells, and/or pharmaceutical compositions as described herein can downregulate SRD5A2 expression and prevent or treat hair loss caused by androgenetic alopecia. The literature Khantham C, et al. Antioxidation, Anti-Inflammation, and Regulation of SRD5A Gene Expression of Oryza sativa cv. Bue Bang 3 CMU Husk and Bran Extracts as Androgenetic Alopecia Molecular Treatment Substances. Plants. 2022; 11(3): 330. https: //doi.org/10.3390/plants11030330 describes plant extracts that can downregulate SRD5A2 expression for AGA, the full text of which is incorporated herein by reference.

Based on common knowledge in the field, the above-mentioned preferred conditions can be combined arbitrarily to obtain the preferred embodiments of the present invention.

The present invention will be further illustrated below by way of examples, but the invention is not limited to the scope of the examples described herein. Experimental methods not specified in the following examples were performed according to conventional methods and conditions, or according to the product instructions.

**Examples**

In the following embodiments, in order to verify the effect of targeting each target RNA, the applicant's newly discovered highly active Cas13 protein, namely C13-2(also known as CasRfg.4), was used.

Amino acid sequence of C13-2(SEQ ID NO: 1):

MSKDKKKTKAKRMGVKALLAHGEDKLTMTTFGKGNRSKIEFTEGYHGRALETPKHFGIRGFEVRRIDENVDLCGDLEEG
KTIEALLVNPSEKVGEDYLKLKGTLEKRFFGREFPHDNIRIQLIYNILDIYKILGMNVADILYALGNMQDTELDIDMFGQS
LNNEDNLKECLKRMRPYMGYFGDIFKISPKGENIADREHNKKVLRCISVLRNATAHDKQDEYPWFKSSDIYETKIFKAD
MWKIIKDQYREKIKKVNKDFLSKNAVNMAILFDLLNARDVEQKKQITDEFYRFTIRKDGKNLGMNLVKIREIIIDRYASG
LRDKKHDPHRQKINVIADFLIFRALSQNQGIIDKTVSSLRLTKDEEEKDHVYQNAAELVWGMVSNCLTPYFNDPKNKYI
LKYKDAKTPGDFEDWITSKISEDDGEPFVKVLSFLCNFLEGKEINELLTAYIHKFECIQDFLNVISSLGENVQFQPRFALF
NNASFAQNVAVQLRILASIGKMKPDLTEAKRPLYKAAIRMLCPPEKWEKYTSDEWLEKNMLLNSEDRKNDKKKKQVNP
FRNFIAGNVIESRRFMYLVRYSKPKAVRAIMQNRSIVNYVLHRLPSEQVHRYASVFPENFADLEQEIDFLTKKLFEFSFE
ELLHEKDVILNNSRSHKPSLEIERLKAITGLYLSVAYIAIKNIVKANARYYIAFAVFERDKELVKAKDARIQTKIPETDFPD
YFCLTQYYLDRDEEKKFPGDPRDKEAFFEHLRKTKRHFSKQWREWLNEKIADAKSSQATGLLLREARNDVEHLNVLRA
IPDYIQDFRHGEKGETAMNSYFELYHYLMQRLMLKNTELDLSHWSGWIMRSGRPDRDLIQIAFVSLAYNLPRYRNLTK
EHHFDDTVLQKIREKESLD.

The DR(SEQ ID NO: 2) sequence corresponding to C13-2 is: GGAAGATAACTCTACAAACCTGTAGGGTTC TGAGAC.

The applicant also screened another DR with good results, named DR-hf2(SEQ ID NO: 3), with the sequence: GGAAGATAACTCTACAAACCTGTAGAGTTCTGAGAC.

**Example 1. Validation of endogenous CTGF gene editing efficiency**

1. Constructing an editing vector targeting the endogenous gene CTGF

The C13-2-BsaI plasmid with a universal crRNA scaffold expression cassette was synthesized at an outsourcing service company(SEQ ID NO: 4).

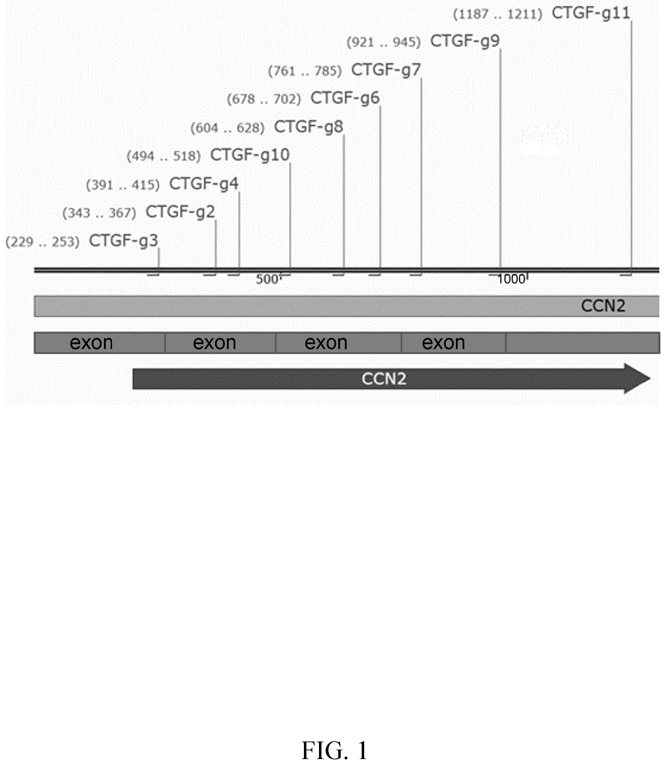

gRNAs were designed for human CCN2 mRNA(NCBI NM_001901.4, SEQ ID NO: 14), as shown in Table 1 and FIG. 1.

Table 1. DesigngRNA guide sequence

| Number of gRNA guide sequence | Guide sequence | SEQ ID NO |
| --- | --- | --- |
| CTGF-g2 | CAGCCGCAGCCGTCCAGCCACGAGGC | 5 |
| CTGF-g3 | GCGAGGAGGACCACGAAGGCGACGC | 6 |
| CTGF-g4 | TCGCGCTCGGTGCACAGCTCGCCCA | 7 |
| CTGF-g6 | CGTCACACACCCACTCCTCGCAGCA | 8 |
| CTGF-g7 | AATCATAGTTGGGTCTGGGCCAAAC | 9 |
| CTGF-g8 | AGACGAACGTCCATGCTGCACAGGG | 10 |
| CTGF-g9 | TCTCTTCCAGGTCAGCTTCGCAAGG | 11 |
| CTGF-g10 | ACCGAAGATGCAGGGAGCACCATCT | 12 |
| CTGF-g11 | TTCAAAGATGTCATTGTCTCCGGGA | 13 |

The sense and antisense strands of the DNA sequence were synthesized. The sense strand was formed by adding agac to the 5' end of the guide sequence shown in Table 1, and the antisense strand was formed by adding aaaa to the 5' end of the inverse complementary sequence of the guide sequence. Fragments targeting the CTGF RNA target site were obtained using primer annealing.

The primer annealing reaction system is shown in Table 2 below. It is incubated at 95°C for 5 minutes in a PCR instrument, and then immediately taken out and incubated on ice for 5 minutes to allow the primers to anneal each other to form double-stranded DNA with sticky ends.

Table 2. Primer annealing reaction system

| |
|---|
| Oligo-F(10 μM) 2 μl |
| Oligo-R(10 μM) 2 μl |
| 10× endonuclease reaction buffer * 2 μl |
| Total deionized water 20 μl |

The synthesized C13-2-BsaI plasmid was digested with the restriction enzyme *Bsa* I. The annealing product and the purified backbone after digestion were ligated by T4. After transformation into E. coli, positive clones were selected and the target plasmid, i.e. the verification vector(which can express C13-2 protein and gRNA targeting CTGF RNA), was extracted for subsequent experiments.

2. Transfection of 293T cells with the vector to be verified

Using a 293T cell line that highly expresses CTGF(293T-CTGF cells).

Cell line construction method: The vector Lv-CTGF-T2a-GFP(SEQ ID NO: 15) overexpressing the CTGF gene and the EGFP gene was constructed. CTGF and EGFP were separated by a 2A peptide. The Lv-CTGF-T2a-GFP plasmid was packaged into lentivirus and transduced into 293T cells to form a stable cell line overexpressing the CTGF gene.

Validation vectors carrying different target sites were transfected into 293T-CTGF cells. The negative control group was transfected with the C13-2-BsaI vector.

Transfection was performed in a 24- well plate according to the Lipofectamine 2000(Thermo) instructions.

3. qPCR detection

RNA was extracted from 293T-CTGF cells 72 h post-transfection using the SteadyPure Universal RNA Extraction Kit AG21017, and RNA concentration was detected using a micro-spectrophotometer. RNA products were reverse transcribed using the Evo M-MLV Mix Kit with gDNA Clean for qPCR, and the reverse transcription products were detected using the SYBR Green Premix Pro Taq HS qPCR Kit. A separate 293T-NC control group was also included: CTGF RNA levels in normal 293T cells(not expressing high levels of CTGF, and not transfected with the above gene-editing plasmids) were detected by qPCR.

The primers used for qPCR are shown below:

detection of CTGF(primers my): GCGTGTGCACCGCCAAAGAT(SEQ ID NO: 16), AACGTCCATGCTGCACAGGG (SEQ ID NO: 17);

detection of CTGF(primer 11): CAGCATGGACGTTCGTCTG(SEQ ID NO: 18), AACCACGGTTTGGTCCTTGG (SEQ ID NO: 19);

detection of internal references GAPDH: CCATGGGGAAGGTGAAGGTC(SEQ ID NO: 20), GAAGGGGTCATTG ATGGCAAC(SEQ ID NO: 21).

The reaction system was prepared according to the instructions for use of the SYBR® Green Premix Pro Taq HS qPCR Kit, and the detection was performed using the QuantStudio™ 5 Real-Time PCR System.

This experiment used a relative quantification method, namely the $2^{-\Delta\Delta Ct}$ method, to calculate the level of target RNA. The calculation method is shown below:

$$\triangle Ct = Ct(CTGF) - Ct(GAPDH)$$

$\triangle\triangle Ct = \triangle Ct$(sample to be verified, such as C13-2-CTGF-g2)$-\triangle Ct$(C13-2-BsaI)

$$2^{-\triangle\triangle Ct} = 2^{\wedge}(-\triangle\triangle Ct)$$

Calculate the $2^{-\Delta\Delta Ct}$ value of CTGF according to the above calculation method. Repeat the experiment multiple times and take the average value of the results.

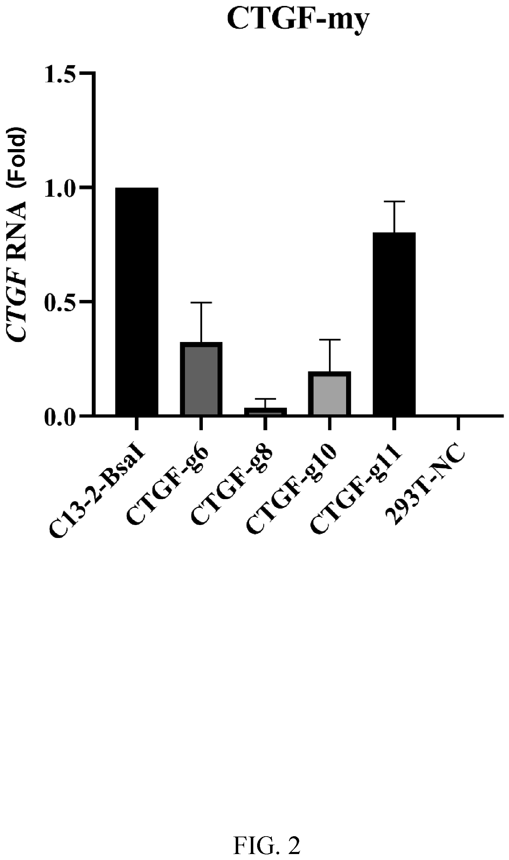

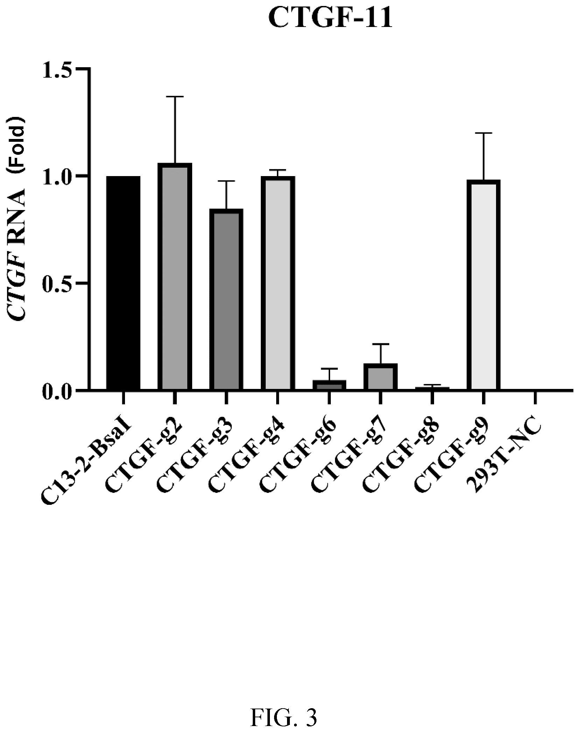

As shown in Figures 2 and 3. FIG. 2 shows the detection results using CTGF-my primers. FIG. 3 shows the detection results using CTGF-11 primers.

qPCR results showed that the combination of Cas13 protein with g6, g7, g8, and g10 gRNAs was highly efficient in editing CTGF RNA and could effectively downregulate CTGF expression, with statistically significant differences(P<0.0001). Editing efficiency: g8>g10>g6>g7. The combination of Cas13 protein with g2, g3, and g4 gRNAs was less efficient in editing CTGF RNA, or failed to successfully knock down CTGF RNA.

### Example 2. Validation of Endogenous MITF Gene Editing Efficiency

Editing efficiency was tested using a method that was essentially the same as in Example 1.

1. Constructing an editing vector targeting the endogenous gene MITF

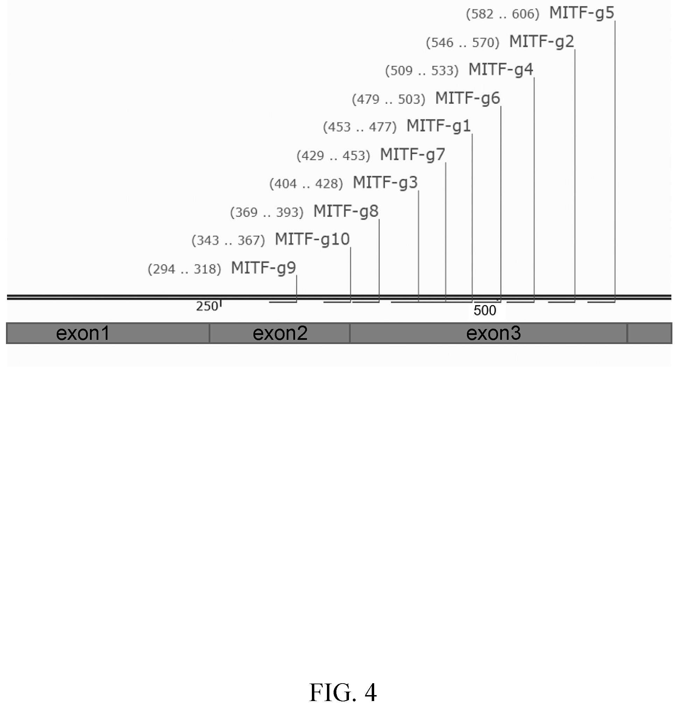

gRNAs were designed targeting human MITF mRNA(NCBI NM_001354607.2, SEQ ID NO: 22). As shown in Table 3 and FIG. 4.

Table 3. Guide sequences for the designed gRNA targeting MITF

| Number of gRNA guide sequence | Guide sequence | SEQ ID NO |
|---|---|---|
| MITF-g1 | CCTGCTCCTGCATCTGCTCACGCAT | 23 |
| MITF-g2 | GCACACTGACGTTTATGGCTGGTGT | 24 |
| MITF-g3 | TGATGTCATACTGGAGGAGCTTATC | 25 |
| MITF-g4 | CACTCTCTGTTGCATGAACTGGGCC | 26 |
| MITF-g5 | CCATCGGCACCTGCGTGGCAGAGGG | 27 |
| MITF-g6 | CAGCTTCTGTGCTGCTCCGTGC | 28 |
| MITF-g7 | TGAGTTGCTGGCGTAGCAAGATGCG | 29 |
| MITF-g8 | AGGCCCCAGGATGCTCGGCGGAACT | 30 |
| MITF-g9 | CCATAGGATCATCAGGTCATCTTTA | 31 |
| MITF-g10 | CTTGCCATTAAGGCACAAGAAGGGA | 32 |

The fragments targeting the MITF RNA target site were obtained using primer annealing in the same manner as in Example 1.
After digesting the C13-2-BsaI plasmid with the restriction enzyme BsaI, the annealing product and the purified backbone after digestion were ligated by T4. After transforming E. coli, positive clones were selected and the target plasmid was extracted, i.e., the verification vector(which can express C13-2 protein and gRNA targeting MITF RNA), for subsequent experiments.

2. Transfection of 293T cells with the vector to be verified

Validation vectors carrying different target sites were transfected into 293T cells. The negative control group was transfected with the C13-2-BsaI vector.
Transfection was performed in 24-well plates according to the Lipofectamine 2000(Thermo) instructions.

3. qPCR detection

72 h after transfection, qPCR was performed using the same method as in Example 1. A 293T blank control group was also set up : qPCR was used to detect the MITF RNA level in untreated 293T cells(untransfected plasmid).
The primers used for qPCR are shown below:

detection of MITF: GCCTCCAAGCCTCCGATAAG(SEQ ID NO: 33), GCACTCTCTGTTGCATGAACT(SEQ ID NO: 34);
detection of internal references GAPDH: CCATGGGGAAGGTGAAGGTC(SEQ ID NO: 20), GAAGGGGTCATTG ATGGCAAC(SEQ ID NO: 21).

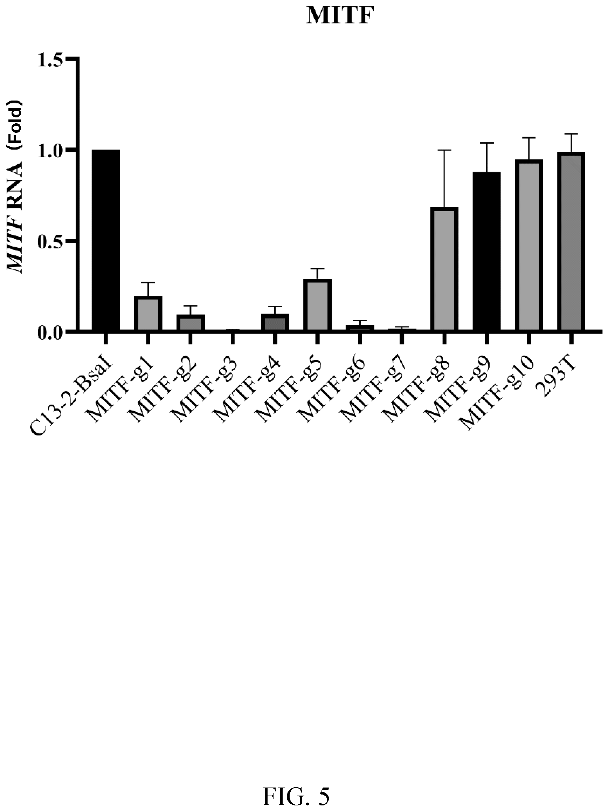

The level of the edited target RNA was calculated using the $2^{-\Delta\Delta Ct}$ method. The experiment was repeated multiple times, and the results were averaged. See FIG. 5.
qPCR results showed that the combination of Cas13 protein with g1, g2, g3, g4, g5, g6, and g7 gRNAs significantly improved the efficiency of MITF RNA editing and effectively downregulated MITF expression(P<0.0001).

The editing efficiency of Cas13 protein combined with g2, g3, g4, g6, and g7 gRNAs exceeded 90%, while the editing efficiency of Cas13 protein combined with g1 and g5 gRNAs was 80% and 71%, respectively. The editing efficiency of Cas13 protein combined with g8, g9, and g10 gRNAs was less than 35%.

**Example 3. Validation of endogenous SRD5A2 gene editing efficiency**

Editing efficiency was tested using a method that was essentially the same as in Example 1.

1. Construction of editing vectors and control vectors targeting the endogenous gene CTGF

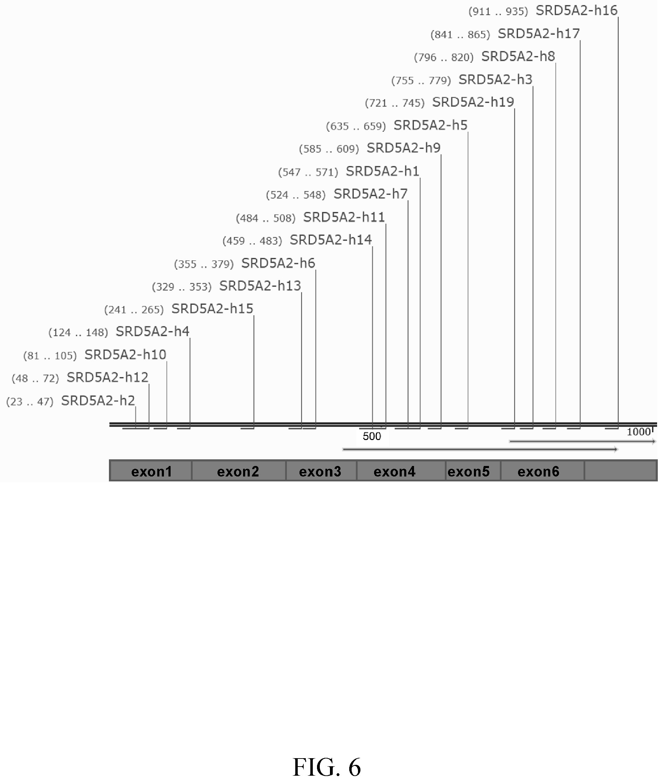

gRNAs were designed targeting human SRD5A2 mRNA(NCBI XM_011533072.3, SEQ ID NO: 35). As shown in Table 4 and FIG. 6.

Table 4. Designed gRNAs targeting SRD5A2

| Number of gRNA guide sequence | Guide sequence | SEQ ID NO |
|---|---|---|
| SRD5A2-h1 | CAGTAAATCAGATAGTAGCCTTGAA | 36 |
| SRD5A2-h2 | GTCTAATATAAGTTGGAAGCTGTAC | 37 |
| SRD5A2-h3 | GATCCATTCAATGATCTCACCGAGG | 38 |
| SRD5A2-h4 | TATGTGTGCATTTCTGAGGCCACTT | 39 |
| SRD5A2-h5 | ATGAATGTTTATTCCCATTCCCAAA | 40 |
| SRD5A2-h6 | CTCCCAACTCCCAAGGCTACCCAAG | 41 |
| SRD5A2-h7 | AAGGACTCCATTTCCAGTGCAGAAG | 42 |
| SRD5A2-h8 | AATGCAAGTGCTGGGAGGGACCAAG | 43 |
| SRD5A2-h9 | ACCGTATGTCTGTGTACCACCCATC | 44 |
| SRD5A2-h10 | TACGTGTCACTCTGACAGCTCCATA | 45 |
| SRD5A2-h11 | ATGAGTATAGCTGGATAAGGCCTCC | 46 |
| SRD5A2-h12 | ATTAATGTCATAGGGAGGAAAGTTG | 47 |
| SRD5A2-h13 | CTGCTAGAGCTTTAGACACTGCATT | 48 |
| SRD5A2-h14 | CTCGATTGAGCAGTGAGTACACAAA | 49 |
| SRD5A2-h15 | TGGATAACTGGTTAGCAGTGCTATG | 50 |
| SRD5A2-h16 | AAAGATGAATGGAATAAGGGCTTTC | 51 |
| SRD5A2-h17 | TGGTGAAAAGCTCGCAGCCCAAGGA | 52 |
| SRD5A2-h18 | AGTAATGTAGGCAGAAGAGGCCCAG | 53 |
| SRD5A2-h19 | CCAGAAACATACGTAAACAAGCCAC | 54 |
| SRD5A2-h20 | CACCGCGAAGGAAGGCAGCTCCTGC | 55 |
| SRD5A2-h21 | CTTCAGGCTCTCCGTGTGCTTCCCG | 56 |
| SRD5A2-h22 | GCGACGTACAAGGCCAGTGCCCCAA | 57 |

The fragment targeting the SRD5A2 RNA target site was obtained using primer annealing in the same manner as in Example 1.

After digesting the C13-2-BsaI plasmid with the restriction enzyme BsaI, the annealing product and the purified backbone after digestion were ligated by T4. After transforming E. coli, positive clones were selected and the target plasmid was extracted, which is the verification vector(which can express C13-2 protein and gRNA targeting SRD5A2 RNA) for subsequent experiments.

Control vectors were also constructed to express shRNA-1 and shRNA-2 targeting SRD5A2 RNA, as shown in Table 5.

Table 5. Designed shRNAs

|  | target sequence | SEQ ID NO |
|---|---|---|
| shRNA-1 | GGAGGCCTTATCCAGCTATAC | 59 |
| shRNA-2 | GGGAATGGGAATAAACATTCA | 60 |

shRNA fragments targeting the SRD5A2 target site were obtained using primer annealing, and the primers are shown in Table 6.

Table 6. Primers corresponding to shRNA vectors

|  | Corresponding primer sequence | SEQ ID NO |
|---|---|---|
| shRNA-1 | caccgGGAGGCCTTATCCAGCTATACCTCGAGGTATA GCTGGATAAGGCCTCCTTTTTT | 61 |
|  | ggccAAAAAAGGAGGCCTTATCCAGCTATACCTCGAG GTATAGCTGGATAAGGCCTCCc | 62 |
| shRNA-2 | caccgGGGAATGGGAATAAACATTCACTCGAGTGAAT GTTTATTCCCATTCCCTTTTTT | 63 |
|  | ggccAAAAAAGGGAATGGGAATAAACATTCACTCGAG TGAATGTTTATTCCCATTCCCc | 64 |

The backbone pAAV-CMV-EGFP(SEQ ID NO: 58) for the vector expressing shRNA was synthesized at an outsourced service company. The backbone was double-digested with restriction enzymes Bsa I and Not I. The annealed product and the purified backbone after digestion were ligated at T4. After transformation into E. coli, positive clones were selected and the target plasmid was extracted. The plasmid can express shRNA-1 and shRNA-2 and is used for subsequent experiments.

2. Transfection of 293T cells with the vector to be verified

Construction of a 293T cell line overexpressing SRD5A2(293T-SRD5A2 cells): A vector Lv-SRD5A2-T2a-GFP(SEQ ID NO: 65) overexpressing the SRD5A2 gene and the EGFP gene was constructed. SRD5A2 and EGFP were separated by a 2A peptide. The Lv-SRD5A2-T2a-GFP plasmid was packaged into lentivirus and transduced into 293T cells to form a stable 293T-SRD5A2 cell line overexpressing the SRD5A2 gene.

Validation vectors carrying different target sites and shRNA control vectors were transfected into 293T-SRD5A2 cells. The negative control group was transfected with the C13-2-BsaI vector.

Transfection was performed in a 24- well plate according to the Lipofectamine 2000 instructions.

3. qPCR detection

72 h after transfection, qPCR detection was performed using the same method as in Example 1. A separate 293T-NC control group was set up: qPCR was used to detect the SRD5A2 RNA level in untreated 293T cells(which did not highly express SRD5A2 and were not transfected with gene editing plasmids or shRNA plasmids).

The primers used for qPCR are shown below:

Detection of SRD5A2(primer 2, P2): ACTGCTCAATCGAGGGAGG(SEQ ID NO: 66), CACCCAAGCTAAACCGTA TGTC(SEQ ID NO: 67);
strain SRD5A2(protein3, P3): CGGTTTAGCTTGGGTGTCTTC(SEQ ID NO: 68), CCGAGGAAATTGGCTCCAGAA (SEQ ID NO: 69);

detection of internal references GAPDH: CCATGGGGAAGGTGAAGGTC(SEQ ID NO: 20), GAAGGGGTCATTG ATGGCAAC(SEQ ID NO: 21).

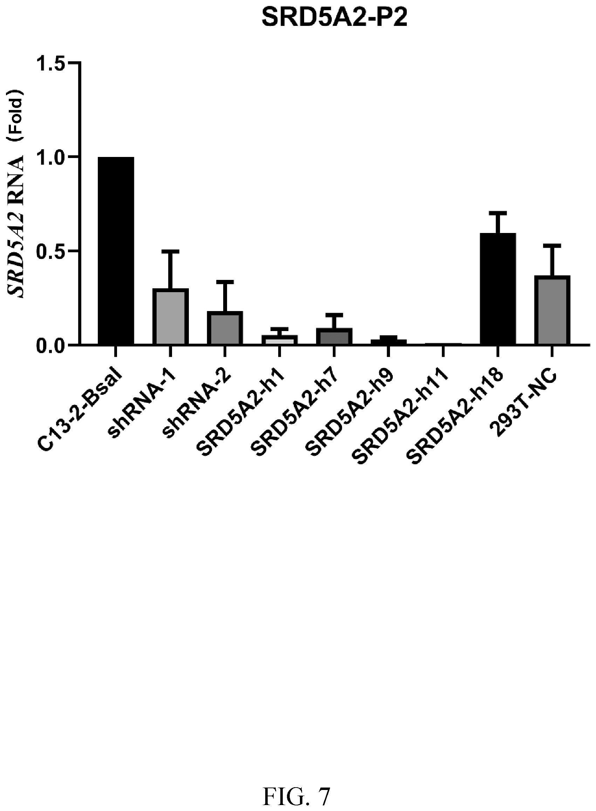

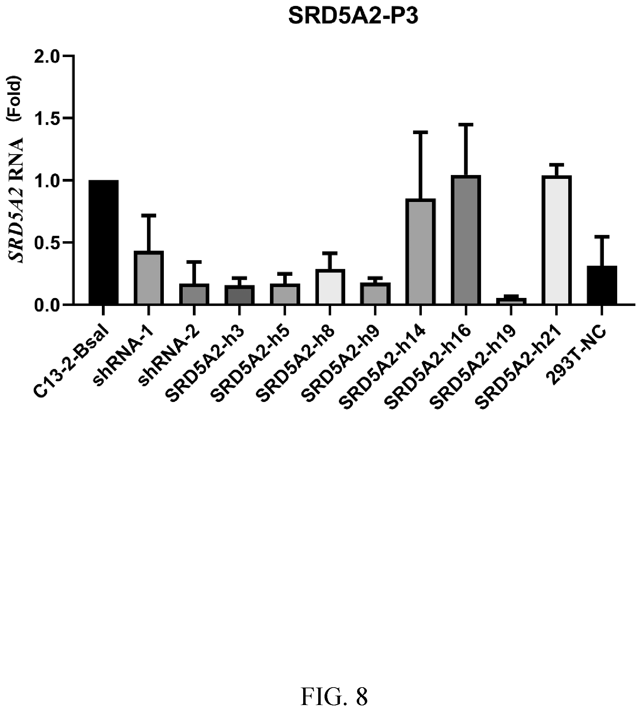

The level of the edited target RNA was calculated using the $2^{-\Delta\Delta Ct}$ method, with C13-2-BsaI serving as the negative control group and counted as 1.00. Multiple experiments were repeated, and the results were averaged. See Figures 7 and 8. FIG. 7 shows the detection results using primer 2(P2). FIG. 8 shows the detection results using primer 3(P3).

In FIG. 7, all gRNA experimental groups effectively downregulated the expression of target RNA compared with the negative control group, showing statistically significant differences($P<0.01$). The editing efficiency($>90\%$) of the h1, h7, h9, and h11 gRNA experimental groups was higher than that of shRNA-1 and shRNA-2. The h9 and h11 gRNA experimental groups showed statistically significant differences compared with shRNA-1($P<0.05$).

In FIG. 8, the h3, h5, h8, h9, and h19 gRNA experimental groups all effectively downregulated the expression of target RNA compared with the negative control group, showing statistically significant differences($P<0.01$). The editing efficiency of the h3, h5, h8, and h9 gRNA experimental groups was higher than that of shRNA-1. The editing efficiency of the h19 gRNA experimental group was 95%, which was higher than that of shRNA-1 and shRNA-2.

**Example 4. Validation of the editing efficiency of the gRNA of the present invention compared with other CTGF-targeting Cas13**

Editing efficiency was tested using a method that was essentially the same as that used in the previous embodiments.

The CasRx-BsaI plasmid was synthesized by an outsourced laboratory, and its sequence is shown in SEQ ID NO: 70. Various CasRx-CTGF gRNA plasmids were constructed by annealing with sequence-associated primers, digestion with BsaI, and ligation with T4 enzyme, which can express CasRx and gRNA targeting CTGF. Transfection was performed in a 293T cell line that highly expresses CTGF, and the knockdown efficiency was detected by qPCR.

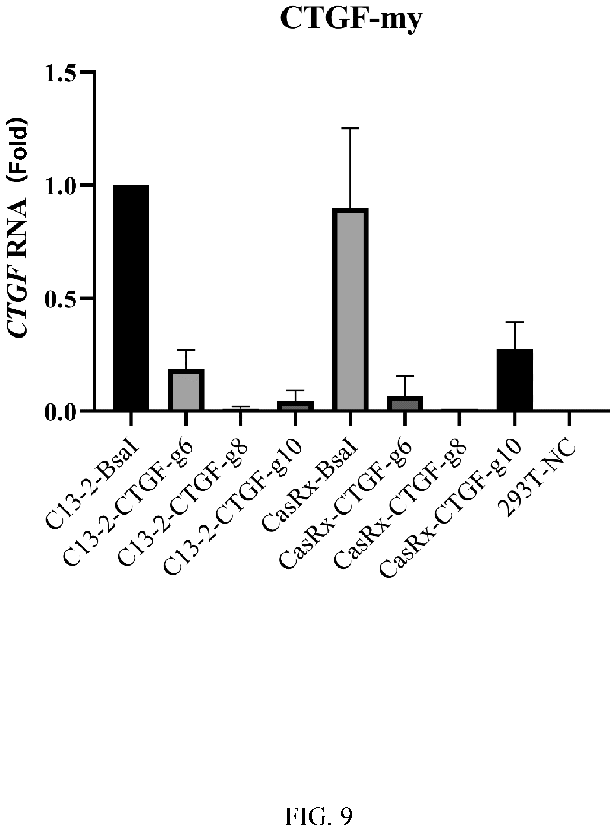

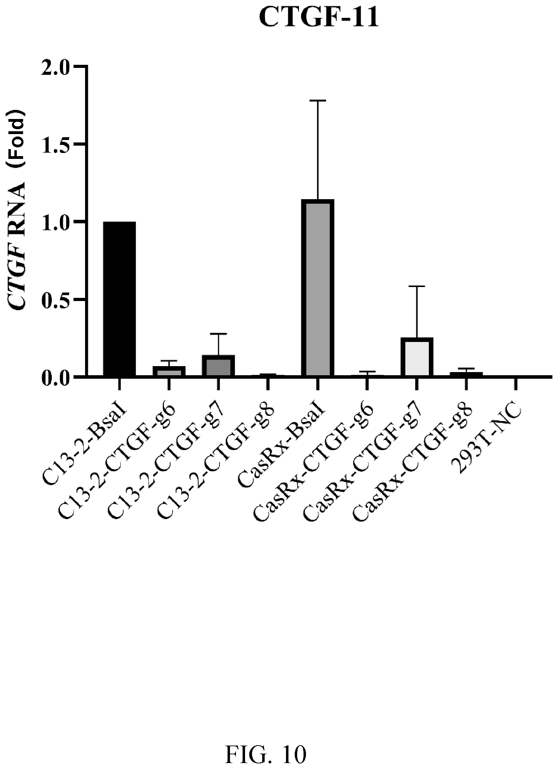

The results are shown in Figures 9 and 10. CasRx also showed high editing efficiency when combined with CTGF-g6, g7, g8, and g10 gRNA.

**Example 5. Validation of the editing efficiency of the gRNA of the present invention with other MITF-targeting Cas13**

The editing efficiency was tested in a manner that was essentially the same as that in the foregoing embodiments.

Each CasRx-MITF gRNA plasmid was constructed by annealing of sequence-related primers, digestion with BsaI enzyme in the CasRx-BsaI plasmid, and ligation with T4 enzyme. The plasmids can express CasRx and gRNA targeting MITF. The plasmids were transfected in 293T cell lines, and the knockdown efficiency was detected by qPCR after 72 h.

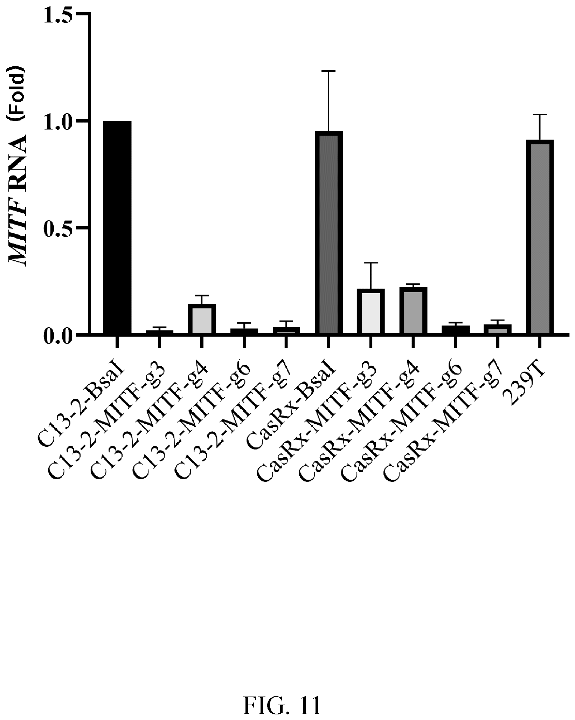

As shown in FIG. 11, CasRx also showed high editing efficiency when combined with MITF-g3, g4, g6, and g7 gRNA.

**Example 6. Off-target detection and analysis of C13-2 combined with gRNA editing MITF**

Take cells transfected for 48 hours in Experiment 5, extract total RNA, and send samples for RNAseq sequencing(n=3 samples per group). The library type is LncRNA chain-specific library, the sequencing data volume is 16G, and the sequencing strategy is PE150.

Principle of RNAseq analysis:

1. Use fastqc and multiqc to perform quality control on the data, and use fastp to remove low-quality reads.

2. The rRNA reads were aligned to the hg38 reference genome using Hisat2 alignment software to remove the rRNA reads.

3. After comparison, the expression level of the genes was quantified using Kallisto software, and then the expression difference(vs. 293T-NC) was analyzed using Sleuth software. Genes with $|b| > 0.5$, $qval < 0.05$, and $mean\_obs > 1$ were considered as differentially expressed genes.

4. Align the sgRNA sequence to the reference cDNA using EMBOSS water software. Transcripts with an alignment base count $>= 18$, mismatched base count $<= 6$, and minimum consecutive base pair count $>= 8$ are considered as predicted off-target transcripts, and the corresponding genes are considered as off-target genes.

5. Take the intersection of the significantly downregulated genes and the predicted off-target genes to obtain the off-target gene set.

Analysis of RNASeq results:

Using the unedited 293T-NC as a baseline, the expression levels of the edited MITF gene in different experimental groups were analyzed, and the results are shown in Table 7 below.

Table 7. Expression levels of the edited MITF gene

| sample | CPM average |
|---|---|
| C13-2-MITF-g3 | 4.65 |
| C13-2-MITF-g4 | 5.98 |
| C13-2-MITF-g6 | 5.75 |
| C13-2-MITF-g7 | 7.49 |
| 239T-NC | 12.44 |

The results of off-target gene analysis are shown in Table 8 below.

Table 8. Results of Off-Target Gene Analysis

| sample | Total number of differentially expressed genes upregulated | Total number of differentially expressed genes downregulated | Predict the number of off-target genes | Number of off-target genes(Down_i sec_Pred) |
|---|---|---|---|---|
| C13-2-MITF-g3 | 30 | 1 | 2235 | 1 |
| C13-2-MITF-g4 | 13 | 1 | 2260 | 1 |
| C13-2-MITF-g6 | 18 | 2 | 5713 | 2 |
| C13-2-MITF-g7 | 13 | 2 | 1425 | 1 |
| 239T-NC | N/A | N/A | N/A | N/A |

Experimental results showed that MITF-g3, MITF-g4, and MITF-g7 gRNAs only produced editing of the MITF target and did not have off-target effects. MITF-g6 gRNA had one off-target gene.

**Example 7. Validation of the editing efficiency of the gRNA of the present invention with other Cas13-targeted SRD5A2**

The editing efficiency was tested in a manner that was essentially the same as that used in the foregoing embodiments.

CasRx-SRD5A2 gRNA plasmids were constructed by annealing of sequence-related primers, digestion with BsaI enzyme in the CasRx-BsaI plasmid, and ligation with T4 enzyme. These plasmids can express CasRx and gRNA targeting SRD5A2. Transfection was performed in a 293T cell line overexpressing the SRD5A2 gene, and the knockdown efficiency was detected by qPCR after 72 h.

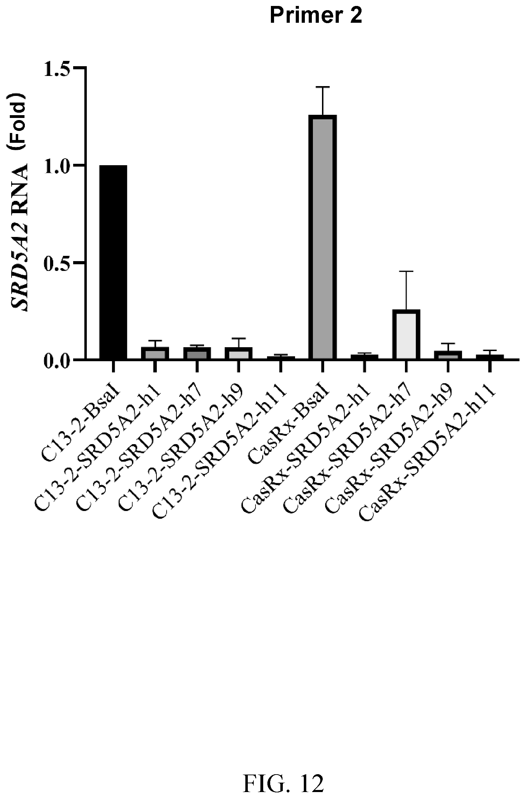

As shown in FIG. 12, CasRx also showed high editing efficiency when combined with SRD5A2-h1, h7, h9, and h11 gRNA.

**Example 8. Validation of Endogenous AR Gene Editing Efficiency**

Using the combination of the Cas13 tool C13-2 protein and gRNA(crRNA), human AR mRNA(NCBI Reference Sequence: NM_000044.6, SEQ ID NO: 394) can be targeted and edited. By downregulating AR expression, androgen receptor expression is downregulated, which can ultimately be used to treat androgenetic alopecia.

(1) Constructing a validation vector targeting the endogenous gene AR

The C13-2-BsaI plasmid with a universal crRNA scaffold expression cassette was synthesized at an outsourced company(SEQ ID NO: 4).

The sense and antisense strands of the DNA sequence were synthesized. The sense strand was formed by adding agac to the 5' end of the guide sequence shown in Table 9, and the antisense strand was formed by adding aaaa to the 5' end of the inverse complementary sequence of the guide sequence. Fragments targeting AR RNA sites were obtained using primer annealing.

The primer annealing reaction system is shown in the table below. It is incubated at 95°C for 5 minutes in a PCR instrument, and then immediately taken out and incubated on ice for 5 minutes to allow the primers to anneal each other to form double-stranded DNA with sticky ends.

Table 10. Primer annealing reaction system

Oligo-F(10 μM) 2 μl
Oligo-R(10 μM) 2 μl
10× endonuclease reaction buffer * 2 μl
Total deionized water 20 μl

The C13-2-BsaI plasmid was digested with *BsaI* restriction enzyme, and the backbone was purified and recovered. The annealing product was ligated with T4 ligase to obtain the validation vector plasmid(CMV-C13-2-U6-gRNA). After transformation of E. coli, positive clones were selected and the plasmid was extracted for subsequent experiments.

(2) Transfection of 293T cells with the vector to be verified

Validation vectors carrying different target site sequences and control vector(C13-2-BsaI) were transfected into 293T cells.

Transfect 24- well plates according to the Lipofectamine 2000(Thermo) instructions.

(3) qPCR detection

RNA was extracted from cells 72 h post-transfection using the SteadyPure Universal RNA Extraction Kit AG21017, and RNA concentration was detected using a micro-spectrophotometer. RNA products were reverse transcribed using the Evo M-MLV Mix Kit with gDNA Clean for qPCR AG11728, and the reverse transcription products were detected using the SYBR Green Premix Pro Taq HS qPCR Kit(Low Rox Plus).

The primers used for qPCR are shown below:

Detection of AR(primer 4): GACGACCAGATGGCTGTCATT(SEQ ID NO: 380), GGGCGAAGTAGAGCATCCT(SEQ ID NO: 381).
Detection of AR(primer 5): CCAGGGACCATGTTTTGCC(SEQ ID NO: 382), CGAAGACGACAAGATGGACAA(SEQ ID NO: 383).
Detection of internal references GAPDH: CCATGGGGAAGGTGAAGGTC(SEQ ID NO: 384), GAAGGGGTCATT GATGGCAAC(SEQ ID NO: 385).

The reaction system was prepared according to the instructions of SYBR® Green Premix Pro Taq HS qPCR Kit(Rox Plus) and the detection was performed using QuantStudio™ 5 Real-Time PCR System.

This experiment used a relative quantification method, namely the $2^{-\triangle\triangle Ct}$ method, to calculate the level of the target RNA. The calculation method is shown below:

$$\triangle Ct = Ct(AR) - Ct(GAPDH)$$

$\triangle\triangle Ct = \triangle Ct(\text{vector to be verified, such as C13-2-AR-h1}) - \triangle Ct(\text{control C13-2-BsaI})$

$$2^{-\triangle\triangle Ct} = 2^{\wedge}(-\triangle\triangle Ct)$$

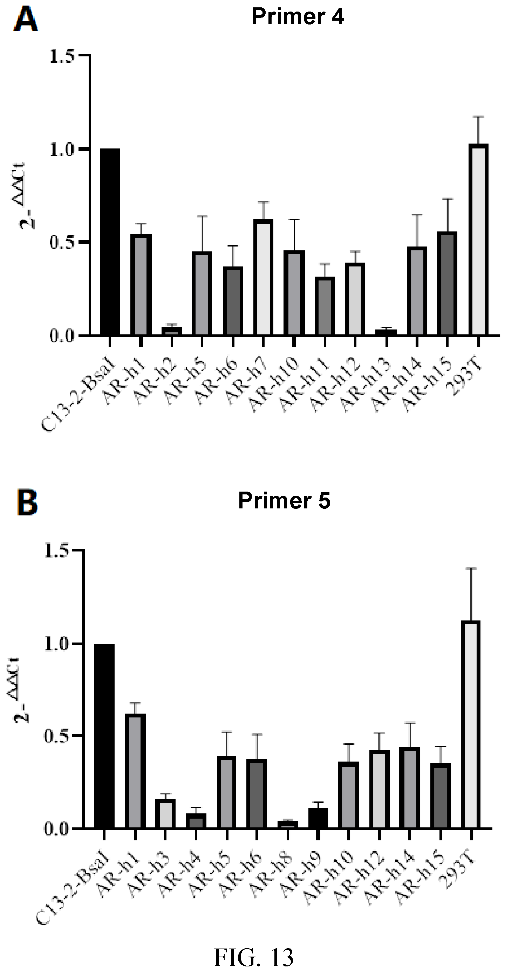

The $2^{-\Delta\Delta Ct}$ values of the edited AR RNA were calculated according to the above calculation method, as shown in Table 11, Table 12 and FIG. 13 below.

Table 11. qPCR detection of edited AR RNA levels(primer 4)

| Grouping | Edited AR RNA levels |
|---|---|
| C13-2-BsaI | 1.000 |
| C13-2-AR-h1 | 0.544 |
| C13-2-AR-h2 | 0.044 |
| C13-2-AR-h5 | 0.452 |
| C13-2-AR-h6 | 0.371 |
| C13-2-AR-h7 | 0.623 |
| C13-2-AR-h10 | 0.457 |
| C13-2-AR-h11 | 0.318 |
| C13-2-AR-h12 | 0.389 |
| C13-2-AR-h13 | 0.031 |
| C13-2-AR-h14 | 0.480 |
| C13-2-AR-h15 | 0.561 |
| 293T(NC) | 1.029 |
| *NC refers to untransfected 293T cells as a blank control. | |

Table 12. qPCR detection of edited AR RNA levels(primer 5)

| Grouping | Edited AR RNA levels |
|---|---|
| C13-2-BsaI | 1.000 |
| C13-2-AR-h1 | 0.621 |
| C13-2-AR-h3 | 0.159 |
| C13-2-AR-h4 | 0.082 |
| C13-2-AR-h5 | 0.391 |
| C13-2-AR-h6 | 0.378 |
| C13-2-AR-h8 | 0.040 |
| C13-2-AR-h9 | 0.111 |
| C13-2-AR-h10 | 0.361 |
| C13-2-AR-h12 | 0.428 |
| C13-2-AR-h14 | 0.444 |
| C13-2-AR-h15 | 0.354 |
| 293T(NC) | 1.123 |
| *NC refers to untransfected 293T cells as a blank control. | |

qPCR results showed that C13-2 combined with gRNA could downregulate AR RNA expression levels.

## Example 9. Editing effect test of different Cas13

(1) Constructing a validation vector targeting the endogenous gene AR

Different control groups were set up to compare the editing effects of C13-2+gRNA, CasRx+gRNA, and shRNA on AR RNA.

The CasRx-BsaI plasmid(SEQ ID NO: 70), the control vector shRNA-asiAR72 plasmid(SEQ ID NO: 387), and the shRNA-ARstart plasmid(SEQ ID NO: 388) with a universal crRNA backbone expression cassette were synthesized by an outsourced company.

The shRNA-asiAR72 plasmid was used to express shRNA, and its target sequence was: GTTCACTTTTGA CCTGCTAAT(SEQ ID NO: 390), located in exon 8 of the AR gene.

The shRNA-ARstart plasmid is used to express shRNA, and its target sequence is: GACCTACCGAGGAGC TTTC(SEQ ID NO: 391), located in exon 1 of the AR gene.

The CasRx+gRNA vector(CMV-CasRx-U6-gRNA) was constructed using the method described in Example 9. The sense and antisense strands of the DNA sequence were synthesized. The sense strand was constructed by adding aaac to the 5' end of the guide sequence shown in Table 9, and the antisense strand was constructed by adding aaaa to the 5' end of the inverse complementary sequence of the guide sequence. Fragments targeting AR RNA sites were obtained using primer annealing. These fragments were then ligated with the *BsaI digestion fragment of* the CasRx-BsaI plasmid using T4 ligase. Positive clones were selected after transformation into E. coli, and the plasmid was extracted for subsequent experiments.

(2) Transfection of 293T cells with the vector to be verified

293T cells were transfected with vectors carrying different target sites and control vectors. Untransfected 293T cells served as blank controls(NC group).

Transfect 24-well plates according to the Lipofectamine 2000(Thermo) instructions.

(3) qPCR detection

Cells 72 h post-transfection were subjected to qPCR detection using the same method as in Example 8. The level of target RNA was calculated using a relative quantification method, namely the $2^{-\Delta\Delta Ct}$ method.

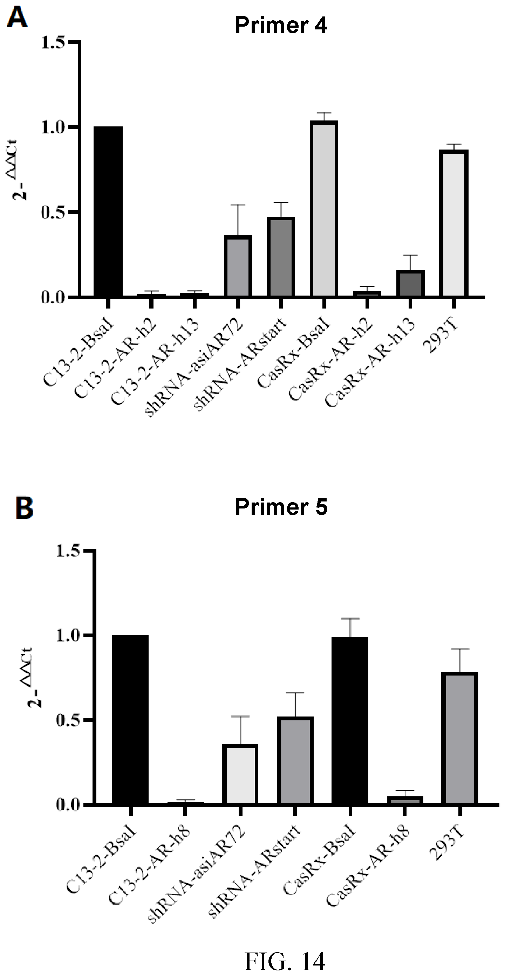

Tables 13, 14 and FIG. 14 show the comparative test results with shRNA(all were repeated experiments with 3 times each, and the results were averaged). AR-h2, AR-13 and AR-h8 gRNAs were more effective at knocking down AR RNA than shRNA.

Table 13. Comparison of qPCR and shRNA detection results(primer 4)

| Grouping | Edited AR RNA levels |
| --- | --- |
| C13-2-BsaI | 1.00 |
| C13-2-AR-h2 | 0.02 |
| C13-2-AR-h13 | 0.03 |
| shRNA-asiAR72 | 0.36 |
| shRNA-ARstart | 0.47 |
| CasRx-BsaI | 1.04 |
| CasRx-AR-h2 | 0.04 |
| CasRx-AR-h13 | 0.16 |
| 293T(NC) | 0.87 |

Table 14. Comparison of qPCR and shRNA detection results(primer 5)

| Grouping | Edited AR RNA levels |
| --- | --- |
| C13-2-BsaI | 1.00 |
| C13-2-AR-h8 | 0.02 |
| shRNA-asiAR72 | 0.36 |
| shRNA-ARstart | 0.52 |
| CasRx-BsaI | 0.99 |
| CasRx-AR-h8 | 0.05 |

(continued)

| Grouping | Edited AR RNA levels |
|---|---|
| 293T(NC) | 0.78 |

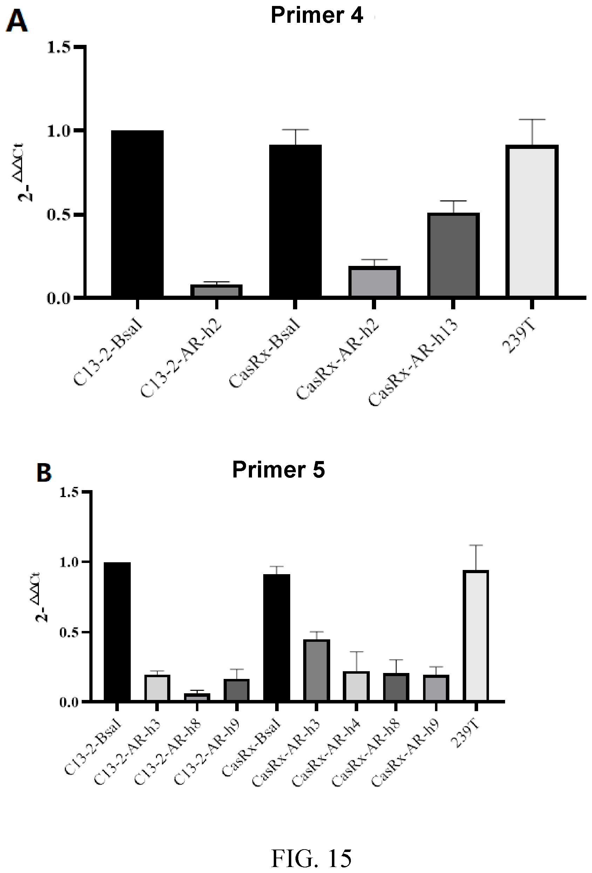

Tables 15, 16 and FIG. 15 show the test results of C13-2 and CasRx in the same batch of experiments(all were repeated 3 times, and the results were averaged). C13-2 and CasRx can knock down AR RNA when combined with different gRNAs, and C13-2 has a slightly better editing efficiency than CasRx.

Table 15. Results of qPCR detection and CasRx testing in the same batch of experiments(primer 4)

| Grouping | Edited AR RNA levels |
|---|---|
| C13-2-BsaI | 1.00 |
| C13-2-AR-h2 | 0.08 |
| CasRx-BsaI | 0.91 |
| CasRx-AR-h2 | 0.19 |
| CasRx-AR-h13 | 0.51 |
| 293T(NC) | 0.92 |

Table 16. Results of qPCR detection and CasRx testing in the same batch of experiments(primer 5)

| Grouping | Edited AR RNA levels |
|---|---|
| C13-2-BsaI | 1.00 |
| C13-2-AR-h3 | 0.20 |
| C13-2-AR-h8 | 0.06 |
| C13-2-AR-h9 | 0.16 |
| CasRx-BsaI | 0.91 |
| CasRx-AR-h3 | 0.45 |
| CasRx-AR-h4 | 0.22 |
| CasRx-AR-h8 | 0.21 |
| CasRx-AR-h9 | 0.19 |
| 293T(NC) | 0.95 |

(4) Protein level detection(WB)

Cells 72 h after transfection were digested with trypsin, centrifuged, washed with PBS, and lysed on ice for 30 min with 100 μL of cold RIPA lysis buffer. 5× loading buffer was added, the cells were boiled in a water bath for 5 min, centrifuged at maximum speed for 1 min, and the supernatant was collected for protein electrophoresis. After electrophoresis, the cells were transferred to a PVDF membrane for Western blotting. The internal control was detected using GAPDH antibody(cellsignal, #5174), and the target gene protein expression was detected using AR antibody(abcam, ab133273). Goat anti-rabbit secondary antibody(Sigma-Aldrich, A0545) was used.

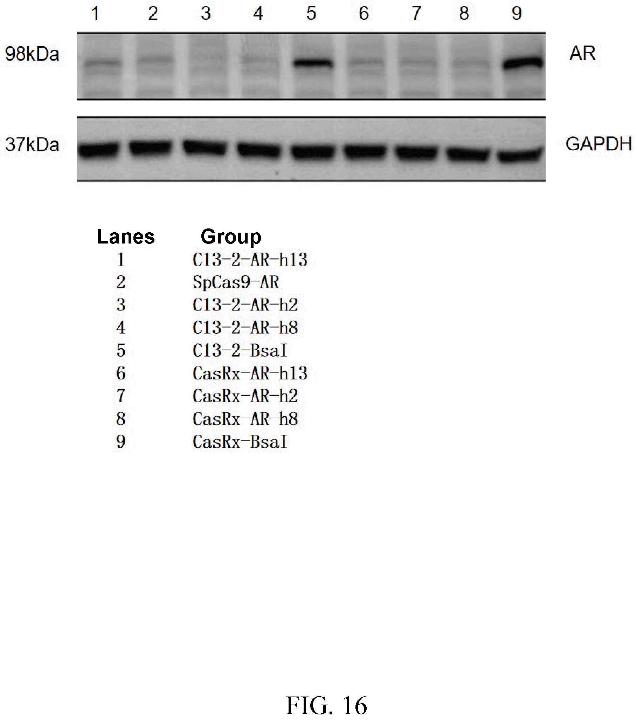

The WB results are shown in FIG. 16. As can be seen from the figure, compared with the C13-2-BsaI and CasRx-BsaI control groups, the C13-2 and CasRx experimental groups can significantly downregulate the expression of AR protein.

### Example 10. Off-target detection

Construction of control vector

Since the 293T cells do not contain the EGFP sequence, a C13-2-GFP vector targeting EGFP was constructed using the method of Example 8 as a negative control. The spacer targeting GFP was tgccgttcttctgcttgtcggccatgatat(SEQ ID NO: 393).

RNAseq sequencing

Using the method of Example 8, a validation vector expressing C13-2 and AR-h2, AR-h4, AR-h8 or AR-h9 gRNA was constructed and used as the experimental group. The vector and the negative control group were transfected into 293T cells. After 48 hours, total RNA samples were extracted and RNAseq was performed(n=3 samples per group). The library type was LncRNA chain-specific library, the sequencing data volume was 16G, and the sequencing strategy was PE150.

Principles of RNAseq analysis

Use fastqc and multiqc for data quality control, and use fastp to remove low-quality reads.
The sequence of human rRNA was removed and aligned to the hg38 reference genome using Hisat2 alignment software.
After comparison, the expression level of the genes was quantified using Kallisto software, and then the expression difference analysis was performed using Sleuth software. Genes with |b| > 0.5, qval < 0.05, and mean_obs > 2 were considered as differentially expressed genes(DEG).
The guide sequence of gRNA was aligned to the reference cDNA using EMBOSS water software. Transcripts with ≥18 aligned bases, ≤6 mismatched bases, and ≥8 minimum consecutive paired bases were considered as predicted off-target transcripts, and the corresponding genes were considered as predicted off-target genes.
The intersection of the genes that are significantly downregulated in DEG and the genes that are predicted to be off-target is taken, and the AR genes are removed to obtain the off-target gene set.

Analysis of RNASeq Results

The analysis results are shown in Table 17 below.

Table 17 Off-target detection analysis results

| Grouping | Predicted off-target genes | After taking the intersection and removing the AR gene The number of off-target genes determined |
|---|---|---|
| C13-2-GFP | 160 | 0 |
| C13-2-AR-h2 | 2354 | 0 |
| C13-2-AR-h4 | 3022 | 0 |
| C13-2-AR-h8 | 2597 | 0 |
| C13-2-AR-h9 | 1979 | 0 |

RNAseq results showed that no off-target effects related to the guide sequence were detected after C13-2 was edited in combination with AR-h2, AR-h4, AR-h8 and AR-h9 gRNA.
Although specific embodiments of the present invention have been described above, those skilled in the art should understand that these are merely illustrative examples, and various changes or modifications can be made to these embodiments without departing from the principles and essence of the present invention. Therefore, the scope of protection of the present invention is defined by the appended claims.

**Claims**

1. An inhibitor of CTGF RNA, MITF RNA, or SRD5A2 RNA, wherein the inhibitor is a gene editing system; optionally, the gene editing system knocks down the levels of CTGF RNA, MITF RNA or SRD5A2 RNA, or inhibits the translation of CTGF RNA, MITF RNA or SRD5A2 RNA.

2. A guide RNA(gRNA) for a gene editing system, the guide RNA comprising a guide sequence for hybridization with a target RNA, the target RNA being CTGF RNA, MITF RNA, or SRD5A2 RNA;

   optionally, the target RNA is CTGF pre-mRNA, MITF pre-mRNA, or SRD5A2 pre-mRNA, or CTGF mRNA, MITF mRNA, or SRD5A2 mRNA;
optionally, the target RNA is mammalian CTGF RNA, MITF RNA, or SRD5A2 RNA.

3. A pharmaceutical composition comprising the inhibitor according to claim 1 or the guide RNA according to claim 2; optionally, the pharmaceutical composition comprises a pharmaceutically acceptable excipient.

4. Use of the inhibitor according to claim 1 or the guide RNA according to claim 2 in preparation of a medicament for diagnosing, treating, or preventing a disease or condition associated with the target RNA;

> optionally, the disease or condition associated with the target RNA is a disease or condition caused by abnormally high expression of the target RNA;
> optionally, the target RNA is CTGF pre-mRNA, MITF pre-mRNA, or SRD5A2 pre-mRNA, or CTGF mRNA, MITF mRNA, or SRD5A2 mRNA;
> optionally, the target RNA is human CTGF RNA, MITF RNA, or SRD5A2 RNA;
> optionally, the target RNA sequence is as shown in SEQ ID NO: 14, 22 or 35;
> optionally, the target RNA sequence is nucleotides 494 to 785 of the sequence shown in SEQ ID NO: 14, nucleotides 404 to 606 of the sequence shown in SEQ ID NO: 22, or nucleotides 484 to 820 of the sequence shown in SEQ ID NO: 35.

5. Use of the inhibitor according to claim 1 or the guide RNA according to claim 2 in the preparation of cosmetics.

6. A cosmetic comprising the inhibitor according to claim 1 or the guide RNA according to claim 2.

7. Use of a gene-editing system in the manufacture of a medicament for diagnosing, treating, or preventing androgenetic alopecia, wherein the gene-editing system knocks down the level of androgen receptor (AR) RNA or inhibits translation of AR RNA.

8. A gene-editing system, wherein the gene-editing system knocks down the level of AR RNA or inhibits translation of AR RNA.

9. A guide RNA(gRNA) for a gene editing system, wherein the guide RNA comprises a guide sequence that hybridizes to AR RNA.

10. A pharmaceutical composition comprising the gene-editing system according to claim 2.

FIG. 1

FIG. 2

FIG. 3

FIG. 4

**MITF**

FIG. 5

FIG. 6

FIG. 7

FIG. 8

FIG. 9

FIG. 10

FIG. 11

**Primer 2**

FIG. 12

FIG. 13

FIG. 14

FIG. 15

|       |              |
|-------|--------------|
| **Lanes** | **Group**      |
| 1     | C13-2-AR-h13 |
| 2     | SpCas9-AR    |
| 3     | C13-2-AR-h2  |
| 4     | C13-2-AR-h8  |
| 5     | C13-2-BsaI   |
| 6     | CasRx-AR-h13 |
| 7     | CasRx-AR-h2  |
| 8     | CasRx-AR-h8  |
| 9     | CasRx-BsaI   |

FIG. 16

## INTERNATIONAL SEARCH REPORT

| International application No. |
|---|
| **PCT/CN2024/109575** |

**A. CLASSIFICATION OF SUBJECT MATTER**

C12N15/113(2010.01)i; A61K48/00(2006.01)i; C12N15/85(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C12N, A61K, A61P

Documentation searched other than minimum documentation to the extent that such documents are included in the fields searched

Electronic data base consulted during the international search (name of data base and, where practicable, search terms used)

DWPI, VEN, CNABS, CNTXT, CNKI, PubMed, GenBank, EBI, STN, WPABSC, WPABS, ENTXT, ENTXTC, WANFANG DATABASE, ISI web of Knowledge: CTGF, 结缔组织生长因子, connective tissue growth factor, ctgrofact, CCN2, MITF, 小眼相关转录因子, Microphthalmia-associated transcription factor, 微眼炎组织因子, SRD5A2, 3-oxo-5α-类固醇4-脱氢酶2, 2型5α-还原酶, 5αR2, 5αreductase, antisense oligonucleotides, ASOs, gRNA, 基因编辑, 雄激素性脱发, Androgenetic alopecia, AGA, mRNA, hair, 抑制, inhibit+

**C. DOCUMENTS CONSIDERED TO BE RELEVANT**

| Category* | Citation of document, with indication, where appropriate, of the relevant passages | Relevant to claim No. |
|---|---|---|
| X | CN 103429270 A (EXCALIARD PHARMACEUTICALS, INC. et al.) 04 December 2013 (2013-12-04) <br> description, paragraphs 28-64 | 1-6, 10 |
| X | WO 2022062440 A1 (GUANGZHOU REFORGENE MEDICINE CO., LTD.) 31 March 2022 (2022-03-31) <br> claims 1-13 | 1-6, 10 |
| X | CN 110540990 A (WUHAN ZEZHI BIOLOGICAL PHARMACEUTICAL CO., LTD.) 06 December 2019 (2019-12-06) <br> claims 1-10, and description, paragraphs 2-11 | 1-6, 10 |
| X | US 2014336371 A1 (JIANGSU GENECON BIOTECHNOLOGIES CO., LTD.) 13 November 2014 (2014-11-13) <br> description, the embodiments | 1-6, 10 |

☑ Further documents are listed in the continuation of Box C.    ☑ See patent family annex.

| | |
|---|---|
| \*   Special categories of cited documents: <br> "A"   document defining the general state of the art which is not considered to be of particular relevance <br> "D"   document cited by the applicant in the international application <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
|---|---|
| **10 September 2024** | **11 October 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/109575** |

**C.   DOCUMENTS CONSIDERED TO BE RELEVANT**

| Category* | Citation of document, with indication, where appropriate, of the relevant passages | Relevant to claim No. |
| --- | --- | --- |
| X | WO 2005045037 A2 (SIRNA THERAPEUTICS, INC. et al.) 19 May 2005 (2005-05-19)<br>claims 1-35 | 1-10 |
| X | US 2019382771 A1 (OLIX PHARMACEUTICALS, INC.) 19 December 2019 (2019-12-19)<br>claims 1-11 | 1-10 |
| X | CN 101054579 A (SHANGHAI FUDAN XINYANG BIOTECHNOLOGY CO., LTD. et al.)<br>17 October 2007 (2007-10-17)<br>claims 1-5 | 7-9 |
| X | US 2021369859 A1 (MOOGENE MEDI CO., LTD.) 02 December 2021 (2021-12-02)<br>claims 1-7 | 1-6, 10 |
| A | CN 111073881 A (KIM HYUN SEOK) 28 April 2020 (2020-04-28)<br>description, paragraphs 24-28 | 1-10 |
| A | US 2022249509 A1 (ICAHN SCHOOL OF MEDICINE AT MOUNT SINAI) 11 August 2022<br>(2022-08-11)<br>description, table 1 | 1-10 |
| A | WO 2023128862 A1 (LIVIUS PTE. LTD.) 06 July 2023 (2023-07-06)<br>claims 1-29 | 1-10 |

Form PCT/ISA/210 (second sheet) (July 2022)

## INTERNATIONAL SEARCH REPORT

| International application No. |
|---|
| **PCT/CN2024/109575** |

| **Box No. I** | **Nucleotide and/or amino acid sequence(s) (Continuation of item 1.c of the first sheet)** |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

| International application No. |
| --- |
| **PCT/CN2024/109575** |

| Patent document cited in search report | | | Publication date (day/month/year) | Patent family member(s) | | | Publication date (day/month/year) |
| --- | --- | --- | --- | --- | --- | --- | --- |
| CN | 103429270 | A | 04 December 2013 | IL | 211230 | A0 | 28 April 2011 |
| | | | | IL | 211230 | A | 29 January 2015 |
| | | | | GB | 201002626 | D0 | 31 March 2010 |
| | | | | GB | 2465902 | A | 09 June 2010 |
| | | | | GB | 2465902 | B | 08 December 2010 |
| | | | | ZA | 201101549 | B | 27 June 2012 |
| | | | | CO | 6341577 | A2 | 21 November 2011 |
| | | | | KR | 20170089946 | A | 04 August 2017 |
| | | | | KR | 101877698 | B1 | 12 July 2018 |
| | | | | HK | 1158965 | A1 | 27 July 2012 |
| | | | | US | 2019323013 | A1 | 24 October 2019 |
| | | | | AU | 2010224447 | A1 | 21 October 2010 |
| | | | | AU | 2010224447 | B2 | 09 March 2017 |
| | | | | JP | 2016041067 | A | 31 March 2016 |
| | | | | NZ | 601660 | A | 30 May 2014 |
| | | | | CA | 2733262 | A1 | 15 April 2010 |
| | | | | CA | 2733262 | C | 10 December 2019 |
| | | | | EP | 3081648 | A1 | 19 October 2016 |
| | | | | KR | 20110081154 | A | 13 July 2011 |
| | | | | KR | 101762734 | B1 | 28 July 2017 |
| | | | | IL | 224697 | A | 31 July 2016 |
| | | | | SG | 196769 | A1 | 13 February 2014 |
| | | | | WO | 2010042281 | A2 | 15 April 2010 |
| | | | | WO | 2010042281 | A9 | 06 October 2011 |
| | | | | MX | 343397 | B | 03 November 2016 |
| | | | | JP | 2019129828 | A | 08 August 2019 |
| | | | | US | 2017335329 | A1 | 23 November 2017 |
| | | | | US | 2010130595 | A1 | 27 May 2010 |
| | | | | US | 8252762 | B2 | 28 August 2012 |
| | | | | BRPI | 0912923 | A2 | 10 October 2017 |
| | | | | BRPI | 0912923 | A8 | 12 December 2017 |
| | | | | US | 2013190380 | A1 | 25 July 2013 |
| | | | | US | 9096851 | B2 | 04 August 2015 |
| | | | | HK | 1144908 | A1 | 18 March 2011 |
| | | | | DK | 2331141 | T3 | 04 April 2016 |
| | | | | MX | 2011002143 | A | 20 July 2011 |
| | | | | JP | 2017113007 | A | 29 June 2017 |
| | | | | JP | 2013099338 | A | 23 May 2013 |
| | | | | JP | 6058391 | B2 | 11 January 2017 |
| | | | | NZ | 591416 | A | 30 November 2012 |
| | | | | US | 2015376623 | A1 | 31 December 2015 |
| | | | | US | 9688987 | B2 | 27 June 2017 |
| | | | | EP | 2331141 | A2 | 15 June 2011 |
| | | | | EP | 2331141 | A4 | 23 January 2013 |
| | | | | EP | 2331141 | B1 | 06 January 2016 |
| | | | | ES | 2566508 | T3 | 13 April 2016 |
| | | | | US | 2013190382 | A1 | 25 July 2013 |
| | | | | US | 8772260 | B2 | 08 July 2014 |
| | | | | AU | 2009275387 | A1 | 11 March 2010 |
| | | | | AU | 2009275387 | B2 | 08 July 2010 |

Form PCT/ISA/210 (patent family annex) (July 2022)

## INTERNATIONAL SEARCH REPORT
### Information on patent family members

International application No.

**PCT/CN2024/109575**

| Patent document cited in search report | | | Publication date (day/month/year) | Patent family member(s) | | | Publication date (day/month/year) |
|---|---|---|---|---|---|---|---|
| | | | | JP | 2012508560 | A | 12 April 2012 |
| | | | | JP | 5420668 | B2 | 19 February 2014 |
| | | | | IL | 236309 | A0 | 29 January 2015 |
| | | | | IL | 236309 | A | 31 May 2016 |
| | | | | EA | 201170345 | A1 | 29 June 2012 |
| | | | | EA | 022207 | B1 | 30 November 2015 |
| WO | 2022062440 | A1 | 31 March 2022 | CA | 3193112 | A1 | 31 March 2022 |
| | | | | US | 2023212615 | A1 | 06 July 2023 |
| CN | 110540990 | A | 06 December 2019 | | None | | |
| US | 2014336371 | A1 | 13 November 2014 | WO | 2013060230 | A1 | 02 May 2013 |
| | | | | US | 9045753 | B2 | 02 June 2015 |
| WO | 2005045037 | A2 | 19 May 2005 | WO | 2005045037 | A3 | 03 November 2005 |
| | | | | WO | 2005045037 | B1 | 29 December 2005 |
| US | 2019382771 | A1 | 19 December 2019 | JP | 2020508699 | A | 26 March 2020 |
| | | | | JP | 6987157 | B2 | 22 December 2021 |
| | | | | CA | 3054001 | A1 | 30 August 2018 |
| | | | | WO | 2018155890 | A1 | 30 August 2018 |
| | | | | EP | 3587577 | A1 | 01 January 2020 |
| | | | | EP | 3587577 | A4 | 21 April 2021 |
| | | | | JP | 2022008307 | A | 13 January 2022 |
| | | | | US | 11118184 | B2 | 14 September 2021 |
| | | | | KR | 20180096330 | A | 29 August 2018 |
| | | | | KR | 102321426 | B1 | 05 November 2021 |
| CN | 101054579 | A | 17 October 2007 | | None | | |
| US | 2021369859 | A1 | 02 December 2021 | KR | 101870694 | B1 | 25 June 2018 |
| | | | | JP | 2020535233 | A | 03 December 2020 |
| | | | | JP | 6944061 | B2 | 06 October 2021 |
| | | | | US | 11766485 | B2 | 26 September 2023 |
| | | | | WO | 2019078611 | A1 | 25 April 2019 |
| CN | 111073881 | A | 28 April 2020 | KR | 20200040943 | A | 21 April 2020 |
| | | | | KR | 102111964 | B1 | 18 May 2020 |
| | | | | EP | 3861979 | A1 | 11 August 2021 |
| | | | | EP | 3861979 | A4 | 05 January 2022 |
| | | | | WO | 2020071662 | A1 | 09 April 2020 |
| | | | | JP | 2022502033 | A | 11 January 2022 |
| | | | | US | 2022040238 | A1 | 10 February 2022 |
| | | | | US | 12011464 | B2 | 18 June 2024 |
| US | 2022249509 | A1 | 11 August 2022 | WO | 2021007108 | A1 | 14 January 2021 |
| | | | | EP | 3993764 | A1 | 11 May 2022 |
| | | | | EP | 3993764 | A4 | 23 August 2023 |
| WO | 2023128862 | A1 | 06 July 2023 | CA | 3239665 | A1 | 06 July 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310969498
- CN 2024071775 W
- GB 275782011 T
- WO 2016123230 A
- WO 2021113522 A1
- CN 111757889 B

**Non-patent literature cited in the description**

- Nomenclature and Terminology of Cosmetics. *National Standard of the People's Republic of China*
- **HENDEL et al.** *Nat. Biotechnol.*, 2015, vol. 33 (9), 985-989
- **COX et al.** *Science*, 2017, vol. 358 (6366), 1019-1027
- **ABUDAYYEH et al.** *Science*, 2019, vol. 365 (6451), 382-386
- **MAEDER et al.** *Nature Medicine*, 2019, vol. 25, 229-233
- **GILLMORE et al.** *N. Engl. J. Med.*, 2021, vol. 385, 493-502
- **PAUNOVSKA et al.** *Nature Reviews Genetics*, 2022, vol. 23, 265-280
- **YI X et al.** MITF-siRNA formulation is a safe and effective therapy for human melasma[J].. *Molecular Therapy*, 2011, vol. 19 (2), 362-371
- **KHANTHAM C et al.** Antioxidation, Anti-Inflammation, and Regulation of SRD5A Gene Expression of Oryza sativa cv. Bue Bang 3 CMU Husk and Bran Extracts as Androgenetic Alopecia Molecular Treatment Substances.. *Plants.*, 2022, vol. 11 (3), 330, https: //doi.org/10.3390/plants11030330
- **CHO KH et al.** Local delivery of CTGF siRNA with poly(sorbitol-co-PEI) reduces scar contraction in cutaneous wound healing[J].. *Tissue Engineering and Regenerative Medicine*, 2017, vol. 14 (3), 211-220